# EUROPÄISCHE PATENTSCHRIFT

(11) **EP 2 118 666 B1**
(45) Veröffentlichungstag und Bekanntmachung des Hinweises auf die Patenterteilung: **03.08.2011**
(21) Anmeldenummer: 08717477.7
(22) Anmeldetag: 06.03.2008
(51) Int. Cl.: G01N 33/68, G01N 33/96

(54) **VERFAHREN ZUR NORMIERUNG DER KONZENTRATION VON ANALYTEN IN EINER URINPROBE**
METHOD FOR THE STANDARDIZATION OF THE CONCENTRATION OF ANALYTES IN A URINE SAMPLE
PROCÉDÉ POUR NORMALISER LA CONCENTRATION D'ANALYTES DANS UN ÉCHANTILLON D'URINE

(30) Priorität: 07.03.2007 EP 07103684
(43) Veröffentlichungstag der Anmeldung: 18.11.2009
(73) Patentinhaber: Mosaiques Diagnostics And Therapeutics AG, 30625 Hannover (DE)
(72) Erfinder: MISCHAK, Harald, 31319 Sehnde (DE)
(74) Vertreter: von Kreisler Selting Werner
(86) Internationale Anmeldenummer: PCT/EP2008/052730
(87) Internationale Veröffentlichungsnummer: WO 2008/107476

(56) Entgegenhaltungen:
- US-A1- 2006 024 757
- US-B2- 6 818 215
- DATABASE MEDLINE [Online] US NATIONAL LIBRARY OF MEDICINE (NLM), BETHESDA, MD, US; 1997, KENT G N: "Standardization of marker assays--pyridinoline/deoxypyridinoline." XP002456785 Database accession no. NLM9127470 & SCANDINAVIAN JOURNAL OF CLINICAL AND LABORATORY INVESTIGATION. SUPPLEMENTUM 1997, Bd. 227, 1997, Seiten 73-79, ISSN: 0085-591X
- SRINIVASAN V ET AL: "The DNA repair enzyme, CPD-photolyase restores the infectivity of UV-damaged fowlpox virus isolated from infected scabs of chickens" VETERINARY MICROBIOLOGY, AMSTERDAM, NL, Bd. 108, Nr. 3-4, 1. Juli 2005 (2005-07-01), Seiten 215-223, XP004933322 ISSN: 0378-1135
- KENT G N: 'Standardization of marker assays--pyridinoline/deoxypyridinoline.' SCANDINAVIAN JOURNAL OF CLINICAL AND LABORATORY INVESTIGATION Bd. 227, 1997, Seiten 73 - 79 ISSN: 0085-591X
- KENT G N: "Standardization of marker assays--pyridinoline/deoxypyridinoline." SCANDINAVIAN JOURNAL OF CLINICAL AND LABORATORY INVESTIGATION. SUPPLEMENTUM 1997, vol. 227, 1997, pages 73-79, ISSN: 0085-591X

## Beschreibung

Die vorliegende Erfindung betrifft ein Verfahren zur Normierung der Konzentration von Analyten in einer Urinprobe.

Urinproben werden schon seit langer Zeit für medizinische Laboruntersuchungen verwendet. Urinproben enthalten eine Vielzahl von Stoffen, die unter medizinischen Gesichtspunkten interessant sind, beispielsweise Abbauprodukte von Medikamenten oder Drogen, Hormone, Krankheitserreger, Eiweiße, etc.

Während früher zur Bewertung einer Urinprobe häufig der (nur) Gesamteiweißgehalt herangezogen wurde, wird mehr und mehr erkannt, dass insbesondere auch die Zusammensetzung der Proteine im Urin relevante medizinische Informationen enthält.

Die WO 01/84140 und die WO 2004/068130 beschreiben Verfahren und Vorrichtungen zur Bestimmung von Protein- und Peptidmustern in eine Flüssigkeitsprobe, beispielsweise eine Urinprobe.

Kent et al. (Scand. J. Clin. Lab. Invest 1997; S. 7, Suppl. 227 : 73 - 29) beschreiben, dass Assays zur Bestimmung der Kollagen-Crosslinker Pyridinolin und Deoxypyridinolin für Blut- und Urinproben und Assays zur Bestimmung der freien Crosslinkermenge und der Gesamt-Crosslinkermenge aus Urinproben entwickelt worden sind.

WO 2005/024409 beschreibt Vorrichtungen und Verfahren zur quantitativen Auswertung der in einer Körperflüssigkeitsprobe enthaltenen Polypeptide sowie Marker zur Erkennung von pathologischen Zuständen. Hier wird beispielsweise gezeigt, wie verschiedene Krankheiten mit den Vorkommen verschiedener Polypeptidmarker verbunden sind. Hierbei wird beobachtet, dass verschiedene Polypeptide bei gewissen Erkrankungen häufiger oder seltener auftreten als bei gesunden Patienten.

WO 2006/106129 beschreibt unter anderem sogenannte Amplitudenmarker, bei denen sich zwei Zustände, beispielsweise Gesund- und Krankheit, nicht durch das Vorkommen, sondern durch die Amplitude, d.h. die Konzentration der Marker in der Probe unterscheiden. Um Unterschiede in der Proteinkonzentration verschiedener Proben auszugleichen, wird in der WO 2006/106129 nach einer massenspektrometrischen Untersuchung der Probe die gesamte Amplitude auf 1 Mio. Counts normiert.

Es besteht jedoch weiterhin einen Bedarf nach spezifischeren Normierungsverfahren, um unterschiedliche Konzentrationen von. Analyten in der Probe zu vergleichen und daher eine bessere Vergleichbarkeit zwischen Referenzproben und gemessenen Proben zu erreichen.

Überraschenderweise kann die Aufgabe gelöst werden durch ein Verfahren zur Normierung der Konzentration mindestens eines Analyten in einer Urinprobe umfassend folgende Schritte:
a) Bestimmung der Konzentration mindestens eines Analyten,
b) Bestimmung der Konzentration mindestens eines Kollagens oder Kollagenfragmentes aus der Urinprobe,
c) Normierung der Konzentration des mindestens-einen Analyten relativ zur Konzentration des mindestens einen Kollagens oder Kollagenfragmentes.

Das erfindungsgemäße Verfahren ist vielfältig anwendbar.

Zunächst erfolgt die Bestimmung- des Gehaltes an Analyten, Kollagen oder Kollagenfragmenten. Zur Bestimmung können beispielsweise UV/VIS-Detektoren, Fluoreszenz-Detektoren, Brechungsindexdetektoren, Diodenarray-Detektoren, Massenspektrometer, Gelscanner die Färbung der Proteine z.B. mit Coommassie Brillant Blue, Silberfärbung, Cy2, Cy3, Cy5, oder Ahnlichem oder die Derivatisierung z.B. mit ITRAC (isotope tags for relative and absolute qualification) und ICAT (Isotope Coded Affinity Tagging) und anschließende Detektion der derivatisierten Peptide verwendet werden.

Zur Normierung der Proben werden erfindungsgemäß Kollagen oder Kollagenfragmente herangezogen. Hierzu wird mindestens ein Kollagen oder Kollagenfragment in der Probe identifiziert werden kann. Hierzu eignet sich insbesondere das Identifizieren über Migrations- oder Retentionszeiten, gegebenenfalls ergänzt durch Massenspektren, UV/VIS-Spektren, Fluoreszenzspektren oder die Affinität zu bestimmten Liganden, beispielsweise spezifischen Antikörpern.

Die Konzentration des Analyten kann dann auf die Konzentration des mindestens einen Kollagens oder Kollagenfragmentes normiert werden, d.h. die Konzentration an Analyten werden relativ zur Konzentration an den herangezogenen Kollagenen oder Kollagenfragmenten gemessen.

Bevorzugt wird jedoch zur Normierung nicht ein Kollagen oder Kollagenfragment eingesetzt, sondern mehrere, beispielsweise zwei oder drei oder vier oder fünf oder 10, 20, 30 40 oder 50 Kollagene bzw. Kollagenfragmente.

In einer bevorzugten Ausführungsform umfasst der Analyt keine Hydroxypyridinium Collagen Crosslinks, insbesondere kein Hydroxylysylpyridinolin (Pyd) und Deoxypyridinolin (Dpd). Bevorzugt wird auch keine diese Verbindung zur Normierung der Konzentration eines anderen Analyten eingesetzt.

Bevorzugt verwendete Kollagenfragmente sind in der folgenden Tabelle aufgeführt:

| Seq. ID Nr. | Protein name | Swissprot name | Accession number | Calc. mass [M] |
|---|---|---|---|---|
| 1 | Collagen alpha-1 (I) chain [777-787] [Homo sapiens] | CO1A1 Human | gi115269 | 1016,441 |
| 2 | Collagen alpha-1 (I) chain [546-558] [Homo sapiens] | CO1A1 Human | gi115269 | 1194,552 |
| 3 | Collagen alpha-1 (I) chain [798-810] [Homo sapiens] | CO1A1 Human | gi115269 | 1250,553 |
| 4 | Collagen alpha-1 (I) chain [546-559] [Homo sapiens] | CO1A1 Human | gi115269 | 1265,589 |
| 5 | Collagen alpha-1 (III) chain [796-808] [Homo sapiens] | CO3A1 Human | gi115306 | 1268,563 |
| 6 | Collagen alpha-1 (II) chain [511-524] [Homo sapiens] | CO2A1 Human | gi115287 | 1378,611 |
| 7 | Collagen alpha-1 (I) chain [543-558] [Homo sapiens] | CO1A1 Human | gi115269 | 1435,658 |
| 8 | Collagen alpha-1 (III) chain [642-657] [Homo sapiens] | CO3A1 Human | gi115306 | 1438,669 |
| 9 | Collagen alpha-1 (I) chain [543-558] [Homo sapiens] | CO1A1 Human | gi115269 | 1451,653 |
| 10 | Collagen alpha-1 (I) chain [542-558] [Homo sapiens] | CO1A1 Human | gi115269 | 1508,674 |
| 11 | Collagen alpha-1 (XV) chain [1093-1109] [Homo sapiens] | COFA1 Human | gi68839886 | 1523,726 |
| 12 | Collagen alpha-1 (I) chain [543-560] [Homo sapiens] | CO1A1 Human | gi115269 | 1579,711 |
| 13 | Collagen alpha-1 (III) chain [587-604] [Homo sapiens] | CO3A1 Human | gi115306 | 1623,724 |
| 14 | Collagen alpha-1 (I) chain [541-560] [Homo sapiens] | CO1A1 Human | gi115269 | 1737,781 |
| 15 | Collagen alpha-1 (III) chain [642-661] [Homo sapiens] | CO3A1 Human | gi115306 | 1834,833 |
| 16 | Collagen alpha-1 (III) chain [1174-1195] [Homo sapiens] | CO3A1 Human | gi115306 | 2025,866 |
| 17 | Collagen alpha-1 (I) chain [648-669] [Homo sapiens] | CO1A1 Human | gi115269 | 2046,949 |
| 18 | Collagen alpha-1 (I) chain [229-249] [Homo sapiens] | CO1A1 Human | gi115269 | 2063,926 |
| 19 | Collagen alpha-1 (III) chain [586-604] [Homo sapiens] | CO3A1 Human | gi115306 | 2078,925 |
| 20 | Collagen alpha-1 (I) chain [647-669] [Homo sapiens] | CO1A1 Human | gi115269 | 2087,976 |
| 21 | Collagen alpha-1 (I) chain [647-669] [Homo sapiens] | CO1A1 Human | gi115269 | 2103,971 |
| 22 | Collagen alpha-1 (I) chain [646-669] [Homo sapiens] | CO1A1 Human | gi115269 | 2159,013 |
| 23 | Collagen alpha-1 (I) chain [432-455] [Homo sapiens] | CO1A1 Human | gi115269 | 2169,977 |
| 24 | Collagen alpha-1 (I) chain [646-669] [Homo sapiens] | CO1A1 Human | gi115269 | 2175,008 |
| 25 | Collagen alpha-1 (I) chain [819-843] [Homo sapiens] | CO1A1 Human | gi115269 | 2220,988 |
| 26 | Collagen alpha-1 (I) chain [431-455] [Homo sapiens] | CO1A1 Human | gi115269 | 2226,999 |
| 27 | Collagen alpha-1 (I) chain [699-725] [Homo sapiens] | CO1A1 Human | gi115269 | 2281,979 |
| 28 | Collagen alpha-1 (I) chain [819-844] [Homo sapiens] | CO1A1 Human | gi115269 | 2292,025 |
| 29 | Collagen alpha-1 (III) chain [813-840] [Homo sapiens] | CO3A1 Human | gi115306 | 2564,149 |
| 30 | Collagen alpha-1 (III) chain [1168-1195] [Homo sapiens] | CO3A1 Human | gi115306 | 2679,202 |
| 31 | Collagen alpha-1 (III) chain [1168-1195] [Homo sapiens] | CO3A1 Human | gi115306 | 2695,197 |
| 32 | Collagen alpha-1 (III) chain [610-639] [Homo sapiens] | CO3A1 Human | gi115306 | 2742,248 |
| 33 | Collagen alpha-1 (III) chain [448-477] [Homo sapiens] | CO3A1 Human | gi115306 | 2825,270 |
| 34 | Collagen alpha-1 (I) chain [1011-1041] [Homo sapiens] | CO1A1 Human | gi115269 | 2942,299 |
| 35 | Collagen alpha-1 (I) chain [537-569] [Homo sapiens] | CO1A1 Human | gi115269 | 3011,386 |
| 36 | Collagen alpha-1 (I) chain [1011-1040] [Homo sapiens] | CO1A1, Human | gi115269 | 3013,336 |
| 37 | Collagen alpha-2 (I) chain [830-865] [Homo sapiens] | CO1A2 Human | gi82654930 | 3248,533 |
| 38 | Collagen alpha-1 (I) chain [1007-1041] [Homo sapiens] | CO1A1 Human | gi115269 | 3266,442 |
| 39 | Collagen alpha-1 (III) chain [910-948] [Homo sapiens] | CO3A1 Human | gi115306 | 3441,603 |
| 40 | Collagen alpha-1 (I) chain [630-674] [Homo sapiens] | CO1A1 Human | gi115269 | 4169,916 |
| 41 | Collagen alpha-1 (III) chain [319-366] [Homo sapiens] | CO3A1 Human | gi115306 | 4289,919 |
| 42 | Collagen alpha-1 (III) chain [319-366] [Homo sapiens] | CO3A1 Human | gi115306 | 4305,914 |

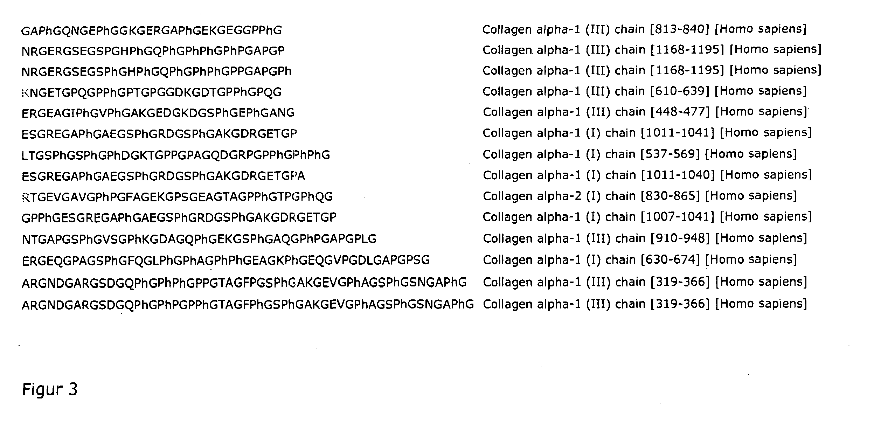

Die Sequenzen sind in Figur 3 angegeben.

Gegenstand der Erfindung ist auch die Verwendung von Kollagenen oder Kollagenfragmenten zur Normierung der Analytkonzentration von Urinproben, wobei die Bestimmung der Konzentration der Kollagene oder Kollagen- fragmente aus Urinprobe erfolgt, insbesondere mit den Kollagenfragmenten, wie sie oben beschrieben sind.

Bevorzugte Analyten, deren Gehalt normiert werden sollen, sind insbesondere Proteine, Peptide, Lipide, Metabolite, Salze und andere ionische Verbindungen, Kohlenhydrate.

Der Einsatz des Verfahrens zur Normierung von Peptidkonzentrationen ist besonders bevorzugt.

In einer Ausführungsform wird die Probe zunächst in Teilproben aufgetrennt.

Hierzu können verschiedene Verfahren eingesetzt werden, beispielsweise
- chromatographische Verfahren wie Affinitätschromatographie, Ionenaustauschchromatographie, reversed phase Chromatographie, Hydrophobe Interaktionschromatographie, Gelfiltration gegebenenfalls unter Verwendung von FPLC oder HPLC;
- Adsorption an bestimmte Materialien wie SELDI-Verfahren, ClinProt Verfahren,
- elektrophoretische Verfahren wie Gel-Elektrophorese, oder Kapillarelektrophorese.

Die Erfindung wird durch die nachfolgenden Beispiele näher erläutert:

### Beispiel 1

Eine Urinprobe wurde mittels HPLC gefolgt von einem Diodenarraydetektor (DAD) untersucht. Die Rohdaten sind in der Tabelle wiedergegeben. Peaks wurden mittels Retentionszeit und Spektren im DAD identifiziert. Die Messung wurde mit vorher bestimmten Standards für diese vier Kollagenfragmente verglichen und jeweils ein Umrechnungsfaktor bestimmt. Der Durchschnitt aus diesen Faktoren (hier 0,80761277) wurde als Normierungsfaktor für alle anderen Signale eingesetzt. Die normierten Signale sind in der letzten Spalte aufgeführt.

| Peak Nr. | Signal | Standard | Normierungsfaktor | Normierte Signalintensität |
|---|---|---|---|---|
| 1 | 126,8033157 | | | 157,0100429 |
| 2 | 146,5503894 | | | 181,4612086 |
| 3 | **3115,519875** | **4073,54509** | **0,76481782** | 3857,690207 |
| 4 | 590,1256269 | | | 730,7036846 |
| 5 | 5461,634925 | | | 6762,690148 |
| 6 | 150,3823428 | | | 186,2059991 |
| 7 | 39,1780827 | | | 48,51097474 |
| 8 | 8,5271778 | | | 10,55849798 |
| 9 | 394,6322538 | | | 488,6404331 |
| 10 | 50,0342319 | | | 61,9532451 |
| 11 | 1577,919488 | | | 1953,807005 |
| 12 | 539,3141163 | | | 667,7879996 |
| 13 | 400,4296182 | | | 495,8188292 |
| 14 | 8,8306578 | | | 10,93427212 |
| 15 | 148,7699163 | | | 184,209465 |
| 16 | 538,5410307 | | | 666,8307518 |
| 17 | 1389,860945 | | | 1720,949688 |
| 18 | 93,4206021 | | | 115,6749937 |
| 19 | 1**024,846846** | **1276,56782** | **0,80281426** | 1268,982963 |
| 20 | 2344,443075 | | | 2902,929673 |
| 21 | 435,1965894 | | | 538,86789 |
| 22 | 114,2890587 | | | 141,514675 |
| 23 | 15,9397308 | | | 19,73684839 |
| 24 | 8,9629632 | | | 11,09809494 |
| 25 | 398,4975981 | | | 493,4265688 |
| 26 | **6940,8252** | **8643,25876** | **0,80303337** | 8594,248947 |
| 27 | 76,7856591 | | | 95,07732162 |
| 28 | 61,2618498 | | | 775,85547438 |
| 29 | 64,5422931 | | | 79,9173756 |
| 30 | 559,1427462 | | | 692,3401497 |
| 31 | 323,2918818 | | | 400,3055594 |
| 32 | 14,1058476 | | | 17,46610272 |
| 33 | 399,1654701 | | | 494,2535393 |
| 34 | 4612,278375 | | | 5711,002284 |
| 35 | 174,3985782 | | | 215,9433142 |
| 36 | 136,4525784 | | | 168,9579257 |
| 37 | 909,9711288 | | | 1126,741877 |
| 38 | 62,1722196 | | | 76,9827099 |
| 39 | 119,8605303 | | | 148,4133668 |
| 40 | **2922,828975** | **3399,48571** | **0,85978563** | 3619,097026 |
| 41 | 2,625588 | | | 3,251048147 |
| 42 | 52,8236532 | | | 65,40715445 |
| 43 | 178,5294621 | | | 221,0582456 |
| | | | | |
| | | | 0,80761277 | |

### Beispiel 2

Eine Urinprobe wurde mittels 2D-Elektrophorese aufgetrennt wie in Thongboonkerd et al. in Kidney Int 62:1461-1469 (2002) beschrieben, über einen pH-Bereich von 3-10 und unter Verwendung eines 22 cm langen 10% Polyacrylamid Gels durchgeführt. Das Gel wurde mit Coommassie gefärbt, und die Kollagene wurden in einem parallel angefertigten Gel mittels immunologischer Methoden (Western Blot) detektiert. Vorbestimmten Standards für diese fünf Proteine wurden eingesetzt und eine lineare Regression durchgeführt. Mit dieser Kalibrationsformel wurden die weiteren Werte umgerechnet. Die Formel hatte den Wert Y=103,82x - 127449. Der R² Wert betrugt 0,957.

| Peak Nr. | Signal | Standard | Normierte Signalintensität |
|---|---|---|---|
| 1 | 4508518,988 | | 467946992,3 |
| 2 | 153935103 | | 15981414944 |
| 3 | 23582568 | | 2448214761 |
| 4 | 44251661,9 | | 4594080090 |
| 5 | 586248,746 | | 60736895,81 |
| 6 | 948640,866 | | 98360445,71 |
| 7 | 1383514,774 | | 143509054,8 |
| 8 | 122812941 | | 12750312086 |
| 9 | 2944789,572 | | 305600604,4 |
| 10 | 30550952,65 | | 3171672456 |
| 11 | **1089289,56** | 117284244,8 | 112962593,1 |
| 12 | 577112,586 | | 59788379,68 |
| 13 | 1130932,024 | | 117285913,7 |
| 14 | 913664,024 | | 94729149,97 |
| 15 | 397773,048 | | 41169348,84 |
| 16 | 6931703,714 | | 719522030,6 |
| 17 | 38342270,25 | | 3980567048 |
| 18 | 2391089,672 | | 248115480,7 |
| 19 | 2250316,422 | | 233500401,9 |
| 20 | 932726,014 | | 96708165,77 |
| 21 | 10695075,82 | | 1110235323 |
| 22 | 1438795,038 | | 149248251,8 |
| 23 | 8548926,298 | | 887422079,3 |
| 24 | 7982762,024 | | 828642904,3 |
| 25 | **519540,4375** | 58011688,21 | 53811239,22 |
| 26 | 4877082,25 | | 506211230,2 |
| 27 | 27564764,65 | | 2861646417 |
| 28 | 20622151,95 | | 2140864367 |
| 29 | 3862566,202 | | 400884174,1 |
| 30 | **219714,4865** | 23759647,58 | 22683308,99 |
| 31 | 13240495,55 | | 1374500799 |
| 32 | 6102716,5 | | 633456578 |
| 33 | 3523566,41 | | 365689215,7 |
| 34 | 1011312,708 | | 104867036,3 |
| 35 | 10681983,25 | | 1108876052 |
| 36 | 390792,574 | | 40444636,03 |
| 37 | 3552944,744 | | 368739274,3 |
| 38 | 8037397,154 | | 834315123,5 |
| 39 | 1966378,988 | | 204022017,5 |
| 40 | 89404658,25 | | 9281864171 |
| 41 | 2051887,75 | | 212899537,2 |
| 42 | 7424138,214 | | 770646580,4 |
| 43 | 32377626,4 | | 3361317724 |
| 44 | 34631829 | | 3595349038 |
| 45 | 1948609,006 | | 202177138 |
| 46 | 107055624,3 | | 11114387461 |
| 47 | 10008414,75 | | 1038946170 |
| 48 | 3575878,756 | | 371120283,4 |
| 49 | 1359764,006 | | 141043250,1 |
| 50 | **631532,685** | 52349499,17 | 65438274,36 |
| 51 | 1981878,154 | | 205631140,9 |
| 52 | 6658241,834 | | 691131218,2 |
| 53 | 8132593,25 | | 844198382,2 |
| 54 | 5853332,798 | | 607565562,1 |
| 55 | 10995087,2 | | 1141382504 |
| 56 | 3309612,762 | | 343476548 |
| 57 | 5692416,786 | | 590859261,7 |
| 58 | 3166113,976 | | 328578504 |
| 59 | 20333775,95 | | 2110925170 |
| 60 | 32900793,65 | | 3415632948 |
| 61 | 5041336,916 | | 523264149,6 |
| 62 | 2926167,976 | | 303667310,3, |
| 63 | 10445490,05 | | 1084323328 |
| 64 | 775850,282 | | 80421327,28 |
| 65 | 339214,044 | | 35089753,05 |
| 66 | 41363211,15 | | 4294201133 |
| 67 | 23456220,45 | | 2435097358 |
| 68 | 15788615 | | 1639046560 |
| 69 | 34321605,15 | | 3563141598 |
| 70 | 897196,084 | | 93019448,44 |
| 71 | **327241,543** | 37343483,31 | 33846767,99 |
| 72 | 9414104,178 | | 977244846,8 |
| 73 | 1965153,274 | | 203894763,9 |
| 74 | 45057999,65 | | 4677794075 |
| 75 | 877939,968 | | 91020278,48 |
| 76 | 3120269,268 | | 323818906,4 |
| 77 | 3584994,964 | | 372066728,2 |
| 78 | 10224568,98 | | 1061387302 |
| 79 | 735893,154 | | 76272978,25 |
| 80 | 5907069,334 | | 613144489,3 |
| 81 | 36156493,15 | | 3753639670 |
| 82 | 10877155,98 | | 1129138884 |
| 83 | 8374707,928 | | 869334728,1 |
| 84 | 16689498,2 | | 1732576254 |
| 85 | 5895062,464 | | 611897936 |
| 86 | 1706771,568 | | 177069575,2 |

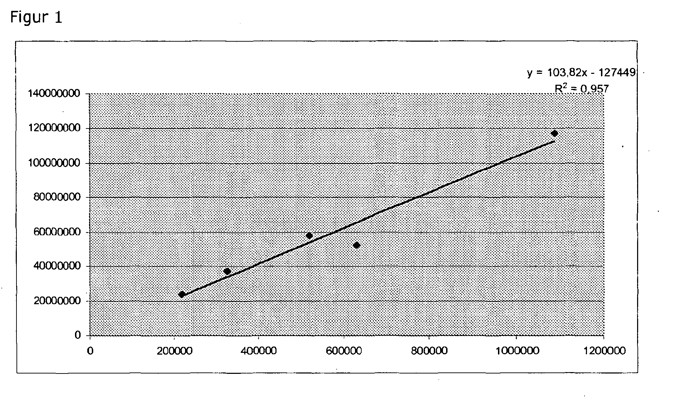

Die Qualität der Regression ist in Figur 1 dargestellt.

### Beispiel 3

Eine Urinprobe wurde mittels Kappilarelektrophorese gekoppelt mit Massenspektrometrie untersucht. Hierbei konnten 27 Kollagenfragmente identifiziert werden, die mit vorher bestimmten Standards verglichen wurden. Es erfolgte eine Normierung mittels linearer Regression mit der Maßgabe, dass die Gerade durch den 0 Wert laufen muss. Entsprechende Messdaten und die Kalibrierung sind in der folgenden Tabelle dargestellt.

| Nummer | CE-Zeit (min) | Masse | Signal | Standard | Normierte Signalintensität |
|---|---|---|---|---|---|
| 1 | 19,948 | 17266,043 | 184882,922 | | 1368,13362 |
| 2 | 19,944 | 17119,789 | 587371,188 | | 4346,54679 |
| 3 | 19,931 | 17062,977 | 240560 | | 1780,144 |
| 4 | 19,946 | 16972,203 | 352392,875 | | 2607,70728 |
| 5 | 19,908 | 16952,465 | 197797,906 | | 1463,7045 |
| 6 | 19,931 | 16918,75 | 2107725,75 | | 15597,1706 |
| 7 | 19,873 | 16806,326 | 124057,313 | | 918,024116 |
| 8 | 19,917 | 16771,715 | 1365796,63 | | 10106,895 |
| 9 | 22,387 | 10342,543 | 119401,375 | | 883,570175 |
| 10 | 20,699 | 9905,148 | 89822,563 | | 664,686966 |
| 11 | 20,727 | 9867,076 | 526946,5 | | 3899,4041 |
| 12 | 20,599 | 9626,376 | 176806,484 | | 1308,36798 |
| 13 | 20,904 | 8875,947 | 124966,711 | | 924,753661 |
| 14 | 20,927 | 8837,85 | 796843,125 | | 5896,63913 |
| 15 | 19,926 | 8578,571 | 17265,779 | | 127,766765 |
| 16 | 20,156 | 8289,725 | 286028,781 | | 2116,61298 |
| 17 | 20,078 | 8176,791 | 674227,938 | | 4989,28674 |
| 18 | 57,332 | 6788,82 | 438,533 | | 3,2451442 |
| 19 | 19,251 | 6491,179 | 95395,688 | | 705,928091 |
| 20 | 23,865 | 6329,103 | 62460,586 | | 462,208336 |
| 21 | 19,542 | 6308,096 | 23674,088 | | 175,188251 |
| 22 | 20,842 | 6237,104 | 134147,797 | | 992,693698 |
| 23 | 23,72 | 6225,298 | 67887,742 | | 502,369291 |
| 24 | 23,66 | 6208,243 | 85896,305 | | 635,632657 |
| 25 | 23,741 | 6186,583 | 288461,313 | | 2134,61372 |
| 26 | 23,643 | 6170,012 | 399146,313 | | 2953,68272 |
| 27 | 19,662 | 5861,38 | 218662,969 | | 1618,10597 |
| 28 | 22,258 | 5635,462 | 14248,398 | | 105,438145 |
| 29 | 22,266 | 5612,497 | 112633,008 | | 833,484259 |
| 30 | 22,301 | 5574,535 | 988766,188 | | 7316,86979 |
| 31 | 20,377 | 5118,564 | 18909,668 | | 139,931543 |
| 32 | 20,441 | 5096,631 | 93874,516 | | 694,671418 |
| 33 | 20,448 | 5058,56 | 250174,453 | | 1851,29095 |
| 34 | 23,737 | 5039,495 | 283460,031 | | 2097,60423 |
| 35 | 22,982 | 5038,431 | 174797,016 | | 1293,49792 |
| 36 | 20,431 | 5036,265 | 628288,625 | | 4649,33583 |
| 37 | 23,001 | 5023,362 | 43507,188 | | 321,953191 |
| 38 | 20,438 | 5020,505 | 641471,938 | | 4746,89234 |
| 39 | 23,009 | 5000,172 | 753943,563 | | 5579,18237 |
| 40 | 20,45 | 4998,592 | 2524795 | | 18683,483 |
| 41 | 20,409 | 4982,02 | 313462,719 | | 2319,62412 |
| 42 | 20,549 | 4976,683 | 1167032,75 | | 8636,04235 |
| 43 | 52,301 | 4964,782 | 358,613 | | 2,6537362 |
| 44 | 20,466 | 4960,742 | 14400323 | | 106562,39 |
| 45 | 20,358 | 4933,646 | 223247,875 | | 1652,03428 |
| 46 | 22,651 | 4876,404 | 51850,109 | | 383,690807 |
| 47 | 38,197 | 4861,141 | 494,304 | | 3,6578496 |
| 48 | 22,682 | 4838,021 | 281694,156 | | 2084,53675 |
| 49 | 22,655 | 4822,251 | 52409,906 | | 387,833304 |
| 50 | 22,683 | 4800,063 | 1058541,25 | | 7833,20525 |
| 51 | 48,573 | 4793,838 | 282,485 | | 2,090389 |
| 52 | 55,222 | 4777,634 | 374,187 | | 2,7689838 |
| 53 | 39,763 | 4774,006 | 532,92 | | 3,943608 |
| 54 | 48,826 | 4772,17 | 272,009 | | 2,0128666 |
| 55 | 57,032 | 4766,609 | 339,383 | | 2,5114342 |
| 56 | 19,62 | 4730,656 | 24256,555 | | 179,498507 |
| 57 | 37,899 | 4719,636 | 2192,44 | | 16,224056 |
| 58 | 23,902 | 4692,252 | 68363,453 | | 505,889552 |
| 59 | 54,464 | 4690,256 | 358,075 | | ,649755 |
| 60 | 22,332 | 4671,967 | 96398,766 | | 713,350868. |
| 61 | 25,889 | 4668,661 | 32946,504 | | 243,80413 |
| 62 | 21,078 | 4656,171 | 37012,164 | | 273,890014 |
| 63 | 23,915 | 4654,204 | 252635,781 | | 1869,50478 |
| 64 | 19,366 | 4651,778 | 422,893 | | 3,1294082 |
| 65 | 25,936 | 4630,038 | 76921,227 | | 569,21708 |
| 66 | 54,567 | 4620,673 | 895,427 | | 6,6261598 |
| 67 | 23,396 | 4619,578 | 16829,051 | | 124,534977 |
| 68 | 52,701 | 4602,985 | 298,8 | | 2,21112 |
| 69 | 58,14 | 4602,554 | 423,734 | | 3,1356316 |
| 70 | 57,637 | 4594,426 | 391,032 | | 2,8936368 |
| 71 | 52,603 | 4577,292 | 340,97 | | 2,523178 |
| 72 | 20,741 | 4566,496 | 232446,344 | | 1720,10295 |
| 73 | 49,024 | 4560,303 | 642,982 | | 4,7580668 |
| 74 | 54,011 | 4559,723 | 4928,111 | | 36,4680214 |
| 75 | 18,86 | 4552,372 | 345,112 | | 2,5538288 |
| 76 | 51,846 | 4548,482 | 373,052 | | 2,7605848 |
| 77 | 26,066 | 4547,384 | 12106,5 | | 89,5881 |
| 78 | 51,365 | 4544,345 | 1851,577 | | 13,7016698 |
| 79 | 53,986 | 4543,723 | 22582,416 | | 167,109878 |
| 80 | 54,024 | 4520,931 | 4122,547 | | 30,5068478 |
| 81 | 40,87 | 4520,884 | 552,198 | | 4,0862652 |
| 82 | 25,763 | 4468,206 | 33454,656 | | 247,564454 |
| 83 | 25,839 | 4452,583 | 21824,857 | | 161,503942 |
| 84 | 48,976 | 4445,646 | 571,726 | | 4,2307724 |
| 85 | 20,501 | 4443,026 | 18179,678 | | 134,529617 |
| 86 | 27,642 | 4439,813 | 97056,883 | | 718,220934 |
| 87 | 24,137 | 4436,181 | 404617,531 | | 2994,16973 |
| 88 | 58,694 | 4420,792 | 432,338 | | 3,1993012 |
| 89 | 25,813 | 4414,273 | 15183,697 | | 112,359358 |
| 90 | 19,965 | 4410,955 | 338675,063 | | 2506,19547 |
| 91 | 20,515 | 4405,046 | 291842,344 | | 2159,63335 |
| 92 | 25,558 | 4404,052 | 42032,641 | | 311,041543 |
| 93 | 20,055 | 4391,25 | 224259,906 | | 1659,5233 |
| 94 | 59,551 | 4390,04 | 363,251 | | 2,6880574 |
| 95 | 59,07 | 4383,307 | 677,349 | | 5,0123826 |
| 96 | 20,018 | 4375,046 | 105928,648 | | 783,871995 |
| 97 | 25,571 | 4366,142 | 216682,813 | | 1603,45282 |
| 98 | 20,066 | 4353,102 | 1819283,25 | | 13462,6961 |
| 99 | 22,383 | 4349,45 | 216404,641 | | 1601,39434 |
| 100 | 25,569 | 4344,106 | 288232,313 | | 2132,91912 |
| 101 | 20,337 | 4334,652 | 45377,477 | | 335,79333 |
| 102 | 25,603 | 4328,157 | 542624 | | 4015,4176 |
| 103 | 60,458 | 4319,592 | 330,163 | | 2,4432062 |
| 104 | 27,298 | 4307,342 | 30545,982 | | 226,040267 |
| 105 | **25,614** | **4306,212** | **529838,313** | 3100,93516 | 3920,80352 |
| 106 | 18,866 | 4302,308 | 277,252 | | 2,0516648 |
| 107 | **25,618** | **4290,197** | **708976,625** | 3872,09443 | 5246,42703 |
| 108 | 25,58 | 4274,179 | 57546,574 | | 425,844648 |
| 109 | 25,607 | 4252,228 | 98721,375 | | 730,538175 |
| 110 | 23,903 | 4239,119 | 31304,807 | | 231,655572 |
| 111 | 20,202 | 4233,57 | 31109,875 | | 230,213075 |
| 112 | 25,741 | 4229,222 | 27980,471 | | 207,055485 |
| 113 | 23,93 | 4217,721 | 698545,563 | | 5169,23717 |
| 114 | 28,392 | 4208,093 | 85557,367 | | 633,124516 |
| 115 | 20,629 | 4196,917 | 227998,453 | | 1687,18855 |
| 116 | 28,393 | 4192,087 | 152065,688 | | 1125,28609 |
| 117 | 27,019 | 4180,957 | 29489,236 | | 218,220346 |
| 118 | **28,396** | **4170,166** | **176045,016** | 1291,45231 | 1302,73312 |
| 119 | 23,8 | 4160,604 | 154588,094 | | 1143,9519 |
| 120 | 22,532 | 4160,021 | 318391,75 | | 2356,09895 |
| 121 | 27,033 | 4160,011 | 69551,938 | | 514,684341 |
| 122 | 28,349 | 4154,089 | 35176,504 | | 260,30613 |
| 123 | 27,009 | 4144,035 | 62706,73 | | 464,029802 |
| 124 | 22,513 | 4144,034 | 67626,914 | | 500,439164 |
| 125 | 23,294 | 4135,927 | 40501,199 | | 299,708873 |
| 126 | 22,526 | 4122,078 | 1111014,88 | | 8221,51008 |
| 127 | 27,052 | 4121,97 | 143200,75 | | 1059,68555 |
| 128 | 26,993 | 4106,017 | 56078,742 | | 414,982691 |
| 129 | 20,242 | 4105,763 | 27811,58 | | 205,805692 |
| 130 | 28,055 | 4102,563 | 18823,371 | | 139,292945 |
| 131 | 27,022 | 4084,008 | 103050,93 | | 762,576882 |
| 132 | 28,057 | 4082,969 | 35312,172 | | 261,310073 |
| 133 | 28,103 | 4063,103 | 70996,594 | | 525,374796 |
| 134 | 20,494 | 4062,357 | 192299,219 | | 1423,01422 |
| 135 | 20,46 | 4046,127 | 45463,988 | | 336,433511 |
| 136 | 20,849 | 4044,743 | 40488,367 | | 299,613916 |
| 137 | 28,08 | 4044,394 | 166345,453 | | 1230,95635 |
| 138 | 20,276 | 4044,247 | 575594,75 | | 4259,40115 |
| 139 | 20,865 | 4028,943 | 54097,566 | | 400,321988 |
| 140 | 20,601 | 4025,685 | 273715,43838 | | 2025,49424 |
| 141 | 28,152 | 4025,156 | 75278,781 | | 557,062979 |
| 142 | 25,055 | 4015,3 | 13494,786 | | 99,8614164 |
| 143 | 20,423 | 4009,552 | 46446,355 | | 343,703027 |
| 144 | 20,88 | 4007,199 | 241439,844 | | 1786,65485 |
| 145 | 28,076 | 4006,013 | 98433,141 | | 728,405243 |
| 146 | 20,719 | 3996,902 | 309862,719 | | 2292,98412 |
| 147 | 20,438 | 3986,617 | 1697006,63 | | 12557,849 |
| 148 | 20,881 | 3968,808 | 427206,25 | | 3161,32625 |
| 149 | 19,8 | 3960,04 | 129275,078 | | 956,635577 |
| 150 | 19,704 | 3913,814 | 25934,791 | | 191,917453 |
| 151 | 19,645 | 3900,239 | 29670,904 | | 219,56469 |
| 152 | 19,631 | 3877,959 | 85786,75 | | 634,82195 |
| 153 | 19,619 | 3861,996 | 59334,113 | | 439,072436 |
| 154 | 23,401 | 3860,224 | 31707,752 | | 234,637365 |
| 155 | 19,105 | 3856,541 | 110644,117 | | 818,766466 |
| 156 | 28,332 | 3839,987 | 28982,951 | | 214,473837 |
| 157 | 19,659 | 3839,739 | 105607,102 | | 781,492555 |
| 158 | 25,36 | 3832,015 | 15375,597 | | 113,779418 |
| 159 | 23,419 | 3822,288 | 21333,066 | | 157,864688 |
| 160 | 20,048 | 3814,276 | 379764,75 | | 2810,25915 |
| 161 | 28,373 | 3801,977 | 68342,133 | | 505,731784 |
| 162 | 27,677 | 3801,896 | 13939,659 | | 103,153477 |
| 163 | 27,719 | 3794,861 | 51831,453 | | 383,552752 |
| 164 | 21,453 | 3782,819 | 17585,135 | | 130,129999 |
| 165 | 31,428 | 3775,772 | 53642,129 | | 396,951755 |
| 166 | 27,744 | 3772,916 | 129047,891 | | 954,954393 |
| 167 | 21,446 | 3760,889 | 121781,781 | | 901,185179 |
| 168 | 19,224 | 3760,068 | 42083,434 | | 311,417412 |
| 169 | 21,836 | 3757,834 | 12074,36 | | 89,350264 |
| 170 | 27,7 | 3756,982 | 327179,375 | | 2421,12738 |
| 171 | 21,886 | 3741,82 | 31431,242 | | 232,591191 |
| 172 | 27,715 | 3739,881 | 56333,398 | | 416,867145 |
| 173 | 27,678 | 3735,445 | 121638,703 | | 900,126402 |
| 174 | 19,018 | 3729,583 | 19175,627 | | 141,89964 |
| 175 | 21,454 | 3722,747 | 1143063,38 | | 8458,66898 |
| 176 | 21,905 | 3720,201 | 182391,875 | | 1349,69988 |
| 177 | 27,69 | 3718,93 | 211491,453 | | 1565,03675 |
| 178 | 19,466 | 3710,955 | 28338,004 | | 209,70123 |
| 179 | 21,427 | 3706,46 | 187850,703 | | 1390,0952 |
| 180 | 21,882 | 3703,562 | 156398,141 | | 1157,34624 |
| 181 | 27,706 | 3702,282 | 83424,43 | | 617,340782 |
| 182 | 27,527 | 3699,89 | 27447,908 | | 203,114519 |
| 183 | 33,259 | 3695,777 | 61599,129 | | 455,833555 |
| 184 | 21,69 | 3686,37 | 179506,266 | | 1328,34637 |
| 185 | 31,253 | 3674,799 | 30919,297 | | 228,802798 |
| 186 | 22,842 | 3669,826 | 17117,689 | | 126,670899 |
| 187 | 18,862 | 3662,674 | 745,133 | | 5,5139842 |
| 188 | 33,247 | 3657,754 | 144639,984 | | 1070,33588 |
| 189 | 21,345 | 3630,767 | 245992,188 | | 1820,34219 |
| 190 | 19,415 | 3620,771 | 139033,516 | | 1028,84802 |
| 191 | 28,076 | 3616,437 | 45247,488 | | 334,831411 |
| 192 | 19,346 | 3605,381 | 42433,102 | | 314,004955 |
| 193 | 26,967 | 3593,676 | 72137,609 | | 533,818307 |
| 194 | 19,373 | 3582,885 | 557225,625 | | 4123,46963 |
| 195 | 27,002 | 3571,638 | 158852,625 | | 1175,50943 |
| 196 | 27,423 | 3562,87 | 16845,193 | | 124,654428 |
| 197 | 23,372 | 3559,398 | 131749,125 | | 974,943525 |
| 198 | 22,789 | 3556,364 | 63784,465 | | 472,005041 |
| 199 | 26,97 | 3555,582 | 288436,906 | | 2134,4331 |
| 200 | 26,448 | 3538,979 | 67951,078 | | 502,837977 |
| 201 | 26,967 | 3533,677 | 606124,563 | | 4485,32177 |
| 202 | 27,421 | 3531,154 | 21793,037 | | 161,268474 |
| 203 | 24,129 | 3530,806 | 68264,656 | | 505,158454 |
| 204 | 27,416 | 3524,921 | 101030,172 | | 747,623273 |
| 205 | 26,508 | 3520,84 | 46995,484 | | 347,766582 |
| 206 | 27,773 | 3518,717 | 37848,461 | | 280,078611 |
| 207 | 26,936 | 3517,813 | 749714,938 | | 5547,89054 |
| 208 | 34,02 | 3515,52 | 7304,833 | | 544,0557642. |
| 209 | 27,065 | 3511,743 | 412161,094 | | 3049,9921 |
| 210 | 27,442 | 3509,717 | 25078,984 | | 185,584482 |
| 211 | 21,028 | 3504,793 | 29887,633 | | 221,168484 |
| 212 | 19,053 | 3501,893 | 21114,822 | | 156,249683 |
| 213 | 27,027 | 3495,682 | 2833316,75 | | 20966,544 |
| 214 | 27,411 | 3493,91 | 98902,625 | | 731,879425 |
| 215 | 27,681 | 3479,778 | 17606,455 | | 130,287767 |
| 216 | 26,998 | 3479,73 | 2020814,75 | | 14954,0292 |
| 217 | 27,076 | 3473,736 | 673718 | | 4985,5132 |
| 218 | 27,432 | 3471,466 | 127158,477 | | 940,97273 |
| 219 | 19,129 | 3462,563 | 197973,266 | | 1465,00217 |
| 220 | 22,439 | 3461,026 | 35283,965 | | 261,101341 |
| 221 | 27,678 | 3457,877 | 9517,13 | | 70,426762 |
| 222 | 27,023 | 3457,775 | 3209066 | | 23747,0884 |
| 223 | 31,081 | 3456,538 | 54283,066 | | 401,694688 |
| 224 | 27,408 | 3455,346 | 234864,938 | | 1738,00054 |
| 225 | 21,428 | 3449,671 | 27877,854 | | 206,29612 |
| 226 | **26,975** | **3441,772** | **1378114,63** | 7943,47051 | 10198,0482 |
| 227 | 23,78 | 3439,69 | 46195,805 | | 341,848957 |
| 228 | 33,995 | 3438,31 | 29525,637 | | 218,489714 |
| 229 | 27,307 | 3437,3 | 69127,938 | | 511,546741 |
| 230 | 27,418 | 3433,157 | 184062,516 | | 1362,06262 |
| 231 | 25,03 | 3426,535 | 31401,645 | | 232,372173 |
| 232 | 26,945 | 3425,419 | 241693,109 | | 1788,52901 |
| 233 | 39,51 | 3422,974 | 19537,455 | | 144,577167 |
| 234 | 21,122 | 3422,818 | 189054,766 | | 1399,00527 |
| 235 | 23,945 | 3422,32 | 323563,813 | | 2394,37222 |
| 236 | 27,356 | 3416,57 | 286254,313 | | 2118,28192 |
| 237 | 27,752 | 3409,818 | 70792,961 | | 523,867911 |
| 238 | 23,959 | 3404,934 | 516072,625 | | 3818,93743 |
| 239 | 23,771 | 3402,278 | 118509,031 | | 876,966829 |
| 240 | 22,537 | 3401,814 | 94982,938 | | 702,873741 |
| 241 | 27,278 | 3397,817 | 332921,625 | | 2463,62003 |
| 242 | 32,149 | 3390,576 | 31476,922 | | 232,929223 |
| 243 | 23,818 | 3385,656 | 511551,25 | | 3785,47925 |
| 244 | 26,67 | 3385,149 | 22069,303 | | 163,312842 |
| 245 | 30,445 | 3379,519 | 15737,849 | | 116,460083 |
| 246 | 47,154 | 3378,513 | 38705,031 | | 286,417229 |
| 247 | 32,345 | 3374,63 | 87858,953 | | 650,156252 |
| 248 | 22,155 | 3374,546 | 23420,533 | | 173,311944 |
| 249 | 32,06 | 3368,627 | 127597,813 | | 944,223816 |
| 250 | 26,668 | 3363,626 | 74247,625 | | 549,432425 |
| 251 | 27,298 | 3359,786 | 207612,594 | | 1536,3332 |
| 252 | 30,446 | 3357,522 | 68808,484 | | 509,182782 |
| 253 | 32,051 | 3352,655 | 181481,938 | | 1342,96634 |
| 254 | 19,112 | 3348,489 | 28785,637 | | 213,013714 |
| 255 | 26,668 | 3347,627 | 69073,266 | | 511,142168 |
| 256 | 30,435 | 3341,531 | 95088,156 | | 703,652354 |
| 257 | 21,994 | 3341,505 | 21804,309 | | 161,351887 |
| 258 | 47,117 | 3340,88 | 42485,211 | | 314,390561 |
| 259 | 22,583 | 3338,746 | 84856,977 | | 627,94163 |
| 260 | 22,108 | 3337,336 | 52467,945 | | 388,262793 |
| 261 | 19,069 | 3332,833 | 143378,078 | | 1060,99778 |
| 262 | 32,236 | 3330,681 | 741472,813 | | 5486,89882 |
| 263 | 19,595 | 3329,771 | 22509,027 | | 166,5668 |
| 264 | 22,011 | 3325,803 | 31233,105 | | 231,124977 |
| 265 | 26,671 | 3325,558 | 349720,469 | | 2587,93147 |
| 266 | 30,442 | 3319,545 | 242232,234 | | 1792,51853 |
| 267 | 47,101 | 3319,288 | 35056,176 | | 259,415702 |
| 268 | 22,112 | 3318,936 | 56471,188 | | 417,886791 |
| 269 | 32,133 | 3314,687 | 255600,891 | | 1891,44659 |
| 270 | 23,088 | 3311,52 | 14913,211 | | 110,357761 |
| 271 | 26,667 | 3308,661 | 233464,578 | | 1727,63788 |
| 272 | 47,562 | 3305,413 | 8993,222 | | 66,5498428 |
| 273 | 22,13 | 3303,866 | 168833,016 | | 1249,36432 |
| 274 | 26,694 | 3303,689 | 52945,629 | | 391,797655 |
| 275 | 30,428 | 3303,55 | 190189,688 | | 1407,40369 |
| 276 | 23,897 | 3302,688 | 21441,1 | | 158,66414 |
| 277 | 47,167 | 3302,666 | 31385,865 | | 232,255401 |
| 278 | 22,207 | 3296,58 | 29308,854 | | 216,88552 |
| 279 | 32,299 | 3292,687 | 1206767,88 | | 8930,08228 |
| 280 | 22,855 | 3290,677 | 116553,609 | | 862,496707 |
| 281 | 22,129 | 3287,533 | 19792,754 | | 146,46638 |
| 282 | 26,675 | 3286,98 | 664343,75 | | 4916,14375 |
| 283 | 30,451 | 3281,458 | 136281,25 | | 1008,48125 |
| 284 | 23,91 | 3280,708 | 71741,43 | | 530,886582 |
| 285 | 22,104 | 3280,6 | 132426,766 | | 979,958068 |
| 286 | 27,844 | 3278,421 | 54118,781 | | 400,478979 |
| 287 | 26,669 | 3271,229 | 201540,094 | | 1491,3967 |
| 288 | 21,895 | 3268,819 | 33018,098 | | 244,333925 |
| 289 | **22,073** | **3266,64** | **409110,469** | 2799,38997 | 3027,41747 |
| 290 | 47,665 | 3266,519 | 3664,112 | | 27,1144288 |
| 291 | 26,645 | 3264,684 | 39645,684 | | 293,378062 |
| 292 | 30,445 | 3264,57 | 33225,082 | | 245,865607 |
| 293 | 32,145 | 3261,544 | 154194,859 | | 1141,04196 |
| 294 | 33,87 | 3260,577 | 9901,048 | | 73,2677552 |
| 295 | 22,173 | 3258,623 | 204077,922 | | 1510,17662 |
| 296 | 27,787 | 3256,555 | 242475 | | 1794,315 |
| 297 | 19,366 | 3253,774 | 9140 | | 67,636 |
| 298 | 25,228 | 3252,451 | 40806,734 | | 301,969832 |
| 299 | **26,638** | **3248,195** | **345009,313** | 1768,79215 | 2553,06892 |
| 300 | 21,996 | 3247,551 | 279443,219 | | 2067,87982 |
| 301 | 30,051 | 3238,459 | 73868,313 | | 546,625516 |
| 302 | 21,997 | 3231,53 | 106226,703 | | 786,077602 |
| 303 | 21,23 | 3223,641 | 53736,922 | | 397,653223 |
| 304 | 32,12 | 3222,565 | 133680,984 | | 989,239282 |
| 305 | 26,44 | 3218,542 | 24706,822 | | 182,830483 |
| 306 | 22,018 | 3209,292 | 976666,75 | | 7227,33395 |
| 307 | 19,50.1 | 3205,379 | 172521,531 | | 1276,65933 |
| 308 | 25,911 | 3200,625 | 30798,475 | | 227,908715 |
| 309 | 30,054 | 3200,553 | 44472,367 | | 329,095516 |
| 310 | 26,275 | 3197,269 | 120978,656 | | 895,242054 |
| 311 | 18,965 | 3195,145 | 1365,552 | | 10,1050848 |
| 312 | 21,98 | 3193,339 | 46203,789 | | 341,908039 |
| 313 | 47,564 | 3190,567 | 6346,223 | | 46,9620502 |
| 314 | 26,894 | 3187,444 | 70904,891 | | 524,696193 |
| 315 | 47,302 | 3187,001 | 26800,559 | | 198,324137 |
| 316 | 31,823 | 3183,572 | 62859,313 | | 465,158916 |
| 317 | 27,686 | 3181,527 | 130513,648 | | 965,800995 |
| 318 | 20,726 | 3180,29 | 149180,203 | | 1103,9335 |
| 319 | 26,411 | 3175,418 | 95767,344 | | 708,678346 |
| 320 | 26,942 | 3166,985 | 32730,686 | | 242,207076 |
| 321 | 21,419 | 3166,416 | 143203,063 | | 1059,70267 |
| 322 | 26,351 | 3159,36 | 255623,938 | | 1891,61714 |
| 323 | 29,385 | 3158,431 | 24115,967 | | 178,458156 |
| 324 | 20,733 | 3153,308 | 214732,906 | | 1589,0235 |
| 325 | 30,597 | 3152,559 | 13341,057 | | 98,7238218 |
| 326 | 23,226 | 3152,484 | 36119,008 | | 267,280659 |
| 327 | 26,922 | 3149,624 | 114393,813 | | 846,514216 |
| 328 | 47,176 | 3148,686 | 135798,828 | | 1004,91133 |
| 329 | 22,866 | 3148,426 | 81875,969 | | 605,882171 |
| 330 | 32,019 | 3145,601 | 228017,438 | | 1687,32904 |
| 331 | 27,665 | 3143,604 | 316068,156 | | 2338,90435 |
| 332 | 26,033 | 3139,523 | 182642,438 | | 1351,55404 |
| 333 | 60,141 | 3138,904 | 21558,727 | | 159,53458 |
| 334 | 26,459 | 3137,595 | 153225,172 | | 1133,86627 |
| 335 | 29,41 | 3137,466 | 52329,82 | | 387,240668 |
| 336 | 26,846 | 3130,813 | 68584,234 | | 507,523332 |
| 337 | 30,606 | 3130,447 | 81592,641 | | 603,785543 |
| 338 | 47,079 | 3126,892 | 51823,832 | | 383,496357 |
| 339 | 26,408 | 3121,15 | 278297,125 | | 2059,39873 |
| 340 | 26,149 | 3117,737 | 33942,793 | | 251,176668 |
| 341 | 19,395 | 3116,31 | 11625,266 | | 86,0269684 |
| 342 | 29,428 | 3115,482 | 75318,172 | | 557,354473 |
| 343 | 21,751 | 3111,371 | 83680,453 | | 619,235352 |
| 344 | 26,927 | 3108,555 | 45816,859 | | 339,044757 |
| 345 | 47,062 | 3106,709 | 11864,485 | | 87,797189 |
| 346 | 26,248 | 3101,309 | 144908,688 | | 1072,32429 |
| 347 | 56,788 | 3101,218 | 12576,415 | | 93,065471 |
| 348 | 59,981 | 3101,187 | 25661,803 | | 189,897342 |
| 349 | 29,415 | 3099,508 | 130302,75 | | 964,24035 |
| 350 | 26,467 | 3099,349 | 120340,961 | | 890,523111 |
| 351 | 38,257 | 3093,515 | 133819,891 | | 990,267193 |
| 352 | 26,873 | 3092,646 | 79548,953 | | 588,662252 |
| 353 | 25,409 | 3091,628 | 74034,063 | | 547,852066 |
| 354 | 21,782 | 3089,383 | 140512,359 | | 1039,79146 |
| 355 | 26,165 | 3086,295 | 48012,742 | | 355,294291 |
| 356 | 60,105 | 3085,019 | 7315,923 | | 54,1378302 |
| 357 | 28,833 | 3083,524 | 10016,655 | | 74,123247 |
| 358 | 45,098 | 3080,227 | 54908,891 | | 406,325793 |
| 359 | 26,28 | 3079,63 | 200529,125 | | 1483,91553 |
| 360 | 29,426 | 3077,473 | 365503,375 | | 2704,72498 |
| 361 | 19,423 | 3076,296 | 244458,344 | | 1808,99175 |
| 362 | 21,716 | 3073,391 | 19542,326 | | 144,613212 |
| 363 | 20,524 | 3064,362 | 67399,18 | | 498,753932 |
| 364 | 26,328 | 3063,327 | 269186,313 | | 1991,97872 |
| 365 | 29,434 | 3061,477 | 296489,781 | | 2194,02438 |
| 366 | 23,304 | 3060,546 | 57847,941 | | 428,074763 |
| 367 | 22,449 | 3059,408 | 131458,734 | | 972,794632 |
| 368 | 38,508 | 3055,55 | 361900,688 | | 2678,06509 |
| 369 | 21,798 | 3051,223 | 597188,688 | | 4419,19629 |
| 370 | 26,15 | 3049,373 | 185624,422 | | 1373,62072 |
| 371 | 28,742 | 3045,463 | 33384,316 | | 247,043938 |
| 372 | 22,439 | 3043,271 | 33506,316 | | 247,946738 |
| 373 | 26,25 | 3041,552 | 275291,813 | | 2037,15942 |
| 374 | 29,434 | 3039,565 | 1232005,13 | | 9116,83793 |
| 375 | 31,704 | 3039,504 | 14986,015 | | 110,896511 |
| 376 | 31,401 | 3039,5 | 17652,994 | | 130,632156 |
| 377 | 30,288 | 3038,548 | 53233,535 | | 393,928159 |
| 378 | 21,744 | 3036,442 | 318586,125 | | 2357,53733 |
| 379 | 26,1 | 3033,408 | 63530,199 | | 470,123473 |
| 380 | 29,428 | 3023,508 | 440285,75 | | 3258,11455 |
| 381 | 23,309 | 3023,197 | 35494,395 | | 262,658523 |
| 382 | 20,855 | 3022,938 | 49940,453 | | 369,559352 |
| 383 | 22,458 | 3021,485 | 264509 | | 1957,3666 |
| 384 | 38,552 | 3018,517 | 10132,168 | | 74,9780432 |
| 385 | 47,558 | 3016,717 | 14738,372 | | 109,063953 |
| 386 | 19,317 | 3015,296 | 7210,649 | | 53,3588026 |
| 387 | **21,764** | **3013,407** | **2745916,75** | | **20319,784** |
| 388 | 52,853 | 3013,224 | 6434,849 | | 47,6178826 |
| 389 | **26,165** | **3011,541** | **328700,25** | 1828,86053 | **2432,38185** |
| 390 | 53,981 | 3011,136 | 9101,616 | | 67,3519584 |
| 391 | 22,771 | 3002,372 | 31709,063 | | 234,647066 |
| 392 | 29,689 | 3001,477 | 1807418,13 | | 13374,8941 |
| 393 | 29,788 | 2999,396 | 10075,938 | | 74,5619412 |
| 394 | 21,692 | 2999,39 | 458502,219 | | 3392,91642 |
| 395 | 47,256 | 2994,527 | 99911,469 | | 739,344871 |
| 396 | 54,066 | 2994,525 | 27062,277 | | 200,26085 |
| 397 | 21,71 | 2979,845 | 137706,313 | | 1019,02672 |
| 398 | 47,277 | 2978,338 | 98216,141 | | 726,799443 |
| 399 | 25,825 | 2977,451 | 51783,652 | | 383,199025 |
| 400 | 19,356 | 2977,313 | 141777,906 | | 1049,1565 |
| 401 | 28,834 | 2976,801 | 13806,644 | | 102,169166 |
| 402 | 22,995 | 2973,765 | 71126,297 | | 526,334598 |
| 403 | 54,065 | 2973,1 | 72137,359 | | 533,816457 |
| 404 | 23,115 | 2961,255 | 24611,352 | | 182,124005 |
| 405 | 29,606 | 2959,461 | 93377,992 | | 690,997141 |
| 406 | 54,087 | 2956,985 | 34407,879 | | 254,618305 |
| 407 | 47,177 | 2956,613 | 146180,453 | | 1081,73535 |
| 408 | 47,261 | 2939,827 | 19831,465 | | 146,752841 |
| 409 | 28,882 | 2939,32 | 8665,473 | | 64,1245002 |
| 410 | 23,159 | 2939,275 | 60206,324 | | 445,526798 |
| 411 | 47,074 | 2935,554 | 119347,43 | | 883,170982 |
| 412 | 54,081 | 2935,165 | 71573,266 | | 529,642168 |
| 413 | 29,202 | 2930,452 | 119639,5 | | 885,3323 |
| 414 | 23,112 | 2923,278 | 70444,055 | | 521,286007 |
| 415 | 47,154 | 2918,618 | 78469,992 | | 580,677941 |
| 416 | 25,694 | 2917,375 | 149087,75 | | 1103,24935 |
| 417 | 47,052 | 2914,597 | 30523,621 | | 225,874795 |
| 418 | 29,136 | 2914,306 | 40968,273 | | 303,16522 |
| 419 | 29,197 | 2913,596 | 18412,203 | | 136,250302 |
| 420 | 19,329 | 2913,459 | 99422,539 | | 735,726789 |
| 421 | 23,807 | 2912,353 | 384302,219 | | 2843,83642 |
| 422 | 23,119 | 2901,3 | 312914,094 | | 2315,5643 |
| 423 | 25,911 | 2898,456 | 18701,76 | | 138,393024 |
| 424 | 38,603 | 2898,411 | 5865,952 | | 43,4080448 |
| 425 | 29,2 | 2892,487 | 466333,719 | | 3450,86952 |
| 426 | 19,686 | 2887,621 | 72285,609 | | 534,913507 |
| 427 | 23,124 | 2885,349 | 248764,953 | | 1840,86065 |
| 428 | 23,181 | 2879,347 | 44899,32 | | 332,254968 |
| 429 | 29,188 | 2876,501 | 149361,734 | | 1105,27683 |
| 430 | 20,174 | 2864,504 | 170329,094 | | 1260,4353 |
| 431 | 23,108 | 2863,359 | 1062454 | | 7862,1596 |
| 432 | 25,816 | 2861,674 | 45627,336 | | 337,642286 |
| 433 | 29,189 | 2854,531 | 504398,031 | | 3732,54543 |
| 434 | 22,467 | 2849,122 | 98682,781 | | 730,252579 |
| 435 | 23,101 | 2847,369 | 329935,469 | | 2441,52247 |
| 436 | 30,997 | 2840,951 | 29558,09 | | 218,729866 |
| 437 | 23,206 | 2840,788 | 288407,906 | | 2134,2185 |
| 438 | 45,501 | 2839,222 | 19862,145 | | 146,979873 |
| 439 | 22,887 | 2838,17 | 401500,781 | | 2971,10578 |
| 440 | 23,479 | 2837,179 | 47790,191 | | 353,647413 |
| 441 | **23,12** | **2825,369** | **2478449** | **13220,0253** | 18340,5226 |
| 442 | 47,398 | 2824,479 | 66262,844 | | 490,345046 |
| 443 | 25,837 | 2823,447 | 161446,953 | | 1194,70745 |
| 444 | 23,493 | 2823,263 | 13751,687 | | 101,762484 |
| 445 | 31,001 | 2818,979 | 87299,125 | | 646,013525 |
| 446 | 25,738 | 2818,354 | 25736,781 | | 190,452179 |
| 447 | 45,063 | 2816,103 | 114974,227 | | 850,80928 |
| 448 | 23,099 | 2808,413 | 98189,281 | | 726,600679 |
| 449 | 47,299 | 2802,496 | 96102,875 | | 711,161275 |
| 450 | 25,713 | 2802,334 | 14592,676 | | 107,985802 |
| 451 | 34,356 | 2802,334 | 85762,539 | | 634,642789 |
| 452 | 23,512 | 2799,188 | 1044906,38 | | 7732,30718 |
| 453 | 47,289 | 2786,471 | 144146,313 | | 1066,68272 |
| 454 | 29,59 | 2781,738 | 10014,258 | | 74,1055092 |
| 455 | 25,737 | 2780,371 | 61741,945 | | 456,890393 |
| 456 | 56,764 | 2772,108 | 7783,357 | | 57,5968418 |
| 457 | 54,717 | 2772,02 | 10717,143 | | 79,3068582 |
| 458 | 21,387 | 2766,811 | 49527,543 | | 366,503818 |
| 459 | 47,176 | 2764,54 | 301319,875 | | 2229,76708 |
| 460 | 34,412 | 2764,419 | 401417,969 | | 2970,49297 |
| 461 | 22,615 | 2755,244 | 63484,922 | | 469,788423 |
| 462 | 26,046 | 2754,507 | 67685,281 | | 500,871079 |
| 463 | 45,398 | 2751,163 | 21037,008 | | 155,673859 |
| 464 | 47,208 | 2747,715 | 118178,953 | | 874,524252 |
| 465 | 47,1 | 2743,596 | 170694,891 | | 1263,14219 |
| 466 | **25,731** | **2742,398** | **138049,344** | **1296,81025** | 1021,56515 |
| 467 | 19,271 | 2733,962 | 31792,85 | | 235,26709 |
| 468 | 22,636 | 2733,228 | 122354,352 | | 905,422205 |
| 469 | 47,344 | 2726,574 | 36973,457 | | 273,603582 |
| 470 | 34,503 | 2726,404 | 137639,047 | | 1018,52895 |
| 471 | 21,913 | 2720,199 | 13717,608 | | 101,510299 |
| 472 | 19,219 | 2718,289 | 12883,684 | | 95,3392616 |
| 473 | 19,051 | 2717,641 | 33752,184 | | 249,766162 |
| 474 | 22,626 | 2717,287 | 228040,188 | | 1687,49739 |
| 475 | 34,147 | 2706,346 | 28067,836 | | 207,701986 |
| 476 | 22,609 | 2700,793 | 87403,336 | | 646,784686 |
| 477 | 19,26 | 2696,284 | 111610,914 | | 825,920764 |
| 478 | **22,644** | **2695,322** | **489907,813** | **3209,57073** | 3625,31782 |
| 479 | 29,391 | 2688,188 | 62864,418 | | 465,196693 |
| 480 | 53,432 | 2685,952 | 9794,151 | | 72,4767174 |
| 481 | 21,026 | 2682,281 | 54992,879 | | 406,947305 |
| 482 | 21,953 | 2682,264 | 164811,766 | | 1219,60707 |
| 483 | 19,234 | 2680,347 | 52036,555 | | 385,070507 |
| 484 | **22,642** | **2679,325** | **348845,031** | **2843,92567** | 2581,45323 |
| 485 | 53,436 | 2669,979 | 28587,529 | | 211,547715 |
| 486 | 29,453 | 2666,015 | 134377,281 | | 994,391879 |
| 487 | 21,154 | 2663,759 | 77780,211 | | 575,573561 |
| 488 | 22,618 | 2663,307 | 45806,359 | | 338,967057 |
| 489 | 45,5 | 2663,131 | 10745,179 | | 79,5143246 |
| 490 | 45,202 | 2663,093 | 9994,085 | | 73,956229 |
| 491 | 19,308 | 2658,394 | 889974 | | 6585,8076 |
| 492 | 22,792 | 2653,835 | 10012,66 | | 74,093684 |
| 493 | 29,409 | 2649,748 | 209835,844 | | 1552,78525 |
| 494 | 53,492 | 2647,894 | 109457,719 | | 809,987121 |
| 495 | 34,123 | 2646,285 | 83952,914 | | 621,251564 |
| 496 | 21,014 | 2644,389 | 143070,844 | | 1058,72425 |
| 497 | 24,958 | 2641,325 | 9244,972 | | 68,4127928 |
| 498 | 22,26 | 2640,197 | 37788,34 | | 279,633716 |
| 499 | 28,958 | 2638,48 | 18692,865 | | 138,327201 |
| 500 | 25,376 | 2637,34 | 43694,086 | | 323,336236 |
| 501 | 47,401 | 2632,529 | 85583,328 | | 633,316627 |
| 502 | 53,456 | 2631,985 | 46383,336 | | 343,236686 |
| 503 | 23,975 | 2631,124 | 19223,193 | | 142,251628 |
| 504 | 34,021 | 2630,294 | 11402,769 | | 84,3804906 |
| 505 | 29,459 | 2628,371 | 42183,633 | | 312,158884 |
| 506 | 22,281 | 2624,209 | 24509,521 | | 181,370455 |
| 507 | 29,621 | 2622,336 | 14705,738 | | 108,822461 |
| 508 | 25,361 | 2620,846 | 14378,705 | | 106,402417 |
| 509 | 25,496 | 2620,25 | 48204,504 | | 356,71333 |
| 510 | 22,285 | 2618,226 | 25010,121 | | 185,074895 |
| 511 | 47,465 | 2615,493 | 7661,221 | | 56,6930354 |
| 512 | 47,164 | 2615,485 | 6246,06 | | 46,220844 |
| 513 | 29,331 | 2611,332 | 51577,367 | | 381,672516 |
| 514 | 47,268 | 2610,486 | 133230,797 | | 985,907898 |
| 515 | 53,423 | 2609,623 | 65116,852 | | 481,864705 |
| 516 | 34,267 | 2608,302 | 477189,5 | | 3531,2023 |
| 517 | 36,436 | 2608,217 | 49190,086 | | 364,006636 |
| 518 | 25,258 | 2606,235 | 8864,643 | | 65,5983582 |
| 519 | 22,254 | 2602,233 | 250098,953 | | 1850,73225 |
| 520 | 28,955 | 2600,859 | 42927,066 | | 317,660288 |
| 521 | 47,321 | 2594,475 | 174972,719 | | 1294,79812 |
| 522 | 23,962 | 2593,309 | 31956,934 | | 236,481312 |
| 523 | 34,191 | 2592,376 | 73723,086 | | 545,550836 |
| 524 | 29,194 | 2589,416 | 24432,801 | | 180,802727 |
| 525 | 21,055 | 2586,81 | 447437,063 | | 3311,03427 |
| 526 | 29,643 | 2584,386 | 51728,656 | | 382,792054 |
| 527 | 25,285 | 2583,339 | 57463,207 | | 425,227732 |
| 528 | 22,289 | 2580,278 | 184628,453 | | 1366,25055 |
| 529 | 25,458 | 2579,266 | 17502,498 | | 129,518485 |
| 530 | 23,288 | 2577,366 | 55801,965 | | 412,934541 |
| 531 | 45,349 | 2575,055 | 55573,074 | | 411,240748 |
| 532 | 47,193 | 2572,49 | 283804,656 | | 2100,15445 |
| 533 | 34,366 | 2570,327 | 278517,688 | | 2061,03089 |
| 534 | 53,788 | 2565,094 | 15309,151 | | 113,287717 |
| 535 | **22,285** | **2564,171** | **522868,969** | **5032,24845** | 3869,23037 |
| 536 | 25,111 | 2563,298 | 69871,031 | | 517,045629 |
| 537 | 21,035 | 2563,271 | 214366,938 | | 1586,31534 |
| 538 | 28,935 | 2560,247 | 22648,59 | | 167,599566 |
| 539 | 19,241 | 2559,267 | 287009,563 | | 2123,87077 |
| 540 | 47,221 | 2555,777 | 175942,031 | | 1301,97103 |
| 541 | 23,992 | 2554,181 | 34969,023 | | 258,77077 |
| 542 | 47,112 | 2551,558 | 249344,906 | | 1845,1523 |
| 543 | 54,133 | 2549,837 | 10977,327 | | 81,2322198 |
| 544 | 53,85 | 2548,39 | 47193,648 | | 349,232995 |
| 545 | 21,105 | 2548,131 | 191514,047 | | 1417,20395 |
| 546 | 25,467 | 2541,55 | 46554,676 | | 344,504602 |
| 547 | 22,079 | 2540,416 | 70642,961 | | 522,757911 |
| 548 | 47,061 | 2539,548 | 9106,932 | | 67,3912968 |
| 549 | 22,976 | 2538,006 | 43224,359 | | 319,860257 |
| 550 | 23,949 | 2536,124 | 11100,246 | | 82,1418204 |
| 551 | 47,048 | 2534,558 | 93213,172 | | 689,777473 |
| 552 | 53,76 | 2531,854 | '6839,3 | | 50,61082 |
| 553 | 47,021 | 2530,606 | 81025,352 | | 599,587605 |
| 554 | 20,664 | 2527,292 | 182897,938 | | 1353,44474 |
| 555 | 53,86 | 2525,836 | 124043,008 | | 917,918259 |
| 556 | 25,163 | 2525,357 | 195437,094 | | 1446,2345 |
| 557 | 24,934 | 2521,218 | 35385,203 | | 261,850502 |
| 558 | 49,205 | 2520,943 | 7070,417 | | 52,3210858 |
| 559 | 22,186 | 2518,416 | 12192,377 | | 90,2235898 |
| 560 | 23,995 | 2514,154 | 31468,1 | | 232,86394 |
| 561 | 53,893 | 2509,854 | 74708,906 | | 552,845904 |
| 562 | 53,849 | 2503,849 | 66345,75 | | 490,95855 |
| 563 | 22,127 | 2502,45 | 883793,5 | | 6540,0719 |
| 564 | 23,949 | 2498,133 | 13605,738 | | 100,682461 |
| 565 | 30,369 | 2491,037 | 55242,645 | | 408,795573 |
| 566 | 53,921 | 2487,847 | 73487,078 | | 543,804377 |
| 567 | 45,471 | 2487,024 | 20252,117 | | 149,865666 |
| 568 | 53,508 | 2483,887 | 9788,317 | | 72,4335458 |
| 569 | 55,095 | 2483,245 | 22470,289 | | 166,280139 |
| 570 | 24,966 | 2483,135 | 101523,984 | | 751,277482 |
| 571 | 50,133 | 2480,983 | 6654,337 | | 49,2420938 |
| 572 | 28,907 | 2480,171 | 48322,785 | | 357,588609 |
| 573 | 37,468 | 2479,164 | 10220,078 | | 75,6285772 |
| 574 | 29,264 | 2471,298 | 17940,619 | | 132,760581 |
| 575 | 25,284 | 2468,22 | 27459,982 | | 203,203867 |
| 576 | 53,575 | 2466,851 | 50476,148 | | 373,523495 |
| 577 | 55,082 | 2466,643 | 85574,617 | | 633,252166 |
| 578 | 28,208 | 2466,149 | 19103,148 | | 141,363295 |
| 579 | 30,326 | 2452,741 | 105720,547 | | 782,332048 |
| 580 | 25,383 | 2452,241 | 25429,555 | | 188,178707 |
| 581 | 53,558 | 2444,922 | 132386,922 | | 979,663223 |
| 582 | 55,038 | 2444,863 | 166593,141 | | 1232,78924 |
| 583 | 54,419 | 2443,898 | 14516,007 | | 107,418452 |
| 584 | 28,896 | 2442,177 | 99161,281 | | 733,793479 |
| 585 | 47,436 | 2440,325 | 82396,445 | | 609,733693 |
| 586 | 22,331 | 2439,349 | 26883,18 | | 198,935532 |
| 587 | 20,725 | 2437,156 | 67598,68 | | 500,230232 |
| 588 | 30,367 | 2430,738 | 112506,719 | | 832,549721 |
| 589 | 25,309 | 2430,18 | 9069,742 | | 67,1160908 |
| 590 | 37,568 | 2430,001 | 10098,263 | | 74,7271462 |
| 591 | 55,058 | 2428,844 | 151840,578 | | 1123,62028 |
| 592 | 53,565 | 2428,839 | 97552,461 | | 721,888211 |
| 593 | 21,874 | 2427,305 | 277859,406 | | 2056,1596 |
| 594 | 28,784 | 2422,657 | 49308,391 | | 364,882093 |
| 595 | 47,264 | 2418,405 | 102784,953 | | 760,608652 |
| 596 | 28,611 | 2418,047 | 23545,922 | | 174,239823 |
| 597 | 30,328 | 2415,783 | 100262,688 | | 741,943891 |
| 598 | 20,7 | 2415,168 | 241525,406 | | 1787,288 |
| 599 | 28,755 | 2415,065 | 27699,828 | | 204,978727 |
| 600 | 19,242 | 2414,29 | 19697,506 | | 145,761544 |
| 601 | 55,019 | 2411,833 | 8743,657 | | 64,7030618 |
| 602 | 37,119 | 2408,022 | 95515,813 | | 706,817016 |
| 603 | 24,956 | 2407,143 | 19052,658 | | 140,989669 |
| 604 | 56,103 | 2406,969 | 2105,086 | | 15,5776364 |
| 605 | 53,549 | 2406,949 | 105913 | | 783,7562 |
| 606 | 55,077 | 2406,902 | 128330,906 | | 949,648704 |
| 607 | 21,91 | 2405,312 | 22780,617 | | 168,576566 |
| 608 | 20,28 | 2404,141 | 42368,875 | | 313,529675 |
| 609 | 47,296 | 2402,426 | 168612,344 | | 1247,73135 |
| 610 | 20,557 | 2401,462 | 109291,289 | | 808,755539 |
| 611 | 28,781 | 2398,085 | 20548,543 | | 152,059218 |
| 612 | 28,626 | 2396,215 | 186626 | | 1381,0324 |
| 613 | 54,355 | 2395,78 | 17539,246 | | 129,79042 |
| 614 | 19,791 | 2395,33 | 38127,117 | | 282,140666 |
| 615 | 53,832 | 2395,108 | 25210,896 | | 186,56063 |
| 616 | 22,181 | 2393,58 | 28325,973 | | 209,6122 |
| 617 | 37,389 | 2392,069 | 32789,227 | | 242,64028 |
| 618 | 36,842 | 2392,022 | 16480,713 | | 121,957276 |
| 619 | 53,509 | 2390,96 | 7808,705 | | 57,784417 |
| 620 | 55,017 | 2390,746 | 56777,559 | | 420,153937 |
| 621 | 21,858 | 2389,367 | 1415681,5 | | 10476,0431 |
| 622 | 28,783 | 2384,878 | 67750,172 | | 501,351273 |
| 623 | 54,213 | 2381,804 | 9437,86 | | 69,840164 |
| 624 | 53,96 | 2379,819 | 13638,621 | | 100,925795 |
| 625 | 28,586 | 2379,768 | 179069,688 | | 1325,11569 |
| 626 | 20,738 | 2377,238 | 713037,375 | | 5276,47658 |
| 627 | 28,792 | 2377,105 | 243979,281 | | 1805,44668 |
| 628 | 54,034 | 2374,369 | 98473,961 | | 728,707311 |
| 629 | 38,119 | 2370,071 | 587311,313 | | 4346,10372 |
| 630 | 55,054 | 2368,829 | 45047,953 | | 333,354852 |
| 631 | 49,833 | 2368,79 | 7574,214 | | 56,0491836 |
| 632 | 21,712 | 2364,035 | 14890,036 | | 110,186266 |
| 633 | 47,251 | 2363,877 | 169502,969 | | 1254,32197 |
| 634 | 47,112 | 2359,46 | 226706,625 | | 1677,62903 |
| 635 | 54,06 | 2358,796 | 271180 | | 2006,732 |
| 636 | 28,638 | 2358,069 | 484792 | | 3587,4608 |
| 637 | 30,392 | 2356,841 | 24718,348 | | 182,915775 |
| 638 | 49,349 | 2356,439 | 49754,129 | | 368,180555 |
| 639 | 22,229 | 2355,161 | 64185,695 | | 474,974143 |
| 640 | 37,22 | 2354,086 | 71290,633 | | 527,550684 |
| 641 | 54,995 | 2351,738 | 14965,655 | | 110,745847 |
| 642 | 19,26 | 2343,309 | 31234,203 | | 231,133102 |
| 643 | 54,345 | 2342,704 | 55254,418 | | 408,882693 |
| 644 | 21,753 | 2342,053 | 57971,406 | | 428,988404 |
| 645 | 53,764 | 2341,883 | 102171,742 | | 756,070891 |
| 646 | 28,619 | 2341,757 | 395942,094 | | 2929,9715 |
| 647 | 24,143 | 2341,072 | 185665,328 | | 1373,92343 |
| 648 | 28,832 | 2339,135 | 119528,75 | | 884,51275 |
| 649 | 54,092 | 2336,821 | 849316,625 | | 6284,94303 |
| 650 | 49,582 | 2334,924 | 16711,031 | | 123,661629 |
| 651 | 49,932 | 2332,825 | 7154,382 | | 52,9424268 |
| 652 | 38,111 | 2332,118 | 724905,563 | | 5364,30117 |
| 653 | 28,429 | 2332,011 | 26272,477 | | 194,41633 |
| 654 | 49,784 | 2330,848 | 18374,775 | | 135,973335 |
| 655 | 20,777 | 2329,976 | 13845,191 | | 102,454413 |
| 656 | 20,781 | 2328,12 | 114579,438 | | 847,887841 |
| 657 | 21,695 | 2326,051 | 33955,465 | | 251,270441 |
| 658 | 28,514 | 2325,829 | 27821,584 | | 205,879722 |
| 659 | 24,109 | 2325,039 | 347883,406 | | 2574,3372 |
| 660 | 18,808 | 2323,646 | 3501,718 | | 25,9127132 |
| 661 | 21,857 | 2322,105 | 27377,75 | | 202,59535 |
| 662 | 54,036 | 2320,85 | 1445477 | | 10696,5298 |
| 663 | 21,736 | 2320,083 | 17368,453 | | 128,526552 |
| 664 | 28,756 | 2320,083 | 1394689,88 | | 10320,7051 |
| 665 | 31,151 | 2319,995 | 18706,303 | | 138,426642 |
| 666 | 50,101 | 2319,65 | 21160,838 | | 156,590201 |
| 667 | 49,596 | 2318,781 | 65589,969 | | 485,365771 |
| 668 | 53,96 | 2314,817 | 5407,608 | | 40,0162992 |
| 669 | 28,434 | 2313,129 | 21076,289 | | 155,964539 |
| 670 | 20,695 | 2312,394 | 183844,266 | | 1360,44757 |
| 671 | 45,447 | 2310,83 | 8350,151 | | 61,7911174 |
| 672 | 28,377 | 2309,901 | 32903,531 | | 243,486129 |
| 673 | 24,091 | 2309,052 | 53750,734 | | 397,755432 |
| 674 | 28,352 | 2308,815 | 65167,617 | | 482,240366 |
| 675 | 19,413 | 2306,093 | 11910,861 | | 88,1403714 |
| 676 | 47,374 | 2305,43 | 20267,41 | | 149,978834 |
| 677 | 30,479 | 2304,938 | 64574,715 | | 477,852891 |
| 678 | 54,005 | 2304,69 | 601818,875 | | 4453,45968 |
| 679 | 55,551 | 2304,254 | 20926,719 | | 154,857721 |
| 680 | 28,66 | 2304,2 | 441698,625 | | 3268,56983 |
| 681 | 21,748 | 2304,056 | 491680,813 | | 3638,43802 |
| 682 | 54,026 | 2298,86 | 2803671 | | 20747,1654 |
| 683 | 20,539 | 2298,221 | 189552,234 | | 1402,68653 |
| 684 | 30,798 | 2295,988 | 10958,861 | | 81,0955714 |
| 685 | 50,105 | 2295,884 | 10295,519 | | 76,1868406 |
| 686 | 19,179 | 2295,451 | 24928,195 | | 184,468643 |
| 687 | 28,816 | 2295,237 | 260138,016 | | 1925,02132 |
| 688 | 28,385 | 2295,094 | 55681,145 | | 412,040473 |
| 689 | 21,739 | 2288,101 | 57605,777 | | 426,28275 |
| 690 | 24,096 | 2287,057 | 1262872,75 | | 9345,25835 |
| 691 | 28,388 | 2287,048 | 297568,75 | | 2202,00875 |
| 692 | 20,5 | 2286,111 | 21349,635 | | 157,987299 |
| 693 | 24,027 | 2283,095 | 24350,102 | | 180,190755 |
| 694 | 54,068 | 2282,898 | 2728088,75 | | 20187,8568 |
| 695 | **28,732** | **2282,105** | **667956,938** | **6281,39651** | 4942,88134 |
| 696 | 21,782 | 2282,103 | 58645,039 | | 433,973289 |
| 697 | 30,387 | 2282 | 115145,461 | | 852,076411 |
| 698 | 38,199 | 2281,901 | 31642,785 | | 234,156609 |
| 699 | 38,703 | 2281,894 | 8948,516 | | 66,2190184 |
| 700 | 40,729 | 2281,884 | 69357,18 | | 513,243132 |
| 701 | 37,847 | 2281,877 | 7632,981 | | 56,4840594 |
| 702 | 49,628 | 2280,479 | 11230,125 | | 83,102925 |
| 703 | 53,207 | 2278,702 | 26455,098 | | 195,767725 |
| 704 | 28,903 | 2273,115 | 293589,719 | | 2172,56392 |
| 705 | 28,157 | 2272,659 | 27751,025 | | 205,357585 |
| 706 | 28,368 | 2271,091 | 240296,203 | | 1778,1919 |
| 707 | 23,951 | 2271,069 | 46381,387 | | 343,222264 |
| 708 | 54,563 | 2266,722 | 53080,328 | | 392,794427 |
| 709 | 53,746 | 2266,569 | 194273,734 | | 1437,62563 |
| 710 | 21,735 | 2266,113 | 834853,813 | | 6177,91822 |
| 711 | 47,142 | 2264,341 | 11589,805 | | 85,764557 |
| 712 | 25,004 | 2264,038 | 10342,722 | | 76,5361428 |
| 713 | 54,111 | 2260,906 | 3409623 | | 25231,2102 |
| 714 | 39,005 | 2259,881 | 8171,715 | | 60,470691 |
| 715 | 38,018 | 2259,88 | 89646,383 | | 663,383234 |
| 716 | 29,265 | 2257,021 | 369411,719 | | 2733,64672 |
| 717 | 28,144 | 2250,717 | 73907,656 | | 546,916654 |
| 718 | 28,424 | 2249,076 | 762669,688 | | 5643,75569 |
| 719 | 20,437 | 2249,042 | 143049,578 | | 1058,56688 |
| 720 | 47,446 | 2248,591 | 114672,375 | | 848,575575 |
| 721 | 53,175 | 2246,379 | 18363,697 | | 135,891358 |
| 722 | 54,105 | 2244,921 | 1221368,75 | | 9038,12875 |
| 723 | 53,209 | 2240,749 | 57652,742 | | 426,630291 |
| 724 | 49,832 | 2237,759 | 5953,025 | | 44,052385 |
| 725 | 28,924 | 2235,175 | 214885,766 | | 1590,15467 |
| 726 | 28,13 | 2234,935 | 156541,781 | | 1158,40918 |
| 727 | 28,391 | 2233,099 | 305448,406 | | 2260,3182 |
| 728 | 20,474 | 2232,684 | 41022,949 | | 303,569823 |
| 729 | 25,029 | 2231,969 | 11965,051 | | 88,5413774 |
| 730 | 23,853 | 2230,051 | 38128,531 | | 282,151129 |
| 731 | 25,127 | 2227,077 | 31312,469 | | 231,712271 |
| 732 | 47,253 | 2226,358 | 138834,063 | | 1027,37207 |
| 733 | 24,982 | 2226,04 | 37025,727 | | 273,99038 |
| 734 | 54,067 | 2222,955 | 1451186,5 | | 10738,7801 |
| 735 | 55,587 | 2222,909 | 18411,467 | | 136,244856 |
| 736 | 39,072 | 2221,946 | 758603,125 | | 5613,66313 |
| 737 | 33,365 | 2221,747 | 13675,914 | | 101,201764 |
| 738 | 49,924 | 2221,487 | 22766,725 | | 168,473765 |
| 739 | 28,601 | 2216,12 | 62841,969 | | 465,030571 |
| 740 | 21,813 | 2215,931 | 38498,379 | | 284,888005 |
| 741 | 28,165 | 2212,947 | 407865,344 | | 3018,20355 |
| 742 | 20,541 | 2211,332 | 175885,453 | | 1301,55235 |
| 743 | 28,425 | 2211,08 | 604871,438 | | 4476,04864 |
| 744 | 47,358 | 2210,38 | 198223,844 | | 1466,85645 |
| 745 | 24,99 | 2209,092 | 148361,516 | | 1097,87522 |
| 746 | 27,351 | 2208,986 | 43820,238 | | 324,269761 |
| 747 | 34,776 | 2208,878 | 7891,04 | | 58,393696 |
| 748 | 23,983 | 2208,045 | 1445503,75 | | 10696,7278 |
| 749 | 33,037 | 2207,824 | 20242,492 | | 149,794441 |
| 750 | 53,207 | 2207,742 | 5340,262 | | 39,5179388 |
| 751 | 29,961 | 2206,693 | 69426,469 | | 513,755871 |
| 752 | 39,013 | 2205,963 | 658740,688 | | 4874,68109 |
| 753 | 54,008 | 2203,897 | 11335,302 | | 83,8812348 |
| 754 | 53,189 | 2202,779 | 65413,605 | | 484,060677 |
| 755 | 19,293 | 2200,036 | 22179,658 | | 164,129469 |
| 756 | 55,068 | 2199,737 | 7793,02 | | 57,668348 |
| 757 | 28,127 | 2197,011 | 487901,156 | | 3610,46855 |
| 758 | 20,181 | 2194,916 | 131013,984 | | 969,503482 |
| 759 | 50,327 | 2194,728 | 41764,328 | | 309,056027 |
| 760 | 24,981 | 2193,839 | 73573,773 | | 544,44592 |
| 761 | 20,446 | 2193,108 | 20270,781 | | 150,003779 |
| 762 | 54,947 | 2192,113 | 67683,648 | | 500,858995 |
| 763 | 23,925 | 2192,015 | 526681,563 | | 3897,44357 |
| 764 | 21,89 | 2191,903 | 167538 | | 1239,7812 |
| 765 | 50,864 | 2191,723 | 32037,535 | | 237,077759 |
| 766 | 47,244 | 2188,395 | 251956,906 | | 1864,4811 |
| 767 | 32,649 | 2188,058 | 78885,688 | | 583,754091 |
| 768 | 24,014 | 2186,089 | 116699,281 | | 863,574679 |
| 769 | 29,978 | 2184,703 | 233078,734 | | 1724,78263 |
| 770 | 39,09 | 2183,992 | 1648277,63 | | 12197,2544 |
| 771 | 50,193 | 2178,759 | 33248,797 | | 246,041098 |
| 772 | 54,895 | 2176,709 | 42103,77 | | 311,567898 |
| 773 | 21,423 | 2175,984 | 37351,434 | | 276,400612 |
| 774 | 51,031 | 2175,813 | 37432,242 | | 276,998591 |
| 775 | **28,214** | **2175,135** | **523507,813** | **4308,36938** | 3873,95782 |
| 776 | 55,139 | 2174,695 | 12196,703 | | 90,2556022 |
| 777 | 47,254 | 2172,427 | 139909,953 | | 1035,33365 |
| 778 | 24,955 | 2171,845 | 392253,938 | | 2902,67914 |
| 779 | 54,95 | 2170,835 | 83249,852 | | 616,048905 |
| 780 | **23,99** | **2170,06** | **2801978,75** | **16950,086** | 20734,6428 |
| 781 | 33,051 | 2169,816 | 115270,117 | | 852,998866 |
| 782 | 28,029 | 2169,083 | 41560,289 | | 307,546139 |
| 783 | 29,942 | 2168,741 | 50702,242 | | 375,196591 |
| 784 | 38,918 | 2168 | 625043,188 | | 4625,31959 |
| 785 | 47,105 | 2167,452 | 166361,109 | | 1231,07221 |
| 786 | 23,628 | 2166,176 | 62101,336 | | 459,549886 |
| 787 | 27,877 | 2163,994 | 17963,393 | | 132,929108 |
| 788 | 19,292 | 2163,03 | 26186,1 | | 193,77714 |
| 789 | **28,177** | **2159,127** | **165196,703** | **2003,94316** | 1222,4556 |
| 790 | 21,729 | 2157,014 | 371334,938 | | 2747,87854 |
| 791 | 50,303 | 2156,445 | 98210,313 | | 726,756316 |
| 792 | 24,927 | 2156,036 | 134981,219 | | 998,861021 |
| 793 | 54,905 | 2155,728 | 215793,328 | | 1596,87063 |
| 794 | 50,291 | 2153,919 | 356736,531 | | 2639,85033 |
| 795 | 33,038 | 2153,853 | 17994,725 | | 133,160965 |
| 796 | 24,073 | 2153,761 | 687819,375 | | 5089,86338 |
| 797 | 21,431 | 2151,975 | 13115,051 | | 97,0513774 |
| 798 | 47,014 | 2150,457 | 80450,156 | | 595,331154 |
| 799 | 24,985 | 2150,074 | 124303,188 | | 919,843591 |
| 800 | 23,697 | 2148,078 | 14427,769 | | 106,765491 |
| 801 | 47,017 | 2146,51 | 33126,008 | | 245,132459 |
| 802 | 39,031 | 2146,033 | 1517131 | | 11226,7694 |
| 803 | 28,033 | 2142,049 | 116757,086 | | 864,002436 |
| 804 | 50,317 | 2140,053 | 49425,555 | | 365,749107 |
| 805 | 54,907 | 2139,687 | 131492,109 | | 973,041607 |
| 806 | 19,34 | 2139,264 | 16454,809 | | 121,765587 |
| 807 | 21,438 | 2138,028 | 170402,016 | | 1260,97492 |
| 808 | 49,775 | 2137,904 | 122028,063 | | 903,007666 |
| 809 | 51,01 | 2137,833 | 107543,789 | | 795,824039 |
| 810 | 24,98 | 2134,064 | 184060,313 | | 1362,04632 |
| 811 | 23,947 | 2133,997 | 14915,034 | | 110,371252 |
| 812 | 54,93 | 2133,748 | 448603,344 | | 3319,66475 |
| 813 | 23,791 | 2132,926 | 98156,297 | | 726,356598 |
| 814 | 33,034 | 2131,834 | 93856,258 | | 694,536309 |
| 815 | 49,73 | 2131,826 | 40604,926 | | 300,476452 |
| 816 | 19,309 | 2126,895 | 11998,439 | | 88,7884486 |
| 817 | 19,23 | 2125,959 | 13855,418 | | 102,530093 |
| 818 | 28,013 | 2125,696 | 179600,594 | | 1329,0444 |
| 819 | 29,033 | 2123,978 | 9567,942 | | 70,8027708 |
| 820 | 28,424 | 2123,872 | 38521,063 | | 285,055866 |
| 821 | 19,288 | 2123,304 | 41390,043 | | 306,286318 |
| 822 | 54,923 | 2117,754 | 676757,125 | | 5008,00273 |
| 823 | 50,182 | 2115,807 | 562227,938 | | 4160,48674 |
| 824 | 32,559 | 2113,632 | 14000,153 | | 103,601132 |
| 825 | 27,977 | 2110,026 | 15948,838 | | 118,021401 |
| 826 | 19,242 | 2109,934 | 16406,662 | | 121,409299 |
| 827 | 23,748 | 2108,995 | 14636,829 | | 108,312535 |
| 828 | 27,756 | 2106,99 | 38564,801 | | 285,379527 |
| 829 | 20,492 | 2105,879 | 76712,156 | | 567,669954 |
| 830 | 54,924 | 2101,744 | 308647,25 | | 2283,98965 |
| 831 | 21,456 | 2101,398 | 246869,797 | | 1826,8365 |
| 832 | 49,813 | 2099,871 | 64825,379 | | 479,707805 |
| 833 | 50,969 | 2099,86 | 21705,629 | | 160,621655 |
| 834 | 22,512 | 2098,949 | 30868,66 | | 228,428084 |
| 835 | 54,919 | 2095,774 | 1133086,88 | | 8384,84288 |
| 836 | 56,91 | 2094,771 | 16988,391 | | 125,714093 |
| 837 | 50,601 | 2094,588 | 22926,068 | | 169,652903 |
| 838 | 23,784 | 2093,206 | 75977,586 | | 562,234136 |
| 839 | **28,084** | **2088,017** | **171200,609** | **1269,99462** | 1266,88451 |
| 840 | 19,264 | 2087,973 | 350660,688 | | 2594,88909 |
| 841 | 23,117 | 2086,65 | 12786,285 | | 94,618509 |
| 842 | 27,791 | 2085,045 | 199885,375 | | 1479,15178 |
| 843 | 20,269 | 2081,018 | 229086,047 | | 1695,23675 |
| 844 | 54,93 | 2079,801 | 993210,188 | | 7349,75539 |
| 845 | 47,595 | 2079,318 | 19015,662 | | 140,715899 |
| 846 | 56,839 | 2078,89 | 19401,766 | | 143,573068 |
| 847 | 49,785 | 2077,893 | 115355,531 | | 853,630929 |
| 848 | 21,811 | 2077,762 | 31063,748 | | 229,871735 |
| 849 | 52,075 | 2077,73 | 6613,918 | | 48,9429932 |
| 850 | 21,41 | 2076,984 | 175348,891 | | 1297,58179 |
| 851 | 51,543 | 2071,648 | 7766,515 | | 57,472211 |
| 852 | 23,759 | 2071,002 | 133591,328 | | 988,575827 |
| 853 | 21,424 | 2070,003 | 109701,07 | | 811,787918 |
| 854 | 51,254 | 2069,625 | 61727,754 | | 456,78538 |
| 855 | 27,817 | 2069,09 | 222562,547 | | 1646,96285 |
| 856 | 20,532 | 2067,921 | 477863,719 | | 3536,19152 |
| 857 | 27,607 | 2063,948 | 71420,422 | | 528,511123 |
| 858 | 54,94 | 2063,804 | 166864,938 | | 1234,80054 |
| 859 | 55,581 | 2062,783 | 10409,057 | | 77,0270218 |
| 860 | 22,44 | 2059,045 | 31675,367 | | 234,397716 |
| 861 | 54,9 | 2057,817 | 1045255,63 | | 7734,89163 |
| 862 | 20,802 | 2056,804 | 23316,27 | | 172,540398 |
| 863 | 47,411 | 2056,32 | 114767,031 | | 849,276029 |
| 864 | 23,834 | 2056,014 | 75674,141 | | 559,988643 |
| 865 | 52,099 | 2054,689 | 8212,415 | | 60,771871 |
| 866 | 51,141 | 2052,646 | 7170,431 | | 53,0611894 |
| 867 | 23,596 | 2050,953 | 30659,434 | | 226,879812 |
| 868 | 23,173 | 2049,013 | 179035,266 | | 1324,86097 |
| 869 | 51,154 | 2047,635 | 93990,273 | | 695,52802 |
| 870 | **27,801** | **2047,175** | **411581** | **1703,37702** | 3045,6994 |
| 871 | 28,43 | 2045,938 | 76368,227 | | 565,12488 |
| 872 | 28,078 | 2041,99 | 11063,896 | | 81,8728304 |
| 873 | 54,923 | 2041,771 | 361323,031 | | 2673,79043 |
| 874 | 21,426 | 2039,213 | 401444,531 | | 2970,68953 |
| 875 | 40,128 | 2036,857 | 62552,297 | | 462,886998 |
| 876 | 40,967 | 2036,734 | 16498,773 | | 122,09092 |
| 877 | 19,377 | 2035,142 | 119280,563 | | 882,676166 |
| 878 | 20,833 | 2034,878 | 107200,859 | | 793,286357 |
| 879 | 51,167 | 2031,663 | 179833,078 | | 1330,76478 |
| 880 | 27,821 | 2031,125 | 226310,281 | | 1674,69608 |
| 881 | 49,933 | 2030,735 | 8329,54 | | 61,638596 |
| 882 | 20,301 | 2029,874 | 59304,598 | | 438,854025 |
| 883 | 27,841 | 2027,979 | 24592,67 | | 181,985758 |
| 884 | 27,615 | 2025,995 | 173888,672 | | 1286,77617 |
| 885 | 21,281 | 2023,988 | 15667,275 | | 115,937835 |
| 886 | 54,897 | 2019,862 | 339981,563 | | 2515,86357 |
| 887 | 47,368 | 2018,356 | 144909,141 | | 1072,32764 |
| 888 | 20,769 | 2017,852 | 41804,027 | | 309,3498 |
| 889 | 50,41 | 2017,309 | 23882,344 | | 176,729346 |
| 890 | 52,145 | 2016,741 | 25066,926 | | 185,495252 |
| 891 | 51,168 | 2015,674 | 15682,338 | | 116,049301 |
| 892 | 21,462 | 2014,872 | 676609,125 | | 5006,90753 |
| 893 | 23,598 | 2013,999 | 118936,234 | | 880,128132 |
| 894 | 27,425 | 2013,957 | 10481,886 | | 77,5659564 |
| 895 | 27,626 | 2009,933 | 150281,469 | | 1112,08287 |
| 896 | 51,219 | 2009,681 | 269662,813 | | 1995,50482 |
| 897 | 51,765 | 2008,693 | 8050,752 | | 59,5755648 |
| 898 | 27,027 | 2008,114 | 36968,852 | | 273,569505 |
| 899 | 49,949 | 2007,869 | 263181,844 | | 1947,54565 |
| 900 | 53,968 | 2007,765 | 23462,338 | | 173,621301 |
| 901 | 47,566 | 2003,37 | 5718,014 | | 42,3133036 |
| 902 | 20,834 | 1996,814 | 335825,125 | | 2485,10593 |
| 903 | 49,681 | 1995,723 | 5319,325 | | 39,363005 |
| 904 | 50,085 | 1995,688 | 7241,29 | | 53,585546 |
| 905 | 21,049 | 1994,926 | 32653,094 | | 241,632896 |
| 906 | 27,528 | 1993,98 | 20820,145 | | 154,069073 |
| 907 | 51,008 | 1993,068 | 187387,828 | | 1386,66993 |
| 908 | 49,882 | 1991,781 | 242254,469 | | 1792,68307 |
| 909 | 27,745 | 1989,977 | 116543,305 | | 862,420457 |
| 910 | 39,9 | 1989,431 | 174175,406 | | 1288,898 |
| 911 | 23,409 | 1987,045 | 367932,188 | | 2722,69819 |
| 912 | 27,172 | 1985,949 | 13387,146 | | 99,0648804 |
| 913 | 20,148 | 1980,776 | 40316,543 | | 298,342418 |
| 914 | 47,304 | 1980,392 | 93012,695 | | 688,293943 |
| 915 | 35,172 | 1977,731 | 80382,313 | | 594,829116 |
| 916 | 27,666 | 1975,777 | 70365,492 | | 520,704641 |
| 917 | 47,124 | 1975,433 | 105832,047 | | 783,157148 |
| 918 | 39,641 | 1972,397 | 211920,281 | | 1568,21008 |
| 919 | 21,264 | 1971,932 | 24054,641 | | 178,004343 |
| 920 | 41,424 | 1971,717 | 16212,052 | | 119,969185 |
| 921 | 40,892 | 1971,699 | 25774,264 | | 190,729554 |
| 922 | 51,25 | 1971,598 | 203815,859 | | 1508,23736 |
| 923 | 23,437 | 1971,004 | 533845,5 | | 3950,4567 |
| 924 | 41,952 | 1970,701 | 11751,374 | | 86,9601676 |
| 925 | 49,899 | 1969,877 | 510840,781 | | 3780,22178 |
| 926 | 54,004 | 1969,775 | 110049,828 | | 814,368727 |
| 927 | 52,803 | 1969,714 | 3832,406 | | 28,3598044 |
| 928 | 40,409 | 1967,647 | 566741,125 | | 4193,88433 |
| 929 | 51,49 | 1966,766 | 8687,899 | | 64,2904526 |
| 930 | 20,143 | 1964,822 | 68999,875 | | 510,599075 |
| 931 | 27,226 | 1963,76 | 22017,5 | | 162,9295 |
| 932 | 35,592 | 1961,849 | 215947,656 | | 1598,01265 |
| 933 | 20,186 | 1958,815 | 182788,188 | | 1352,63259 |
| 934 | 25,235 | 1955,974 | 34034,582 | | 251,855907 |
| 935 | 34,906 | 1955,842 | 23583,658 | | 174,519069 |
| 936 | 35,204 | 1954,875 | 23046,27 | | 170,542398 |
| 937 | 20,225 | 1954,822 | 104457,664 | | 772,986714 |
| 938 | 27,654 | 1954,071 | 25012,77 | | 185,094498 |
| 939 | 49,914 | 1953,531 | 149496,656 | | 1106,27525 |
| 940 | 23,846 | 1951,907 | 11188,573 | | 82,7954402 |
| 941 | 40,048 | 1951,671 | 405308,781 | | 2999,28498 |
| 942 | 49,519 | 1951,462 | 32885,328 | | 243,351427 |
| 943 | 28,011 | 1950,926 | 80130,508 | | 592,965759 |
| 944 | 29,888 | 1950,847 | 95179,586 | | 704,328936 |
| 945 | 21,331 | 1949,954 | 15990,713 | | 118,331276 |
| 946 | 19,872 | 1949,895 | 8138,52 | | 60,225048 |
| 947 | 23,457 | 1949,102 | 3147247 | | 23289,6278 |
| 948 | 27,164 | 1947,721 | 65290,855 | | 483,152327 |
| 949 | 35,176 | 1945,87 | 48158,836 | | 356,375386 |
| 950 | 51,644 | 1945,533 | 5578,079 | | 41,2777846 |
| 951 | 28,503 | 1945,117 | 26344,092 | | 194,946281 |
| 952 | 20,19 | 1942,355 | 176231,625 | | 1304,11403 |
| 953 | 36,009 | 1939,849 | 754929,75 | | 5586,48015 |
| 954 | 21,083 | 1939,728 | 83881,531 | | 620,723329 |
| 955 | 27,622 | 1937,032 | 122677,93 | | 907,816682 |
| 956 | 20,174 | 1935,815 | 14819,995 | | 109,667963 |
| 957 | 55,67 | 1935,509 | 1866,308 | | 13,8106792 |
| 958 | 19,929 | 1934,88 | 185292,125 | | 1371,16173 |
| 959 | 27,6 | 1933,946 | 21970,883 | | 162,584534 |
| 960 | 21,312 | 1933,467, | 120517,273 | | 891,82782 |
| 961 | 23,44 | 1933,132 | 1264035,38 | | 9353,86178 |
| 962 | 49,882 | 1931,835 | 169525,344 | | 1254,48755 |
| 963 | 53,987 | 1931,539 | 7912,462 | | 58,5522188 |
| 964 | 32,897 | 1930,835 | 60372,492 | | 446,756441 |
| 965 | 40,453 | 1929,642 | 1351748,75 | | 10002,9408 |
| 966 | 49,985 | 1928,736 | 30871,664 | | 228,450314 |
| 967 | 19,413 | 1928,014 | 24680,447 | | 182,635308 |
| 968 | 22,284 | 1925,639 | 28804,176 | | 213,150902 |
| 969 | 36,02 | 1923,804 | 788593 | | 5835,5882 |
| 970 | 32,585 | 1923,744 | 18391,229 | | 136,095095 |
| 971 | 51,474 | 1923,542 | 22426,631 | | 165,957069 |
| 972 | 21,155 | 1921,892 | 25471,947 | | 188,492408 |
| 973 | 20,197 | 1920,513 | 484588,406 | | 3585,9542 |
| 974 | 35,145 | 1917,819 | 151947,547 | | 1124,41185 |
| 975 | 20,275 | 1916,865 | 592958 | | 4387,8892 |
| 976 | 32,944 | 1914,85 | 53011,285 | | 392,283509 |
| 977 | 39,532 | 1913,055 | 359803,531 | | 2662,54613 |
| 978 | 27,873 | 1912,956 | 171374,188 | | 1268,16899 |
| 979 | 49,886 | 1912,77 | 90604,273 | | 670,47162 |
| 980 | 23,536 | 1911,131 | 16659075 | | 123277,155 |
| 981 | 19,165 | 1909,169 | 16568,082 | | 122,603807 |
| 982 | 35,112 | 1907,913 | 132959,188 | | 983,897991 |
| 983 | 51,45 | 1907,405 | 55725,84 | | 412,371216 |
| 984 | 20,208 | 1904,837 | 417636,094 | | 3090,5071 |
| 985 | 19,974 | 1901,937 | 12937,851 | | 95,7400974 |
| 986 | 36,035 | 1901,931 | 1493067,88 | | 11048,7023 |
| 987 | 27,44 | 1901,067 | 35484,109 | | 262,582407 |
| 988 | 21,174 | 1898,892 | 49621,984 | | 367,202682 |
| 989 | 26,836 | 1897,634 | 51880,426 | | 383,915152 |
| 990 | 22,569 | 1895,801 | 31370,744 | | 232,143506 |
| 991 | 50,839 | 1895,655 | 14071,792 | | 104,131261 |
| 992 | 19,364 | 1893,904 | 10081,896 | | 74,6060304 |
| 993 | 20,096 | 1893,708 | 16126,631 | | 119,337069 |
| 994 | 23,44 | 1893,144 | 138012,906 | | 1021,2955 |
| 995 | 33,628 | 1892,875 | 666429,375 | | 4931,57738 |
| 996 | 40,178 | 1891,728 | 965003,063 | | 7141,02267 |
| 997 | 23,747 | 1890,939 | 199265,828 | | 1474,56713 |
| 998 | 28,18 | 1889,913 | 10303,565 | | 76,246381 |
| 999 | 51,81 | 1886,097 | 32181,391 | | 238,142293 |
| 1000 | 49,855 | 1886,017 | 9415,063 | | 69,6714662 |
| 1001 | 19,928 | 1885,96 | 15694,74 | | 116,141076 |
| 1002 | 35,93 | 1885,943 | 758995,75 | | 5616,56855 |
| 1003 | 51,337 | 1885,571 | 114489,086 | | 847,219236 |
| 1004 | 20,18 | 1882,925 | 4423241 | | 32731,9834 |
| 1005 | 29,37 | 1882,543 | 68798,625 | | 509,109825 |
| 1006 | 35,135 | 1879,95 | 58723,824 | | 434,556298 |
| 1007 | 22,494 | 1879,257 | 20705,602 | | 153,221455 |
| 1008 | 34,349 | 1878,875 | 33668,203 | | 249,144702 |
| 1009 | 22,591 | 1876,914 | 37062,238 | | 274,260561 |
| 1010 | **21,725** | **1876,902** | **297599,313** | **1550,63508** | 2202,23492 |
| 1011 | 32,986 | 1876,876 | 128455,727 | | 950,57238 |
| 1012 | 55,924 | 1875,624 | 2100,854 | | 15,5463196 |
| 1013 | 26,625 | 1875,501 | 105028,836 | | 777,213386 |
| 1014 | 27,296 | 1874,767 | 67852,57 | | 502,109018 |
| 1015 | 26,891 | 1873,891 | 93489,258 | | 691,820509 |
| 1016 | 21,431 | 1872,932 | 23245,711 | | 172,018261 |
| 1017 | 19,83 | 1872,86 | 146425,875 | | 1083,55148 |
| 1018 | 51,282 | 1869,592 | 141043,234 | | 1043,71993 |
| 1019 | 20,63 | 1867,838 | 31031,188 | | 229,630791 |
| 1020 | 27,992 | 1864,675 | 33520,336 | | 248,050486 |
| 1021 | 47,391 | 1864,278 | 57918,516 | | 428,597018 |
| 1022 | 20,099 | 1864,101 | 137271,891 | | 1015,81199 |
| 1023 | 35,869 | 1863,967 | 524659,438 | | 3882,47984 |
| 1024 | 27,239 | 1863,873 | 52788,363 | | 390,633886 |
| 1025 | 20,031 | 1863,141 | 139697,438 | | 1033,76104 |
| 1026 | 19,87 | 1860,926 | 13068,771 | | 96,7089054 |
| 1027 | 21,157 | 1860,926 | 81917,836 | | 606,191986 |
| 1028 | 26,764 | 1860,34 | 197601,531 | | 1462,25133 |
| 1029 | 27,282 | 1859,5 | 48407,289 | | 358,213939 |
| 1030 | 23,006 | 1859,03 | 193516,188 | | 1432,01979 |
| 1031 | 51,461 | 1858,652 | 43128,02 | | 319,147348 |
| 1032 | 22,605 | 1857,839 | 203838,828 | | 1508,40733 |
| 1033 | 19,267 | 1855,928 | 13138,126 | | 97,2221324 |
| 1034 | 20,105 | 1855,764 | 131766,063 | | 975,068866 |
| 1035 | 33,577 | 1854,908 | 733231,875 | | 5425,91588 |
| 1036 | 26,818 | 1851,8 | 200711,625 | | 1485,26603 |
| 1037 | 60,637 | 1848,46 | 6038,243 | | 44,6829982 |
| 1038 | 19,998 | 1848,263 | 99688,43 | | 737,694382 |
| 1039 | 27,542 | 1847,933 | 53558,961 | | 396,336311 |
| 1040 | 51,445 | 1847,586 | 194541,844 | | 1439,60965 |
| 1041 | 50,289 | 1846,06 | 144120,094 | | 1066,4887 |
| 1042 | 20,658 | 1845,847 | 131101,281 | | 970,149479 |
| 1043 | 26,69 | 1844,668 | 50529,566 | | 373,918788 |
| 1044 | 51,19 | 1844,662 | 11750,058 | | 86,9504292 |
| 1045 | 36,173 | 1843,622 | 40933,469 | | 302,907671 |
| 1046 | 50,239 | 1842,658 | 50739,762 | | 375,474239 |
| 1047 | 51,616 | 1842,648 | 61330,453 | | 453,845352 |
| 1048 | 22,565 | 1841,844 | 52639,223 | | 389,53025 |
| 1049 | 20,14 | 1839,786 | 104989,461 | | 776,922011 |
| 1050 | 26,58 | 1837,73 | 168274,531 | | 1245,23153 |
| 1051 | 27,295 | 1836,888 | 154800,984 | | 1145,52728 |
| 1052 | 19,861 | 1835,81 | 239974,203 | | 1775,8091 |
| 1053 | **26,859** | **1835,179** | **177662,859** | **3450,55729** | 1314,70516 |
| 1054 | 22,947 | 1834,847 | 112151,383 | | 829,920234 |
| 1055 | 22,831 | 1832,844 | 128282,289 | | 949,288939 |
| 1056 | 20,82 | 1831,312 | 39760,145 | | 294,225073 |
| 1057 | 51,429 | 1831,23 | 74989,969 | | 554,925771 |
| 1058 | 20,578 | 1829,857 | 18786,881 | | 139,022919 |
| 1059 | 50,43 | 1829,681 | 33509,582 | | 247,970907 |
| 1060 | 33,814 | 1829,529 | 37306,023 | | 276,06457 |
| 1061 | 49,958 | 1828,949 | 54472,137 | | 403,093814 |
| 1062 | 28,024 | 1827,115 | 55237,465 | | 408,757241 |
| 1063 | 50,335 | 1826,663 | 44625,695 | | 330,230143 |
| 1064 | 51,638 | 1826,652 | 57917,66 | | 428,590684 |
| 1065 | 47,405 | 1826,309 | 86090,477 | | 637,06953 |
| 1066 | 27,216 | 1825,887 | 48555,508 | | 359,310759 |
| 1067 | 20,099 | 1825,86 | 338040,688 | | 2501,50109 |
| 1068 | 23,404 | 1824,068 | 54327,395 | | 402,022723 |
| 1069 | 55,496 | 1823,55 | 1463,899 | | 10,8328526 |
| 1070 | 26,708 | 1822,845 | 185135,641 | | 1370,00374 |
| 1071 | 27,294 | 1821,908 | 36764,754 | | 272,05918 |
| 1072 | 26,419 | 1821,874 | 13089,423 | | 96,8617302 |
| 1073 | 51,474 | 1820,716 | 52165,934 | | 386,027912 |
| 1074 | 22,606 | 1819,804 | 580688,75 | | 4297,09675 |
| 1075 | 20,154 | 1817,761 | 1081253,88 | | 8001,27868 |
| 1076 | 37,543 | 1817,747 | 40002,078 | | 296,015377 |
| 1077 | 27,079 | 1813,82 | 933491,563 | | 6907,83757 |
| 1078 | 22,791 | 1812,882 | 126053,18 | | 932,793532 |
| 1079 | 60,619 | 1810,514 | 9529,108 | | 70,5153992 |
| 1080 | 27,679 | 1809,961 | 61372,824 | | 454,158898 |
| 1081 | 51,36 | 1809,662 | 166998,938 | | 1235,79214 |
| 1082 | 25,419 | 1808,785 | 99647,727 | | 737,39318 |
| 1083 | 32,362 | 1807,95 | 28583,354 | | 211,51682 |
| 1084 | 20,635 | 1807,641 | 329490,406 | | 2438,229 |
| 1085 | 22,31 | 1806,883 | 44412,402 | | 328,651775 |
| 1086 | 40,188 | 1805,588 | 418072,781 | | 3093,73858 |
| 1087 | 50,551 | 1804,673 | 858333,75 | | 6351,66975 |
| 1088 | 54,902 | 1804,666 | 19508,908 | | 144,365919 |
| 1089 | 47,258 | 1804,327 | 91234,57 | | 675,135818 |
| 1090 | 26,77 | 1803,88 | 266848,438 | | 1974,67844 |
| 1091 | 32,344 | 1801,343 | 78405,297 | | 580,199198 |
| 1092 | 30,974 | 1800,629 | 22093,713 | | 163,493476 |
| 1093 | 19,034 | 1800,052 | 117200,094 | | 867,280696 |
| 1094 | 20,101 | 1799,824 | 27975,473 | | 207,0185 |
| 1095 | 27,283 | 1798,711 | 106091,477 | | 785,07693 |
| 1096 | 26,789 | 1796,84 | 115289,117 | | 853,139466 |
| 1097 | 22,952 | 1794,908 | 403840,938 | | 2988,42294 |
| 1098 | 27,655 | 1793,973 | 137558,906 | | 1017,9359 |
| 1099 | 20,703 | 1793,821 | 42362,82 | | 313,484868 |
| 1100 | 26,926 | 1792,934 | 245127,938 | | 1813,94674 |
| 1101 | 25,423 | 1792,81 | 76797,188 | | 568,299191 |
| 1102 | 19,261 | 1790,148 | 27753,096 | | 205,37291 |
| 1103 | 26,987 | 1789,851 | 25226,754 | | 186,67798 |
| 1104 | 38,699 | 1789,61 | 59189,73 | | 438,004002 |
| 1105 | 26,216 | 1788,907 | 15748,181 | | 116,536539 |
| 1106 | 50,525 | 1788,693 | 893111,313 | | 6609,02372 |
| 1107 | 47,617 | 1788,321 | 6225,253 | | 46,0668722 |
| 1108 | 26,718 | 1787,886 | 220469,656 | | 1631,47545 |
| 1109 | 32,412 | 1785,707 | 138173,328 | | 1022,48263 |
| 1110 | 47,08 | 1783,395 | 13617,989 | | 100,773119 |
| 1111 | 24,05 | 1782,919 | 130107,336 | | 962,794286 |
| 1112 | 26,719 | 1779,838 | 13206,108 | | 97,7251992 |
| 1113 | 26,461 | 1778,925 | 77194,57 | | 571,239818 |
| 1114 | 19,165 | 1778,824 | 6165,233 | | 45,6227242 |
| 1115 | 19,281 | 1777,115 | 29204,076 | | 216,110162 |
| 1116 | 26,899 | 1775,901 | 368163,375 | | 2724,40898 |
| 1117 | 22,755 | 1775,826 | 568114,875 | | 4204,05008 |
| 1118 | 50,249 | 1772,72 | 125865,164 | | 931,402214 |
| 1119 | 51,599 | 1772,686 | 30238,418 | | 223,764293 |
| 1120 | 19,906 | 1772,576 | 13414,021 | | 99,2637554 |
| 1121 | 26,14 | 1771,789 | 20342,643 | | 150,535558 |
| 1122 | 60,659 | 1771,538 | 4335,641 | | 32,0837434 |
| 1123 | 25,395 | 1770,678 | 364467,063 | | 2697,05627 |
| 1124 | 32,44 | 1769,84 | 87244,375 | | 645,608375 |
| 1125 | 26,406 | 1768,832 | 73680,18 | | 545,233332 |
| 1126 | 27,285 | 1768 | 51965,754 | | 384,54658 |
| 1127 | 40,282 | 1767,639 | 1154890,88 | | 8546,19248 |
| 1128 | 23,421 | 1767,108 | 56689,273 | | 419,50062 |
| 1129 | 22,915 | 1767,084 | 339444,344 | | 2511,88815 |
| 1130 | 50,388 | 1766,72 | 1244047,25 | | 9205,94965 |
| 1131 | 54,846 | 1766,713 | 18705,223 | | 138,41865 |
| 1132 | 46,966 | 1766,364 | 27324,01 | | 202,197674 |
| 1133 | 26,826 | 1766,124 | 676256,625 | | 5004,29903 |
| 1134 | 27,6 | 1765,883 | 24261,887 | | 179,537964 |
| 1135 | 19,883 | 1764,796 | 43908,137 | | 324,920214 |
| 1136 | 30,95 | 1763,615 | 10578,803 | | 78,2831422 |
| 1137 | 32,353 | 1763,538 | 197962,688 | | 1464,92389 |
| 1138 | 52,236 | 1762,263 | 24308,041 | | 179,879503 |
| 1139 | 19,154 | 1761,794 | 275627,063 | | 2039,64027 |
| 1140 | 32,005 | 1761,361 | 33788,863 | | 250,037586 |
| 1141 | 26,342 | 1760,756 | 28904,85 | | 213,89589 |
| 1142 | 22,781 | 1759,833 | 111226,188 | | 823,073791 |
| 1143 | 26,285 | 1756,792 | 65174,434 | | 482,290812 |
| 1144 | 26,977 | 1755,048 | 485130,938 | | 3589,96894 |
| 1145 | 25,433 | 1754,849 | 154997,719 | | 1146,98312 |
| 1146 | 19,568 | 1752,837 | 9993,905 | | 73,954897 |
| 1147 | 26,365 | 1752,747 | 8704,931 | | 64,4164894 |
| 1148 | 34,142 | 1752,717 | 5691,779 | | 42,1191646 |
| 1149 | 38,509 | 1751,662 | 40417,168 | | 299,087043 |
| 1150 | 51,038 | 1751,619 | 21932,201 | | 162,298287 |
| 1151 | 51,655 | 1750,712 | 48846,887 | | 361,466964 |
| 1152 | 50,127 | 1750,711 | 321034,5 | | 2375,6553 |
| 1153 | 22,842 | 1750,641 | 298645,875 | | 2209,97948 |
| 1154 | 26,646 | 1749,91 | 151839,859 | | 1123,61496 |
| 1155 | 19,696 | 1749,416 | 18542,609 | | 137,215307 |
| 1156 | 47,314 | 1749,411 | 3008,298 | | 22,2614052 |
| 1157 | 27,975 | 1748,147 | 81581,477 | | 603,70293 |
| 1158 | 32,363 | 1747,731 | 89702,695 | | 663,799943 |
| 1159 | 55,946 | 1747,592 | 6465,131 | | 47,8419694 |
| 1160 | 26,449 | 1746,84 | 611283,23 | | 453,495902 |
| 1161 | 35,783 | 1745,692 | 9288,554 | | 68,7352996 |
| 1162 | 56,832 | 1745,568 | 6267,448 | | 46,3791152 |
| 1163 | 36,089 | 1745,067 | 41401,465 | | 306,370841 |
| 1164 | 22,138 | 1744,813 | 35727,973 | | 264,387 |
| 1165 | 36,42 | 1744,373 | 27876,617 | | 206,286966 |
| 1166 | 26,218 | 1740,802 | 26704,641 | | 197,614343 |
| 1167 | 20,752 | 1740,022 | 166723,391 | | 1233,75309 |
| 1168 | 23,583 | 1738,825 | 94085,523 | | 696,23287 |
| 1169 | 26,387 | 1738,795 | 81945,344 | | 606,395546 |
| 1170 | **26,797** | **1737,895** | **254024,781** | **2608,25236** | 1879,78338 |
| 1171 | 22,79 | 1737,77 | 751127,625 | | 5558,34443 |
| 1172 | 26,66 | 1736,361 | 112063,227 | | 829,26788 |
| 1173 | 19,845 | 1734,781 | 21712,186 | | 160,670176 |
| 1174 | 21,937 | 1734,705 | 26412,207 | | 195,450332 |
| 1175 | 26,121 | 1733,55 | 44750,543 | | 331,154018 |
| 1176 | 25,448 | 1732,885 | 675405,813 | | 4998,00302 |
| 1177 | 26,799 | 1731,178 | 590140,125 | | 4367,03693 |
| 1178 | 51,061 | 1730,476 | 53972,723 | | 399,39815 . |
| 1179 | 40,062 | 1729,677 | 777807,625 | | 5755,77643 |
| 1180 | 50,108 | 1728,761 | 243423,203 | | 1801,3317 |
| 1181 | 51,671 | 1728,713 | 12968,45 | | 95,96653 |
| 1182 | 33,569 | 1727,797 | 336655,563 | | 2491,25117 |
| 1183 | 32,353 | 1725,586 | 166671,5 | | 1233,3691 |
| 1184 | 18,942 | 1724,448 | 9412,43 | | 69,651982 |
| 1185 | 52,194 | 1724,241 | 44839,199 | | 331,810073 |
| 1186 | 31,985 | 1723,47 | 62237,195 | | 460,555243 |
| 1187 | 26,609 | 1722,557 | 453472,031 | | 3355,69303 |
| 1188 | 19,582 | 1720,78 | 74309,211 | | 549,888161 |
| 1189 | 27,777 | 1719,806 | 8670,013 | | 64,1580962 |
| 1190 | 26,311 | 1718,82 | 258650,984 | | 1914,01728 |
| 1191 | 22,038 | 1718,203 | 26217,236 | | 194,007546 |
| 1192 | 22,806 | 1717,892 | 71530,109 | | 529,322807 |
| 1193 | 22,835 | 1717,027 | 79037,438 | | 584,877041 |
| 1194 | 25,359 | 1716,873 | 78706,383 | | 582,427234 |
| 1195 | 20,092 | 1716,75 | 98706,563 | | 730,428566 |
| 1196 | 22,344 | 1716,589 | 114901,781 | | 850,273179 |
| 1197 | 56,213 | 1715,625 | 8737,123 | | 64,6547102 |
| 1198 | 51,077 | 1714,783 | 12487,07 | | 92,404318 |
| 1199 | 26,44 | 1712,803 | 97550,773 | | 721,87572 |
| 1200 | 21,961 | 1712,729 | 167742,406 | | 1241,2938 |
| 1201 | 33,485 | 1711,815 | 325279,094 | | 2407,0653 |
| 1202 | 19,769 | 1709,765 | 8169,03 | | 60,450822 |
| 1203 | 26,903 | 1708,88 | 616257 | | 4560,3018 |
| 1204 | 37,1 | 1707,723 | 573278,438 | | 4242,26044 |
| 1205 | 22,128 | 1706,846 | 147145,969 | | 1088,88017 |
| 1206 | 51,017 | 1706,819 | 8774,919 | | 64,9344006 |
| 1207 | 33,49 | 1705,824 | 107182,141 | | 793,147843 |
| 1208 | 26,213 | 1702,549 | 107273,141 | | 793,821243 |
| 1209 | 19,719 | 1701,773 | 10910,079 | | 80,7345846 |
| 1210 | 19,004 | 1701,117 | 286517 | | 2120,2258 |
| 1211 | 26,439 | 1700,815 | 121645,578 | | 900,177277 |
| 1212 | 22,605 | 1700,526 | 452406,844 | | 3347,81065 |
| 1213 | 31,892 | 1698,717 | 236323,828 | | 1748,79633 |
| 1214 | 26,019 | 1697,79 | 32841,801 | | 243,029327 |
| 1215 | 26,766 | 1697,722 | 293863,219 | | 2174,58782 |
| 1216 | 26,392 | 1696,803 | 58345,828 | | 431,759127 |
| 1217 | 22,005 | 1696,517 | 226208,672 | | 1673,94417 |
| 1218 | 33,389 | 1695,815 | 116949,117 | | 865,423466 |
| 1219 | 22,263 | 1693,709 | 34169,855 | | 252,856927 |
| 1220 | 20,506 | 1693,689 | 39384,203 | | 291,443102 |
| 1221 | 22,614 | 1693,346 | 26959,088 | | 199,497251 |
| 1222 | 26,76 | 1692,798 | 553402,438 | | 4095,17804 |
| 1223 | 51,073 | 1692,162 | 64894,574 | | 480,219848 |
| 1224 | 36,441 | 1691,753 | 474631,719 | | 3512,27472 |
| 1225 | 38,476 | 1691,74 | 71431,195 | | 528,590843 |
| 1226 | 33,701 | 1689,84 | 967976,563 | | 7163,02657 |
| 1227 | 20,188 | 1688,745 | 58794,035 | | 435,075859 |
| 1228 | 31,275 | 1688,712 | 23481,848 | | 173,765675 |
| 1229 | 22,111 | 1687,759 | 52805,07 | | 390,757518 |
| 1230 | 19,519 | 1687,737 | 8106,24 | | 59,986176 |
| 1231 | 32,032 | 1687,441 | 57712,715 | | 427,074091 |
| 1232 | 27,427 | 1686,789 | 19094,982 | | 141,302867 |
| 1233 | 26,916 | 1686,781 | 15861,432 | | 117,374597 |
| 1234 | 30,394 | 1686,748 | 8055,863 | | 59,6133862 |
| 1235 | 52,123 | 1686,612 | 18216,238 | | 134,800161 |
| 1236 | 20,925 | 1685,791 | 14135,824 | | 104,605098 |
| 1237 | 26,848 | 1684,778 | 1528617,38 | | 11311,7686 |
| 1238 | 51,06 | 1683,539 | 7311,187 | | 54,1027838 |
| 1239 | 56,025 | 1681,469 | 1456,791 | | 10,7802534 |
| 1240 | 26,327 | 1680,917 | 562451,125 | | 4162,13833 |
| 1241 | 50,711 | 1680,552 | 14415,57 | | 106,675218 |
| 1242 | 23,374 | 1680,067 | 13454,194 | | 99,5610356 |
| 1243 | 22,867 | 1680,06 | 1558934,5 | | 11536,1153 |
| 1244 | 22,348 | 1678,716 | 915839,25 | | 6777,21045 |
| 1245 | 20,024 | 1676,707 | 15076,088 | | 111,563051 |
| 1246 | 51,162 | 1675,553 | 15218,188 | | 112,614591 |
| 1247 | 21,999 | 1674,767 | 657963,75 | | 4868,93175 |
| 1248 | 26,48 | 1674,765 | 332114,281 | | 2457,64568 |
| 1249 | 35,911 | 1674,743 | 13065,354 | | 96,6836196 |
| 1250 | 33,635 | 1673,848 | 621973,75 | | 4602,60575 |
| 1251 | 37,217 | 1673,808 | 10630,163 | | 78,6632062 |
| 1252 | 31,406 | 1673,712 | 59847,359 | | 442,870457 |
| 1253 | 60,508 | 1673,41 | 3005,677 | | 22,2420098 |
| 1254 | 26,223 | 1673,293 | 89194,797 | | 660,041498 |
| 1255 | 20,472 | 1672,139 | 5100,346 | | 37,7425604 |
| 1256 | 37,138 | 1669,765 | 1178668,63 | | 8722,14783 |
| 1257 | 21,202 | 1669,559 | 36920,801 | | 273,213927 |
| 1258 | 51,231 | 1669,502 | 17024,83 | | 125,983742 |
| 1259 | 51,002 | 1668,263 | 32127,834 | | 237,745972 |
| 1260 | 22,682 | 1667,961 | 109042,328 | | 806,913227 |
| 1261 | 19,391 | 1667,748 | 14688,896 | | 108,69783 |
| 1262 | 26,233 | 1664,869 | 140821,266 | | 1042,07737 |
| 1263 | 50,726 | 1664,583 | 47740,727 | | 353,28138 |
| 1264 | 19,792 | 1663,743 | 15229,608 | | 112,699099 |
| 1265 | 23,647 | 1662,778 | 25791,463 | | 190,856826 |
| 1266 | 22,348 | 1662,716 | 194397,094 | | 1438,5385 |
| 1267 | 32,006 | 1662,609 | 175913,266 | | 1301,75817 |
| 1268 | 22,899 | 1661,776 | 484126,406 | | 3582,5354 |
| 1269 | 26,062 | 1659,819 | 69192,805 | | 512,026757 |
| 1270 | 26,413 | 1658,822 | 156914,141 | | 1161,16464 |
| 1271 | 22,01 | 1658,774 | 394689,219 | | 2920,70022 |
| 1272 | 25,845 | 1658,703 | 63723,551 | | 471,554277 |
| 1273 | 33,461 | 1657,828 | 80278,719 | | 594,062521 |
| 1274 | 20,167 | 1657,767 | 21783,213 | | 161,195776 |
| 1275 | 26,24 | 1655,771 | 115510,375 | | 854,776775 |
| 1276 | 25,813 | 1655,737 | 9132,748 | | 67,5823352 |
| 1277 | 20,067 | 1655,57 | 79395,461 | | 587,526411 |
| 1278 | 33,143 | 1654,782 | 45294,531 | | 335,179529 |
| 1279 | 26,579 | 1654,285 | 154289,203 | | 1141,7401 |
| 1280 | 39,156 | 1653,766 | 4578,064 | | 33,8776736 |
| 1281 | 51,132 | 1653,527 | 58067,93 | | 429,702682 |
| 1282 | 36,793 | 1653,059 | 534220,188 | | 3953,22939 |
| 1283 | 19,419 | 1652,746 | 15048,502 | | 111,358915 |
| 1284 | 33,485 | 1651,875 | 413598 | | 3060,6252 |
| 1285 | 34,676 | 1651,855 | 16018,068 | | 118,533703 |
| 1286 | 54,087 | 1651,453 | 14638,714 | | 108,326484 |
| 1287 | 19,538 | 1649,803 | 85486,398 | | 632,599345 |
| 1288 | 26,166 | 1649,782 | 13777,515 | | 101,953611 |
| 1289 | 22,145 | 1649,26 | 149952,375 | | 1109,64758 |
| 1290 | 45,247 | 1649,172 | 16909,164 | | 125,127814 |
| 1291 | 26,493 | 1647,797 | 148031,422 | | 1095,43252 |
| 1292 | 27,777 | 1647,787 | 11980,709 | | 88,6572466 |
| 1293 | 19,142 | 1647,559 | 26187,014 | | 193,783904 |
| 1294 | 60,612 | 1647,414 | 8742,313 | | 64,6931162 |
| 1295 | 20,618 | 1645,815 | 32788,332 | | 242,633657 |
| 1296 | 22,892 | 1645,794 | 215293,266 | | 1593,17017 |
| 1297 | 39,588 | 1644,614 | 24574,563 | | 181,851766 |
| 1298 | 40,996 | 1644,614 | 11609,523 | | 85,9104702 |
| 1299 | 41,529 | 1644,612 | 19104,215 | | 141,371191 |
| 1300 | 26,136 | 1643,782 | 40699,555 | | 301,176707 |
| 1301 | 50,713 | 1642,598 | 458311,781 | | 3391,50718 |
| 1302 | 22,368 | 1640,664 | 1787474 | | 13227,3076 |
| 1303 | 26,149 | 1639,423 | 79805,805 | | 590,562957 |
| 1304 | 20,164 | 1638,929 | 446489,313 | | 3304,02092 |
| 1305 | 31,575 | 1637,789 | 17758,281 | | 131,411279 |
| 1306 | 19,439 | 1637,762 | 13106,555 | | 96,988507 |
| 1307 | 33,079 | 1637,729 | 101983 | | 754,6742 |
| 1308 | 35,229 | 1637,691 | 8278,406 | | 61,2602044 |
| 1309 | 56,178 | 1637,442 | 1820,801 | | 13,4739274 |
| 1310 | 22,209 | 1636,865 | 1771329,38 | | 13107,8374 |
| 1311 | 19,979 | 1636,77 | 55767,305 | | 412,678057 |
| 1312 | 26,466 | 1636,56 | 493809,594 | | 3654,191 |
| 1313 | 33,565 | 1635,841 | 15748,322 | | 116,537583 |
| 1314 | 20,32 | 1635,767 | 14488,574 | | 107,215448 |
| 1315 | 26,222 | 1634,903 | 57033,582 | | 422,048507 |
| 1316 | 26,766 | 1634,835 | 31530,076 | | 233,322562 |
| 1317 | 30,976 | 1634,755 | 38005,172 | | 281,238273 |
| 1318 | 26,945 | 1633,589 | 57031,723 | | 422,03475 |
| 1319 | 56,093 | 1633,539 | 4547,565 | | 33,651981 |
| 1320 | 19,445 | 1633,168 | 33451,688 | | 247,542491 |
| 1321 | 33,285 | 1632,811 | 344321,844 | | 2547,98165 |
| 1322 | 37,063 | 1631,805 | 1000832,06 | | 7406,15727 |
| 1323 | 51,14 | 1631,544 | 14658,414 | | 108,472264 |
| 1324 | 19,444 | 1630,801 | 9351,153 | | 69,1985322 |
| 1325 | 20,708 | 1629,062 | 41170,219 | | 304,659621 |
| 1326 | 31,555 | 1628,503 | 27959,01 | | 206,896674 |
| 1327 | 26,276 | 1627,183 | 152365,281 | | 1127,50308 |
| 1328 | 49,992 | 1626,622 | 129430,719 | | 957,787321 |
| 1329 | 51,37 | 1626,608 | 221118,734 | | 1636,27863 |
| 1330 | 23,667 | 1624,787 | 74086,891 | | 548,242993 |
| 1331 | 31,977 | 1624,65 | 151979,656 | | 1124,64945 |
| 1332 | 22,91 | 1623,802 | 813636,25 | | 6020,90825 |
| 1333 | 20,049 | 1623,721 | 48035,508 | | 355,462759 |
| 1334 | 26,115 | 1622,752 | 54070,082 | | 400,118607 |
| 1335 | 26,587 | 1622,569 | 78495,656 | | 580,867854 |
| 1336 | 31,614 | 1622,541 | 48132,473 | | 356,1803 |
| 1337 | 51,449 | 1622,516 | 42449,113 | | 314,123436 |
| 1338 | 19,446 | 1621,752 | 14741,807 | | 109,089372 |
| 1339 | 33,103 | 1621,727 | 169860,656 | | 1256,96885 |
| 1340 | 36,26 | 1621,714 | 10330,106 | | 76,4427844 |
| 1341 | 25,866 | 1620,713 | 85293,617 | | 631,172766 |
| 1342 | 48,018 | 1619,242 | 4286,134 | | 31,7173916 |
| 1343 | 54,868 | 1618,342 | 4299,275 | | 31,814635 |
| 1344 | 26,239 | 1617,782 | 276657,656 | | 2047,26665 |
| 1345 | 55,144 | 1617,691 | 7426,702 | | 54,9575948 |
| 1346 | 33,243 | 1616,145 | 397344,281 | | 2940,34768 |
| 1347 | 60,515 | 1615,419 | 12032,381 | | 89,0396194 |
| 1348 | 22,542 | 1614,932 | 209708,531 | | 1551,84313 |
| 1349 | 19,439 | 1614,786 | 43254,602 | | 320,084055 |
| 1350 | 36,176 | 1614,752 | 366360,5 | | 2711,0677 |
| 1351 | 22,89 | 1613,878 | 29076,914 | | 215,169164 |
| 1352 | 31,579 | 1612,537 | 33888,555 | | 250,775307 |
| 1353 | 50,626 | 1610,636 | 57666,223 | | 426,73005 |
| 1354 | 51,796 | 1610,61 | 5026,365 | | 37,195101 |
| 1355 | 49,488 | 1610,521 | 27563,52 | | 203,970048 |
| 1356 | 26,476 | 1609,682 | 249802,609 | | 1848,53931 |
| 1357 | 19,82 | 1607,438 | 22530,867 | | 166,728416 |
| 1358 | 40,415 | 1606,62 | 6549,202 | | 48,4640948 |
| 1359 | 51,431 | 1606,541 | 53775,559 | | 397,939137 |
| 1360 | 60,308 | 1606,455 | 20217,408 | | 149,608819 |
| 1361 | 45,153 | 1606,051 | 104704,188 | | 774,810991 |
| 1362 | 33,093 | 1605,74 | 107556,352 | | 795,917005 |
| 1363 | 52,135 | 1604,625 | 27542,211 | | 203,812361 |
| 1364 | 51,752 | 1604,623 | 19708,334 | | 145,841672 |
| 1365 | 50,377 | 1604,425 | 498449,438 | | 3688,52584 |
| 1366 | 25,879 | 1602,593 | 80256,023 | | 593,89457 |
| 1367 | 20,142 | 1602,029 | 283347,281 | | 2096,76988 |
| 1368 | 26,208 | 1601,483 | 174360,688 | | 1290,26909 |
| 1369 | 21,843 | 1600,709 | 71368,32 | | 528,125568 |
| 1370 | 33,841 | 1599,775 | 1946478,5 | | 14403,9409 |
| 1371 | 26,365 | 1595,636 | 99707,125 | | 737,832725 |
| 1372 | 55,052 | 1595,379 | 13544,353 | | 100,228212 |
| 1373 | 33,255 | 1594,838 | 133055,125 | | 984,607925 |
| 1374 | 19,115 | 1594,616 | 9341,319 | | 69,1257606 |
| 1375 | 19,447 | 1592,784 | 123089,523 | | 910,86247 |
| 1376 | 52,334 | 1590,926 | 19367,498 | | 143,319485 |
| 1377 | 32,656 | 1590,783 | 42339,105 | | 313,309377 |
| 1378 | 27,674 | 1590,767 | 29748,822 | | 220,141283 |
| 1379 | 31,605 | 1590,551 | 240303,016 | | 1778,24232 |
| 1380 | 51,487 | 1590,549 | 28733,951 | | 212,631237 |
| 1381 | 60,485 | 1590,45 | 6380,994 | | 47,2193556 |
| 1382 | 20,136 | 1589,801 | 129593,336 | | 958,990686 |
| 1383 | 26,161 | 1589,765 | 28449,627 | | 210,52724 |
| 1384 | 26,465 | 1588,799 | 144476,25 | | 1069,12425 |
| 1385 | 50,453 | 1588,666 | 91979,852 | | 680,650905 |
| 1386 | 19,006 | 1588,024 | 23308,154 | | 172,48034 |
| 1387 | 32,774 | 1587,746 | 61703,977 | | 456,60943 |
| 1388 | 25,703 | 1587,197 | 49110,512 | | 363,417789 |
| 1389 | 19,518 | 1585,876 | 20618,105 | | 152,573977 |
| 1390 | 51,336 | 1584,592 | 147183,422 | | 1089,15732 |
| 1391 | 31,605 | 1584,465 | 112075,383 | | 829,357834 |
| 1392 | 26,004 | 1584,374 | 152446,016 | | 1128,10052 |
| 1393 | 33,983 | 1583,763 | 1606523,13 | | 11888,2711 |
| 1394 | 19,229 | 1583,691 | 41368,191 | | 306,124613 |
| 1395 | 60,592 | 1583,443 | 10818,154 | | 80,0543396 |
| 1396 | 22,462 | 1583,401 | 51175,965 | | 378,702141 |
| 1397 | 23,455 | 1580,979 | 750475,688 | | 5553,52009 |
| 1398 | 22,879 | 1580,944 | 82073,273 | | 607,34222 |
| 1399 | **26,267** | **1579,821** | **460781,469** | **2618,98951** | 3409,78287 |
| 1400 | 20,062 | 1579,781 | 1163685,25 | | 8611,27085 |
| 1401 | 55,029 | 1579,406 | 35646,781 | | 263,786179 |
| 1402 | 19,223 | 1578,194 | 34980,523 | | 258,85587 |
| 1403 | 34,082 | 1577,542 | 1220426,38 | | 9031,15518 |
| 1404 | 19,43 | 1577,048 | 211572,891 | | 1565,63939 |
| 1405 | 52,329 | 1575,122 | 15242,363 | | 112,793486 |
| 1406 | 31,579 | 1574,581 | 56584,949 | | 418,728623 |
| 1407 | 26,771 | 1573,768 | 11005,183 | | 81,4383542 |
| 1408 | 26,34 | 1573,766 | 88419,383 | | 654,303434 |
| 1409 | 25,642 | 1571,653 | 18782,912 | | 138,993549 |
| 1410 | 32,726 | 1570,316 | 86676,258 | | 641,404309 |
| 1411 | 26,215 | 1569,753 | 18192,246 | | 134,62262 |
| 1412 | 52,301 | 1569,494 | 49528,332 | | 366,509657 |
| 1413 | 25,953 | 1568,74 | 99835,68 | | 738,784032 |
| 1414 | 51,381 | 1568,572 | 103803,664 | | 768,147114 |
| 1415 | 60,515 | 1568,367 | 17354,266 | | 128,421568 |
| 1416 | 33,2 | 1567,797 | 434310,063 | | 3213,89447 |
| 1417 | 20,165 | 1567,795 | 372919,156 | | 2759,60175 |
| 1418 | 35,532 | 1567,755 | 7017,221 | | 51,9274354 |
| 1419 | 34,952 | 1567,754 | 19015,121 | | 140,711895 |
| 1420 | 22,644 | 1567,696 | 14336,802 | | 106,092335 |
| 1421 | 19,412 | 1567,693 | 15762,474 | | 116,642308 |
| 1422 | 25,669 | 1565,649 | 107737,258 | | 797,255709 |
| 1423 | 31,653 | 1563,506 | 256880,891 | | 1900,91859 |
| 1424 | 55,037 | 1563,437 | 31753,439 | | 234,975449 |
| 1425 | 21,99 | 1562,683 | 442783,563 | | 3276,59837 |
| 1426 | 31,395 | 1562,551 | 49908,277 | | 369,32125 |
| 1427 | 45,126 | 1562,09 | 120262,977 | | 889,94603 |
| 1428 | 33,892 | 1561,784 | 4378570,5 | | 32401,4217 |
| 1429 | 26,083 | 1560,761 | 172583,031 | | 1277,11443 |
| 1430 | 54,978 | 1557,43 | 57674,145 | | 426,788673 |
| 1431 | 47,314 | 1557,371 | 2242,944 | | 16,5977856 |
| 1432 | 25,21 | 1556,715 | 21400,275 | | 158,362035 |
| 1433 | 19,157 | 1556,658 | 40701,605 | | 301,191877 |
| 1434 | 25,762 | 1555,668 | 10494,886 | | 77,6621564 |
| 1435 | 52,317 | 1553,479 | 107733,453 | | 797,227552 |
| 1436 | 31,602 | 1552,596 | 164191 | | 1215,0134 |
| 1437 | 60,548 | 1552,244 | 18100,105 | | 133,940777 |
| 1438 | 32,949 | 1552,029 | 108975,336 | | 806,417486 |
| 1439 | 51,293 | 1551,536 | 9862,231 | | 72,9805094 |
| 1440 | 20,335 | 1551,002 | 41674,418 | | 308,390693 |
| 1441 | 26,24 | 1550,756 | 123754,648 | | 915,784395 |
| 1442 | 25,661 | 1549,661 | 181339,203 | | 1341,9101 |
| 1443 | 31,599 | 1547,554 | 126639,313 | | 937,130916 |
| 1444 | 52,2 | 1547,002 | 3902,257 | | 28,8767018 |
| 1445 | 26,148 | 1546,781 | 397945,156 | | 2944,79415 |
| 1446 | 51,365 | 1546,585 | 141115,656 | | 1044,25585 |
| 1447 | 18,965 | 1546,009 | 8138,867 | | 60,2276158 |
| 1448 | 33,874 | 1545,796 | 2482156,75 | | 18367,96 |
| 1449 | 22,718 | 1545,666 | 96470,289 | | 713,880139 |
| 1450 | 25,71 | 1543,679 | 57332,781 | | 424,262579 |
| 1451 | 55,025 | 1541,204 | 81368,375 | | 602,125975 |
| 1452 | 33,187 | 1539,813 | 342826,781 | | 2536,91818 |
| 1453 | 22,859 | 1539,745 | 17030,666 | | 126,026928 |
| 1454 | 26,143 | 1538,782 | 47694,641 | | 352,940343 |
| 1455 | 52,335 | 1537,494 | 74524,922 | | 551,484423 |
| 1456 | 54,213 | 1537,361 | 3714,262 | | 27,4855388 |
| 1457 | 20,394 | 1536,787 | 47255,895 | | 349,693623 |
| 1458 | 60,536 | 1536,53 | 5179,255 | | 38,326487 |
| 1459 | 26,437 | 1535,394 | 206936,766 | | 1531,33207 |
| 1460 | 26,032 | 1534,45 | 21739,525 | | 160,872485 |
| 1461 | 25,657 | 1533,617 | 116876,422 | | 864,885523 |
| 1462 | 31,429 | 1533,537 | 35516,992 | | 262,825741 |
| 1463 | 32,719 | 1533,021 | 52503,387 | | 388,525064 |
| 1464 | 52,327 | 1531,486 | 189285,625 | | 1400,71363 |
| 1465 | 58,795 | 1531,259 | 3579,956 | | 26,4916744 |
| 1466 | 25,934 | 1530,757 | 53750,309 | | 397,752287 |
| 1467 | 33,111 | 1529,795 | 354812,281 | | 2625,61088 |
| 1468 | 51,31 | 1529,754 | 68137,281 | | 504,215879 |
| 1469 | 22,669 | 1529,696 | 97531,555 | | 721,733507 |
| 1470 | 25,708 | 1527,677 | 557475 | | 4125,315 |
| 1471 | 21,377 | 1525,647 | 95958,109 | | 710,090007 |
| 1472 | 31,604 | 1525,585 | 224192,984 | | 1659,02808 |
| 1473 | 21,109 | 1524,714 | 50138,973 | | 371,0284 |
| 1474 | 20,247 | 1524,422 | 26032,813 | | 192,642816 |
| 1475 | 21,84 | 1523,845 | 1315288,25 | | 9733,13305 |
| 1476 | 33,882 | 1523,824 | 2137835,75 | | 15819,9846 |
| 1477 | 22,403 | 1522,807 | 56880,883 | | 420,918534 |
| 1478 | 26,05 | 1522,795 | 225871,531 | | 1671,44933 |
| 1479 | 20,691 | 1522,736 | 65239,566 | | 482,772788 |
| 1480 | 32,659 | 1522,66 | 48612,965 | | 359,735941 |
| 1481 | 52,275 | 1521,537 | 11822,047 | | 87,4831478 |
| 1482 | 55,043 | 1519,4 | 112879,547 | | 835,308648 |
| 1483 | 19,173 | 1518,7 | 310531,188 | | 2297,93079 |
| 1484 | 45,108 | 1518,042 | 159780,984 | | 1182,37928 |
| 1485 | 42,505 | 1517,544 | 9947,583 | | 73,6121142 |
| 1486 | 32,712 | 1517,013 | 81436,086 | | 602,627036 |
| 1487 | 52,328 | 1515,49 | 260667 | | 1928,9358 |
| 1488 | 51,064 | 1515,057 | 40951,52 | | 303,041248 |
| 1489 | 25,304 | 1514,678 | 105488,594 | | 780,615596 |
| 1490 | 31,295 | 1514,467 | 213922,516 | | 1583,02662 |
| 1491 | 58,997 | 1514,391 | 2223,704 | | 16,4554096 |
| 1492 | 33,008 | 1513,778 | 31166,486 | | 230,631996 |
| 1493 | 51,393 | 1513,492 | 6028,764 | | 44,6128536 |
| 1494 | 25,684 | 1511,67 | 533613,25 | | 3948,73805 |
| 1495 | 59,581 | 1511,401 | 7321,546 | | 54,1794404 |
| 1496 | 20,163 | 1510,747 | 120274,336 | | 890,030086 |
| 1497 | 23,298 | 1510,736 | 31251,807 | | 231,263372 |
| 1498 | 52,276 | 1509,5 | 7264,465 | | 53,757041 |
| 1499 | 21,434 | 1509,107 | 22580,268 | | 167,093983 |
| 1500 | **26,035** | **1508,878** | **416968,781** | **2402,66451** | 3085,56898 |
| 1501 | 21,099 | 1508,716 | 26668,498 | | 197,346885 |
| 1502 | 22,714 | 1507,972 | 296770,906 | | 2196,1047 |
| 1503 | 33,166 | 1507,821 | 377186,125 | | 2791,17733 |
| 1504 | 19,343 | 1507,692 | 21455,111 | | 158,767821 |
| 1505 | 51,294 | 1507,543 | 59747,996 | | 442,13517 |
| 1506 | 25,715 | 1505,656 | 93126,773 | | 689,13812 |
| 1507 | 21,393 | 1504,976 | 224635,625 | | 1662,30363 |
| 1508 | 31,026 | 1504,421 | 61428,184 | | 454,568562 |
| 1509 | 55,107 | 1503,301 | 55199,215 | | 408,474191 |
| 1510 | 22,308 | 1500,84 | 38119,215 | | 282,082191 |
| 1511 | 19,418 | 1500,692 | 21493,119 | | 159,049081 |
| 1512 | 21,883 | 1500,677 | 26562,451 | | 196,562137 |
| 1513 | 32,646 | 1500,676 | 267310,469 | | 1978,09747 |
| 1514 | 52,314 | 1499,535 | 113653,813 | | 841,038216 |
| 1515 | 51,11 | 1499,471 | 22515,533 | | 166,614944 |
| 1516 | 31,725 | 1499,319 | 25246,293 | | 186,822568 |
| 1517 | 31,342 | 1498,522 | 205765,641 | | 1522,66574 |
| 1518 | 56,804 | 1497,389 | 8472,941 | | 62,6997634 |
| 1519 | 31,431 | 1495,577 | 37132,027 | | 274,777 |
| 1520 | 25,701 | 1495,51 | 180634,594 | | 1336,696 |
| 1521 | 59,603 | 1495,327 | 12399,307 | | 91,7548718 |
| 1522 | 45,048 | 1494,97 | 42989,801 | | 318,124527 |
| 1523 | 26,474 | 1494,756 | 60827,902 | | 450,126475 |
| 1524 | 56,739 | 1494,4 | 15389,742 | | 113,884091 |
| 1525 | 50,703 | 1494,051 | 49096,602 | | 363,314855 |
| 1526 | 52,323 | 1493,535 | 513625,938 | | 3800,83194 |
| 1527 | 58,794 | 1493,276 | 3180,229 | | 23,5336946 |
| 1528 | 33,034 | 1491,809 | 39573,719 | | 292,845521 |
| 1529 | 22,69 | 1491,715 | 62520,961 | | 462,655111 |
| 1530 | 51,31 | 1490,596 | 64390,566 | | 476,490188 |
| 1531 | 25,699 | 1489,703 | 1324499,13 | | 9801,29353 |
| 1532 | 27,749 | 1489,695 | 35656,855 | | 263,860727 |
| 1533 | 21,937 | 1488,703 | 58306,789 | | 431,470239 |
| 1534 | 26,674 | 1488,673 | 12532,065 | | 92,737281 |
| 1535 | 26,864 | 1488,669 | 9989,638 | | 73,9233212 |
| 1536 | 31,025 | 1488,435 | 115011,352 | | 851,084005 |
| 1537 | 20,064 | 1487,66 | 60555,18 | | 448,108332 |
| 1538 | 57,5 | 1487,261 | 22979,725 | | 170,049965 |
| 1539 | 21,102 | 1486,746 | 86114,773 | | 637,24932 |
| 1540 | 20,302 | 1485,814 | 35362,301 | | 261,681027 |
| 1541 | 19,391 | 1485,744 | 35368,121 | | 261,724095 |
| 1542 | 22,765 | 1485,739 | 325309,406 | | 2407,2896 |
| 1543 | 21,7 | 1485,577 | 179972,781 | | 1331,79858 |
| 1544 | 32,638 | 1484,686 | 284208,563 | | 2103,14337 |
| 1545 | 20,646 | 1483,769 | 99830,844 | | 738,748246 |
| 1546 | 31,547 | 1483,497 | 66147,68 | | 489,492832 |
| 1547 | 21,97 | 1483,484 | 101875,852 | | 753,881305 |
| 1548 | 25,512 | 1482,712 | 47738,512 | | 353,264989 |
| 1549 | 59,952 | 1482,332 | 3723,846 | | 27,5564604 |
| 1550 | 54,947 | 1481,514 | 27755,322 | | 205,389383 |
| 1551 | 51,342 | 1480,523 | 8275,503 | | 61,2387222 |
| 1552 | 59,684 | 1480,179 | 10350,676 | | 76,5950024 |
| 1553 | 19,868 | 1479,653 | 9464,678 | | 70,0386172 |
| 1554 | 51,641 | 1479,497 | 11117,603 | | 82,2702622 |
| 1555 | 32,695 | 1478,703 | 170849,156 | | 1264,28375 |
| 1556 | 56,784 | 1478,422 | 25985,34 | | 192,291516 |
| 1557 | 19,792 | 1477,648 | 12052,142 | | 89,1858508 |
| 1558 | 52,023 | 1477,537 | 506844,188 | | 3750,64699 |
| 1559 | 26,012 | 1476,751 | 22272,973 | | 164,82 |
| 1560 | 25,289 | 1476,723 | 292965,156 | | 2167,94215 |
| 1561 | 31,286 | 1476,502 | 618812175 | | 4579,21435 |
| 1562 | 50,115 | 1475,578 | 34773,316 | | 257,322538 |
| 1563 | 42,959 | 1475,564 | 14523,597 | | 107,474618 |
| 1564 | 42,656 | 1475,558 | 14509,699 | | 107,371773 |
| 1565 | 18,991 | 1474,936 | 168177,938 | | 1244,51674 |
| 1566 | 59,531 | 1474,323 | 12192,879 | | 90,2273046 |
| 1567 | 45,092 | 1474,032 | 172501,25 | | 1276,50925 |
| 1568 | 25,694 | 1473,735 | 732376,563 | | 5419,58657 |
| 1569 | 22,004 | 1473,732 | 141438,281 | | 1046,64328 |
| 1570 | 57,184 | 1472,278 | 4585,247 | | 33,9308278 |
| 1571 | 58,089 | 1471,285 | 3652,961 | | 27,0319114 |
| 1572 | 21,032 | 1470,737 | 39272,316 | | 290,615138 |
| 1573 | 22,699 | 1469,663 | 479302,844 | | 3546,84105 |
| 1574 | 51,293 | 1469,563 | 30035,428 | | 222,262167 |
| 1575 | 23,445 | 1467,89 | 471461,313 | | 3488,81372 |
| 1576 | 22,953 | 1467,864 | 57678,805 | | 426,823157 |
| 1577 | 22,184 | 1467,716 | 12644,103 | | 93,5663622 |
| 1578 | 21,462 | 1466,901 | 629920,375 | | 4661,41078 |
| 1579 | 25,577 | 1466,726 | 42258,711 | | 312,714461 |
| 1580 | 31,043 | 1466,436 | 690857,25 | | 5112,34365 |
| 1581 | 57,749 | 1466,294 | 25648,756 | | 189,800794 |
| 1582 | 26,832 | 1464,747 | 59844,988 | | 442,852911 |
| 1583 | 52,112 | 1464,549 | 33567,551 | | 248,399877 |
| 1584 | 19,391 | 1463,779 | 92841,156 | | 687,024554 |
| 1585 | 21,836 | 1463,622 | 277416,719 | | 2052,88372 |
| 1586 | 18,809 | 1462,708 | 7145,363 | | 52,8756862 |
| 1587 | 33,265 | 1462,706 | 1376367,75 | | 10185,1214 |
| 1588 | 26,317 | 1461,692 | 7774,391 | | 57,5304934 |
| 1589 | 52,389 | 1461,566 | 83025,867 | | 614,391416 |
| 1590 | 51,105 | 1461,536 | 144334,891 | | 1068,07819 |
| 1591 | 31,735 | 1461,262 | 232331,219 | | 1719,25102 |
| 1592 | 21,435 | 1460,957 | 169996,313 | | 1257,97272 |
| 1593 | 25,385 | 1460,724 | 306236,313 | | 2266,14872 |
| 1594 | 31,326 | 1460,56 | 238888,328 | | 1767,77363 |
| 1595 | 59,746 | 1458,356 | 37428,387 | | 276,970064 |
| 1596 | 25,61 | 1457,725 | 35071,652 | | 259,530225 |
| 1597 | 20,028 | 1455,677 | 29577,705 | | 218,875017 |
| 1598 | 52,304 | 1455,576 | 331660,813 | | 2454,29002 |
| 1599 | 51,028 | 1455,55 | 35265,898 | | 260,967645 |
| 1600 | 60,356 | 1453,339 | 4032,295 | | 29,838983 |
| 1601 | 45,098 | 1453,108 | 32228,975 | | 238,494415 |
| 1602 | 22,717 | 1452,713 | 45172,172 | | 334,274073 |
| 1603 | 20,322 | 1452,661 | 11800,213 | | 87,3215762 |
| 1604 | **25,906** | **1451,759** | **2339633,5** | **20870,565** | 17313,2879 |
| 1605 | 22,074 | 1451,69 | 248246,688 | | 1837,02549 |
| 1606 | 59,326 | 1451,369 | 4813,728 | | 35,6215872 |
| 1607 | 59,701 | 1451,353 | 1536,576 | | 11,3706624 |
| 1608 | 58,268 | 1451,321 | 3431,627 | | 25,3940398 |
| 1609 | 58,798 | 1451,281 | 5105,037 | | 37,7772738 . |
| 1610 | 45,048 | 1450,942 | 91047,344 | | 673,750346 |
| 1611 | 23,167 | 1450,662 | 13077,415 | | 96,772871 |
| 1612 | 31,043 | 1450,47 | 449713,969 | | 3327,88337 |
| 1613 | 21,432 | 1449,717 | 434321,813 | | 3213,98142 |
| 1614 | 22,084 | 1448,686 | 19411,707 | | 143,646632 |
| 1615 | 19,402 | 1447,709 | 167506,141 | | 1239,54544 |
| 1616 | 21,69 | 1447,618 | 597990,688 | | 4425,13109 |
| 1617 | 42,128 | 1447,568 | 8425,077 | | 62,3455698 |
| 1618 | 59,775 | 1447,348 | 5009,105 | | 37,067377 |
| 1619 | 32,688 | 1446,725 | 572850 | | 4239,09 |
| 1620 | 54,385 | 1446,459 | 14195,906 | | 105,049704 |
| 1621 | 21,355 | 1445,637 | 101465,133 | | 750,841984 |
| 1622 | 31,582 | 1445,631 | 231467,766 | | 1712,86147 |
| 1623 | 51,134 | 1445,524 | 69437,68 | | 513,838832 |
| 1624 | 52,726 | 1445,505 | 4868,882 | | 36,0297268 |
| 1625 | 25,539 | 1444,755 | 80478,234 | | 595,538932 |
| 1626 | 20,78 | 1444,136 | 11996,748 | | 88,7759352 |
| 1627 | 20,099 | 1443,858 | 48822,273 | | 361,28482 |
| 1628 | 32,312 | 1443,65 | 21127,52 | | 156,343648 |
| 1629 | 41,941 | 1443,593 | 224228,266 | | 1659,28917 |
| 1630 | 47,591 | 1442,561 | 5376,899 | | 39,7890526 |
| 1631 | 59,774 | 1442,484 | 29060,875 | | 215,050475 |
| 1632 | 52,084 | 1442,129 | 9874,53 | | 73,071522 |
| 1633 | 56,749 | 1440,464 | 17626,166 | | 130,433628 |
| 1634 | 53,231 | 1440,327 | 7973,153 | | 59,0013322 |
| 1635 | 51,285 | 1439,505 | 721402,5 | | 5338,3785 |
| 1636 | 30,909 | 1439,44 | 47137,531 | | 348,817729 |
| 1637 | 25,302 | 1438,762 | 476602,969 | | 3526,86197 |
| 1638 | 26,098 | 1438,714 | 131795,766 | | 975,288668 |
| 1639 | 31,282 | 1438,543 | 532467,688 | | 3940,26089 |
| 1640 | 59,661 | 1436,368 | 26654,971 | | 197,246785 |
| 1641 | **25,623** | **1435,749** | **403066,875** | **5145,30946** | 2982,69488 |
| 1642 | 57,527 | 1434,335 | 61294,555 | | 453,579707 |
| 1643 | 19,291 | 1430,597 | 19614,697 | | 145,148758 |
| 1644 | 45,07 | 1430,006 | 183551,078 | | 1358,27798 |
| 1645 | 31,217 | 1428,461 | 2081349,38 | | 15401,9854 |
| 1646 | 41,061 | 1427,616 | 25458,787 | | 188,395024 |
| 1647 | 21,887 | 1425,708 | 515758,594 | | 3816,6136 |
| 1648 | 32,632 | 1424,748 | 292768,469 | | 2166,48667 |
| 1649 | 20,188 | 1423,596 | 59391,871 | | 439,499845 |
| 1650 | 51,159 | 1423,54 | 678686,125 | | 5022,27733 |
| 1651 | 19,388 | 1423,463 | 14447,822 | | 106,913883 |
| 1652 | 60,406 | 1423,454 | 6015,27 | | 44,512998 |
| 1653 | 21,462 | 1423,091 | 689747,75 | | 5104,13335 |
| 1654 | 31,762 | 1423,015 | 158091,891 | | 1169,87999 |
| 1655 | 25,58 | 1422,755 | 154930,813 | | 1146,48802 |
| 1656 | 59,962 | 1420,822 | 60185,059 | | 445,369437 |
| 1657 | 47,513 | 1420,228 | 39887,438 | | 295,167041 |
| 1658 | 25,381 | 1419,602 | 160612,406 | | 1188,5318 |
| 1659 | 22,881 | 1419,568 | 164075,094 | | 1214,1557 |
| 1660 | 59,173 | 1418,341 | 8607,118 | | 63,6926732 |
| 1661 | 25,701 | 1416,669 | 250310,063 | | 1852,29447 |
| 1662 | 19,933 | 1415,938 | 15313,807 | | 113,322172 |
| 1663 | 59,853 | 1415,354 | 2729,604 | | 20,1990696 |
| 1664 | 60,002 | 1414,327 | 2912,038 | | 21,5490812 |
| 1665 | 25,344 | 1413,599 | 40637,359 | | 300,716457 |
| 1666 | 23,791 | 1413,597 | 50547,738 | | 374,053261 |
| 1667 | 45,052 | 1412,986 | 13919,365 | | 103,003301 |
| 1668 | 25,335 | 1412,84 | 14382,029 | | 106,427015 |
| 1669 | 32,132 | 1412,554 | 54933,371 | | 406,506945 |
| 1670 | 31,045 | 1412,512 | 527367,25 | | 3902,51765 |
| 1671 | 57,556 | 1412,347 | 25780,541 | | 190,776003 |
| 1672 | 32,409 | 1411,64 | 16508,381 | | 122,162019 |
| 1673 | 59,681 | 1410,329 | 5465,604 | | 40,4454696 |
| 1674 | 31,527 | 1407,728 | 41320,148 | | 305,769095 |
| 1675 | 21,351 | 1407,672 | 178818,625 | | 1323,25783 |
| 1676 | 51,17 | 1407,562 | 118187,75 | | 874,58935 |
| 1677 | 52,677 | 1407,503 | 40964,031 | | 303,133829 |
| 1678 | 45,069 | 1406,92 | 71280,117 | | 527,472866 |
| 1679 | 59,199 | 1406,339 | 3843,809 | | 28,4441866 |
| 1680 | 60,013 | 1406,165 | 13561,318 | | 100,353753 |
| 1681 | 22,464 | 1405,749 | 23630,906 | | 174,868704 |
| 1682 | 20,155 | 1405,702 | 79460,164 | | 588,005214 |
| 1683 | 32,604 | 1405,687 | 482839,156 | | 3573,00975 |
| 1684 | 41,281 | 1405,646 | 101633,367 | | 752,086916 |
| 1685 | 59,693 | 1405,026 | 18004,275 | | 133,231635 |
| 1686 | 25,352 | 1403,563 | 92161,961 | | 681,998511 |
| 1687 | 53,201 | 1402,099 | 23272,326 | | 172,215212 |
| 1688 | 51,072 | 1401,522 | 388249,438 | | 2873,04584 |
| 1689 | 31,654 | 1401,509 | 7511,224 | | 55,5830576 |
| 1690 | 30,98 | 1401,473 | 171155,313 | | 1266,54932 |
| 1691 | 25,705 | 1400,678 | 71240,352 | | 527,178605 |
| 1692 | 60,454 | 1399,435 | 15121,45 | | 111,89873 |
| 1693 | 19,068 | 1397,692 | 3839,199 | | 28,4100726 |
| 1694 | 25,389 | 1397,57 | 528235,875 | | 3908,94548 |
| 1695 | 32,288 | 1396,606 | 10441,645 | | 77,268173 |
| 1696 | 31,138 | 1396,434 | 127553,898 | | 943,898845 |
| 1697 | 57,725 | 1396,36 | 55281,977 | | 409,08663 |
| 1698 | 21,421 | 1392,695 | 709464,313 | | 5250,03592 |
| 1699 | 59,374 | 1391,448 | 4601,684 | | 34,0524616 |
| 1700 | 59,651 | 1391,428 | 2721,883 | | 20,1419342 |
| 1701 | 45,062 | 1390,986 | 37551,5 | | 277,8811 |
| 1702 | 31,091 | 1390,532 | 1131354,75 | | 8372,02515 |
| 1703 | 32,449 | 1389,93 | 406237,781 | | 3006,15958 |
| 1704 | 43,567 | 1388,482 | 94132,688 | | 696,581891 |
| 1705 | 51,546 | 1386,784 | 3704,049 | | 27,4099626 |
| 1706 | 25,04 | 1386,521 | 93427,875 | | 691,366275 |
| 1707 | 59,273 | 1386,36 | 24964,27 | | 184,735598 |
| 1708 | 45,063 | 1385,978 | 210238,953 | | 1555,76825 |
| 1709 | 51,138 | 1385,566 | 41882,465 | | 309,930241 |
| 1710 | 30,901 | 1385,488 | 20099,48 | | 148,736152 |
| 1711 | 59,7 | 1382,403 | 30916,771 | | 228,784105 |
| 1712 | 47,077 | 1382,273 | 64972,203 | | 480,794302 |
| 1713 | 25,349 | 1381,698 | 426013,25 | | 3152,49805 |
| 1714 | 21,186 | 1380,693 | 20415,264 | | 151,072954 |
| 1715 | 25,109 | 1380,547 | 10967,781 | | 81,1615794 |
| 1716 | 53,108 | 1380,342 | 13301,506 | | 98,4311444 |
| 1717 | **25,679** | **1378,704** | **342898,781** | **2415,39444** | 2537,45098 |
| 1718 | 49,632 | 1378,394 | 5379,274 | | 39,8066276 |
| 1719 | 18,97 | 1377,85 | 30348,645 | | 224,579973 |
| 1720 | 25,346 | 1375,624 | 46687,473 | | 345,4873 |
| 1721 | 32,125 | 1375,532 | 67528,18 | | 499,708532 |
| 1722 | 37,369 | 1375,445 | 8201,093 | | 60,6880882 |
| 1723 | 34,801 | 1375,438 | 17825,877 | | 131,91149 |
| 1724 | 55,31 | 1374,42 | 31385,559 | | 232,253137 |
| 1725 | 56,785 | 1374,415 | 9927,623 | | 73,4644102 |
| 1726 | 32,405 | 1373,689 | 38635,266 | | 285,900968 |
| 1727 | 60,227 | 1373,36 | 6000,033 | | 44,4002442 |
| 1728 | 45,05 | 1368,949 | 34093,426 | | 252,291352 |
| 1729 | 32,464 | 1367,728 | 132403,016 | | 979,782318 |
| 1730 | 55,22 | 1367,496 | 1103,651 | | 8,1670174 |
| 1731 | 60,375 | 1366,465 | 12431,157 | | 91,9905618 |
| 1732 | 25,359 | 1365,647 | 47731,473 | | 353,2129 |
| 1733 | 45,052 | 1365,037 | 104198,375 | | 771,067975 |
| 1734 | 19,189 | 1364,748 | 11513,005 | | 85,196237 |
| 1735 | 25,813 | 1364,605 | 75452,961 | | 558,351911 |
| 1736 | 25,024 | 1364,429 | 298666,156 | | 2210,12955 |
| 1737 | 51,066 | 1363,59 | 4498,999 | | 33,2925926 |
| 1738 | 30,979 | 1363,458 | 95695,992 | | 708,150341 |
| 1739 | 45,02 | 1362,89 | 110947,891 | | 821,014393 |
| 1740 | 32,068 | 1359,662 | 52750,859 | | 390,356357 |
| 1741 | 25,534 | 1359,656 | 1906445,75 | | 14107,6986 |
| 1742 | 31,049 | 1358,466 | 151038,281 | | 1117,68328 |
| 1743 | 49,608 | 1356,381 | 45604,996 | | 337,47697 |
| 1744 | 59,708 | 1356,319 | 29863,813 | | 220,992216 |
| 1745 | 60,659 | 1355,61 | 1210,575 | | 8,958255 |
| 1746 | 55,572 | 1354,558 | 1049,276 | | 7,7646424 |
| 1747 | 58,894 | 1354,391 | 2738,662 | | 20,2660988 |
| 1748 | 23,999 | 1353,72 | 101441,016 | | 750,663518 |
| 1749 | 23,416 | 1352,847 | 43856,227 | | 324,53608 |
| 1750 | 30,605 | 1352,398 | 90038,555 | | 666,285307 |
| 1751 | 32,39 | 1351,686 | 32230,543 | | 238,506018 |
| 1752 | 34,799 | 1351,387 | 8059,731 | | 59,6420094 |
| 1753 | 25,989 | 1350,605 | 40075,652 | | 296,559825 |
| 1754 | 59,605 | 1350,549 | 6613,854 | | 48,9425196 |
| 1755 | 59,372 | 1349,363 | 5556,154 | | 41,1155396 |
| 1756 | 25,073 | 1348,535 | 290868,375 | | 2152,42598 |
| 1757 | 18,811 | 1347,718 | 12626,824 | | 93,4384976 |
| 1758 | 25,458 | 1343,664 | 766104,375 | | 5669,17238 |
| 1759 | 26,465 | 1342,628 | 17343,17 | | 128,339458 |
| 1760 | 53,156 | 1342,392 | 5754,031 | | 42,5798294 |
| 1761 | 45,041 | 1341,946 | 191931,266 | | 1420,29137 |
| 1762 | 29,936 | 1339,384 | 45313,438 | | 335,319441 |
| 1763 | 22,748 | 1338,66 | 77636,578 | | 574,510677 |
| 1764 | 60,353 | 1338,395 | 2928,564 | | 21,6713736 |
| 1765 | 30,621 | 1336,417 | 37646,219 | | 278,582021 |
| 1766 | 55,091 | 1334,981 | 17385,801 | | 128,654927 |
| 1767 | 59,445 | 1334,35 | 36656,449 | | 271,257723 |
| 1768 | 19,066 | 1332,751 | 11472,639 | | 84,8975286 |
| 1769 | 25,099 | 1332,551 | 113287,906 | | 838,330504 |
| 1770 | 59,549 | 1330,309 | 2890,745 | | 21,391513 |
| 1771 | 59,275 | 1330,277 | 9237,706 | | 68,3590244 |
| 1772 | 25,154 | 1326,551 | 949441,625 | | 7025,86803 |
| 1773 | 45,045 | 1324,926 | 42727,93 | | 316,186682 |
| 1774 | 31,961 | 1322,603 | 28099,598 | | 207,937025 |
| 1775 | 25,586 | 1321,685 | 2478338,5 | | 18339,7049 |
| 1776 | 30,808 | 1321,321 | 58397,359 | | 432,140457 |
| 1777 | 45,004 | 1321,021 | 66336,734 | | 490,891832 |
| 1778 | 20,636 | 1320,662 | 25817,596 | | 191,05021 |
| 1779 | 32,429 | 1319,441 | 11738,492 | | 86,8648408 |
| 1780 | 31,4 | 1319,42 | 6886,302 | | 50,9586348 |
| 1781 | 52,954 | 1319,408 | 3111,002 | | 23,0214148 |
| 1782 | 50,24 | 1319,394 | 4092,932 | | 30,2876968 |
| 1783 | 48,515 | 1319,389 | 12137,362 | | 89,8164788 |
| 1784 | 34,136 | 1319,387 | 43983,848 | | 325,480475 |
| 1785 | 35,227 | 1319,362 | 4880,322 | | 36,1143828 |
| 1786 | 45 | 1318,861 | 87233,422 | | 645,527323 |
| 1787 | 31,907 | 1318,611 | 13398,739 | | 99,1506686 |
| 1788 | 41,768 | 1318,589 | 232563,891 | | 1720,97279 |
| 1789 | 39,529 | 1318,407 | 9566,113 | | 70,7892362 |
| 1790 | 46,954 | 1318,405 | 13212,546 | | 97,7728404 |
| 1791 | 53,154 | 1318,387 | 4069,485 | | 30,114189 |
| 1792 | 39,074 | 1318,375 | 7447,503 | | 55,1115222 |
| 1793 | 59,704 | 1318,363 | 36163,148 | | 267,607295 |
| 1794 | 30,603 | 1314,452 | 239448,859 | | 1771,92156 |
| 1795 | 21,917 | 1312,679 | 77039,18 | | 570,089932 |
| 1796 | 26,005 | 1312,635 | 141686,484 | | 1048,47998 |
| 1797 | 52,047 | 1311,498 | 829,617 | | 6,1391658 |
| 1798 | 25,103 | 1310,563 | 660235,938 | | 4885,74594 |
| 1799 | 60,227 | 1306,401 | 3903,396 | | 28,8851304 |
| 1800 | 45,07 | 1303,966 | 34127,25 | | 252,54165 |
| 1801 | 25,985 | 1302,62 | 19980,832 | | 147,858157 |
| 1802 | 51,644 | 1302,438 | 2354,193 | | 17,4210282 |
| 1803 | 59,746 | 1302,393 | 45806,871 | | 338,970845 |
| 1804 | 22,252 | 1301,272 | 57392,363 | | 424,703486 |
| 1805 | 54,153 | 1300,353 | 8612,625 | | 63,733425 |
| 1806 | 21,95 | 1299,873 | 83524,633 | | 618,082284 |
| 1807 | 51,573 | 1299,424 | 30573,758 | | 226,245809 |
| 1808 | 53,107 | 1299,416 | 25302,227 | | 187,23648 |
| 1809 | 19,258 | 1298,642 | 21156,547 | | 156,558448 |
| 1810 | 59,374 | 1298,359 | 12482,001 | | 92,3668074 |
| 1811 | 45,03 | 1297,919 | 185944,781 | | 1375,99138 |
| 1812 | 19,022 | 1296,685 | 28328,535 | | 209,631159 |
| 1813 | 59,676 | 1295,779 | 9187,956 | | 67,9908744 |
| 1814 | 28,449 | 1295,485 | 170789,578 | | 1263,84288 |
| 1815 | 25,008 | 1294,604 | 46179,98 | | 341,731852 |
| 1816 | 30,249 | 1293,53 | 103213,75 | | 763,78175 |
| 1817 | 23,194 | 1292,502 | 19937,287 | | 147,535924 |
| 1818 | 30,646 | 1290,433 | 8808,077 | | 65,1797698 |
| 1819 | 28,43 | 1289,456 | 18305,74 | | 135,462476 |
| 1820 | 58,946 | 1289,403 | 1384,637 | | 10,2463138 |
| 1821 | 59,627 | 1289,387 | 2301,153 | | 17,0285322 |
| 1822 | 19,693 | 1289,386 | 4377,44 | | 32,393056 |
| 1823 | 25,244 | 1288,534 | 2999592,25 | | 22196,9827 |
| 1824 | 59,353 | 1286,338 | 15882,02 | | 117,526948 |
| 1825 | 30,82 | 1283,472 | 39585,328 | | 292,931427 |
| 1826 | 51,523 | 1283,445 | 6039,294 | | 44,6907756 |
| 1827 | 53,186 | 1283,43 | 8806,266 | | 65,1663684 |
| 1828 | 34,802 | 1283,335 | 8705,754 | | 64,4225796 |
| 1829 | 59,03 | 1282,394 | 15341,116 | | 113,524258 |
| 1830 | 55,529 | 1282,378 | 7111,861 | | 52,6277714 |
| 1831 | 45,022 | 1280,883 | 30969,486 | | 229,174196 |
| 1832 | 19,518 | 1280,665 | 11716,79 | | 86,704246 |
| 1833 | 59,668 | 1280,411 | 49052 | | 362,9848 |
| 1834 | 28,452 | 1279,513 | 51832,395 | | 383,559723 |
| 1835 | 51,44 | 1277,454 | 82467,625 | | 610,260425 |
| 1836 | 52,927 | 1277,446 | 43342,348 | | 320,733375 |
| 1837 | 45,003 | 1276,982 | 70758,93 | | 523,616082 |
| 1838 | 30,44 | 1276,942 | 232610,75 | | 1721,31955 |
| 1839 | 50,608 | 1276,907 | 1109,392 | | 8,2095008 |
| 1840 | 23,116 | 1276,515 | 122081,953 | | 903,406452 |
| 1841 | 44,995 | 1274,825 | 81134,063 | | 600,392066 |
| 1842 | 26,295 | 1272,61 | 41668,891 | | 308,349793 |
| 1843 | 25,135 | 1272,604 | 1010380,88 | | 7476,81848 |
| 1844 | 20,731 | 1272,48 | 34951,859 | | 258,643757 |
| 1845 | 30,456 | 1272,332 | 289698,906 | | 2143,7719 |
| 1846 | 31,95 | 1270,569 | 58033,617 | | 429,448766 |
| 1847 | 54,813 | 1270,498 | 1479,774 | | 10,9503276 |
| 1848 | 59,96 | 1270,475 | 3445,866 | | 25,4994084 |
| 1849 | 57,989 | 1270,447 | 1281,669 | | 9,4843506 |
| 1850 | 59,188 | 1270,424 | 5638,458 | | 41,7245892 |
| 1851 | 37,091 | 1269,475 | 1353,805 | | 10,018157 |
| 1852 | 40,97 | 1269,437 | 2427,428 | | 17,9629672 |
| 1853 | 35,834 | 1269,418 | 2728,887 | | 20,1937638 |
| 1854 | 40,087 | 1269,413 | 1651,728 | | 12,2227872 |
| 1855 | 39,127 | 1269,409 | 5886,622 | | 43,5610028 |
| 1856 | 36,13 | 1269,399 | 4146,72 | | 30,685728 |
| 1857 | 30,571 | 1267,515 | 21216,277 | | 157,00045 |
| 1858 | 59,349 | 1266,399 | 6219,467 | | 46,0240558 |
| 1859 | 54,894 | 1266,362 | 4477,317 | | 33,1321458 |
| 1860 | 22,184 | 1263,606 | 94234,227 | | 697,33328 |
| 1861 | 59,935 | 1263,437 | 57252,406 | | 423,667804 |
| 1862 | 34,938 | 1263,312 | 16850,229 | | 124,691695 |
| 1863 | 37,489 | 1262,942 | 10585,427 | | 78,3321598 |
| 1864 | 55,342 | 1262,696 | 14468,394 | | 107,066116 |
| 1865 | 52,525 | 1262,35 | 8346,335 | | 61,762879 |
| 1866 | 39,287 | 1262,338 | 12846,079 | | 95,0609846 |
| 1867 | 40,214 | 1262,335 | 8975,618 | | 66,4195732 |
| 1868 | 58,04 | 1262,315 | 3522,354 | | 26,0654196 |
| 1869 | 21,765 | 1262,289 | 123903,367 | | 916,884916 |
| 1870 | 51,525 | 1261,458 | 99836,336 | | 738,788886 |
| 1871 | 53,043 | 1261,455 | 83416,992 | | 617,285741 |
| 1872 | 23,116 | 1260,537 | 46480,016 | | 343,952118 |
| 1873 | 34,799 | 1259,311 | 14362,161 | | 106,279991 |
| 1874 | 28,459 | 1257,534 | 298317,219 | | 2207,54742 |
| 1875 | 31,559 | 1255,699 | 116446,039 | | 861,700689 |
| 1876 | 30,3 | 1255,583 | 846248,25 | | 6262,23705 |
| 1877 | 32,837 | 1255,567 | 35492,637 | | 262,645514 |
| 1878 | 33,291 | 1255,561 | 28837,1 | | 213,39454 |
| 1879 | 51,244 | 1255,489 | 2861,904 | | 21,1780896 |
| 1880 | 32,024 | 1254,973 | 95395,18 | | 705,924332 |
| 1881 | 23,278 | 1254,568 | 121895,391 | | 902,025893 |
| 1882 | 59,74 | 1254,366 | 5949,095 | | 44,023303 |
| 1883 | 57,787 | 1254,364 | 4684,266 | | 34,6635684 |
| 1884 | 58,949 | 1254,347 | 2524,573 | | 18,6818402 |
| 1885 | 57,081 | 1254,319 | 8885,642 | | 65,7537508 |
| 1886 | 55,421 | 1254,301 | 4541,622 | | 33,6080028 |
| 1887 | 45,012 | 1253,896 | 147699,641 | | 1092,97734 |
| 1888 | 28,407 | 1251,496 | 141158,859 | | 1044,57556 |
| 1889 | **25,249** | **1250,631** | **3974873,75** | **34371,53** | 29414,0658 |
| 1890 | 58,643 | 1250,455 | 11072,911 | | 81,9395414 |
| 1891 | 18,98 | 1249,756 | 27491,875 | | 203,439875 |
| 1892 | 31,656 | 1248,538 | 26623,307 | | 197,012472 |
| 1893 | 32,92 | 1247,361 | 41003,309 | | 303,424487 |
| 1894 | 51,498 | 1245,498 | 8761,765 | | 64,837061 |
| 1895 | 53,085 | 1245,476 | 44366,938 | | 328,315341 |
| 1896 | 19,632 | 1244,64 | 30831,113 | | 228,150236 |
| 1897 | 59,714 | 1242,427 | 56404,984 | | 417,396882 |
| 1898 | 56,779 | 1241,078 | 2758,177 | | 20,4105098 |
| 1899 | 30,278 | 1239,607 | 317820,063 | | 2351,86847 |
| 1900 | 31,547 | 1239,563 | 59098,148 | | 437,326295 |
| 1901 | 28,472 | 1239,526 | 69276,641 | | 512,647143 |
| 1902 | 51,321 | 1239,492 | 69059,977 | | 511,04383 |
| 1903 | 52,866 | 1239,474 | 32897,426 | | 243,440952 |
| 1904 | 60,102 | 1238,604 | 6424,091 | | 47,5382734 |
| 1905 | 23,198 | 1238,572 | 492393,188 | | 3643,70959 |
| 1906 | 25,057 | 1238,532 | 9391,734 | | 69,4988316 |
| 1907 | 37,49 | 1237,332 | 6531,968 | | 48,3365632 |
| 1908 | 45,022 | 1236,87 | 46682,961 | | 345,453911 |
| 1909 | 19,717 | 1236,611 | 7412,524 | | 54,8526776 |
| 1910 | 40,236 | 1236,283 | 8209,991 | | 60,7539334 |
| 1911 | 52,172 | 1235,898 | 888,905 | | 6,577897 |
| 1912 | 19,301 | 1235,868 | 4725,906 | | 34,9717044 |
| 1913 | 30,417 | 1234,37 | 856949,25 | | 6341,42445 |
| 1914 | 31,611 | 1233,532 | 122064,055 | | 903,274007 |
| 1915 | 25,133 | 1232,595 | 24227,906 | | 179,286504 |
| 1916 | 21,214 | 1231,482 | 60734,938 | | 449,438541 |
| 1917 | 32,839 | 1231,084 | 60950,719 | | 451,035321 |
| 1918 | 42,855 | 1230,887 | 154045,125 | | 1139,93393 |
| 1919 | 30,565 | 1229,563 | 91573,625 | | 677,644825 |
| 1920 | 22,209 | 1228,627 | 18700,881 | | 138,386519 |
| 1921 | 60,379 | 1227,376 | 3367,176 | | 24,9171024 |
| 1922 | 52,522 | 1227,324 | 6150,93 | | 45,516882 |
| 1923 | 51,79 | 1227,323 | 4718,815 | | 34,919231 |
| 1924 | 49,813 | 1227,305 | 11513,576 | | 85,2004624 |
| 1925 | 40,192 | 1227,301 | 7194,075 | | 53,236155 |
| 1926 | 59,636 | 1226,901 | 24848,395 | | 183,878123 |
| 1927 | 21,859 | 1224,549 | 44181,957 | | 326,946482 |
| 1928 | 30,406 | 1224,415 | 69779,727 | | 516,36998 |
| 1929 | 23,094 | 1222,581 | 91712,016 | | 678,668918 |
| 1930 | 51,595 | 1222,471 | 3662,568 | | 27,1030032 |
| 1931 | 30,29 | 1218,52 | 476128,844 | | 3523,35345 |
| 1932 | 31,528 | 1217,669 | 163110,922 | | 1207,02082 |
| 1933 | 25,119 | 1217,539 | 169168,734 | | 1251,84863 |
| 1934 | 23,259 | 1216,597 | 263113,625 | | 1947,04083 |
| 1935 | 44,996 | 1215,355 | 16854,1 | | 124,72034 |
| 1936 | 31,638 | 1211,447 | 117543,125 | | 869,819125 |
| 1937 | 55,57 | 1210,742 | 2894,646 | | 21,4203804 |
| 1938 | 20,68 | 1210,615 | 33522,359 | | 248,065457 |
| 1939 | 31,023 | 1210,478 | 47086,57 | | 348,440618 |
| 1940 | 60,002 | 1210,378 | 2460,045 | | 18,204333 |
| 1941 | 59,452 | 1210,36 | 2822,82 | | 20,888868 |
| 1942 | 53,367 | 1210,321 | 12792,264 | | 94,6627536 |
| 1943 | 56,782 | 1210,2 | 4122,384 | | 30,5056416 |
| 1944 | 45,004 | 1209,865 | 121766,766 | | 901,074068 |
| 1945 | 25,804 | 1209,47 | 31311,213 | | 231,702976 |
| 1946 | 20,729 | 1206,596 | 10410,285 | | 77,036109 |
| 1947 | 51,214 | 1205,491 | 3944,53 | | 29,189522 |
| 1948 | 31,553 | 1203,616 | 23597,301 | | 174,620027 |
| 1949 | 49,516 | 1203,133 | 8821,522 | | 65,2792628 |
| 1950 | 30,195 | 1202,589 | 39710,629 | | 293,858655 |
| 1951 | 50,689 | 1202,365 | 25647,943 | | 189,794778 |
| 1952 | 51,135 | 1201,518 | 6787,769 | | 50,2294906 |
| 1953 | 42,67 | 1200,783 | 72117,422 | | 533,668923 |
| 1954 | 23,167 | 1200,609 | 1313535,88 | | 9720,16548 |
| 1955 | 25,206 | 1200,547 | 16658,26 | | 123,271124 |
| 1956 | 46,71 | 1200,503 | 19274,873 | | 142,63406 |
| 1957 | 30,044 | 1198,562 | 22444,525 | | 166,089485 |
| 1958 | 59,87 | 1198,361 | 4803,752 | | 35,5477648 |
| 1959 | 59,432 | 1198,353 | 4145,694 | | 30,6781356 |
| 1960 | 19,706 | 1198,222 | 12631,365 | | 93,472101 |
| 1961 | 21,013 | 1197,031 | 87272,023 | | 645,81297 |
| 1962 | 30,406 | 1196,417 | 620780,5 | | 4593,7757 |
| 1963 | 60,6 | 1196,352 | 7650,589 | | 56,6143586 |
| 1964 | 31,578 | 1195,579 | 464075,188 | | 3434,15639 |
| 1965 | 59,222 | 1190,363 | 5847,735 | | 43,273239 |
| 1966 | 53,252 | 1188,334 | 55063,84 | | 407,472416 |
| 1967 | 59,825 | 1187,418 | 3255,71 | | 24,092254 |
| 1968 | 21,882 | 1186,597 | 192521,25 | | 1424,65725 |
| 1969 | 60,255 | 1186,382 | 6004,868 | | 44,4360232 |
| 1970 | 30,174 | 1185,657 | 58927,867 | | 436,066216 |
| 1971 | 42,453 | 1184,524 | 53516,508 | | 396,022159 |
| 1972 | 59,174 | 1184,402 | 4914,623 | | 36,3682102 |
| 1973 | 21,325 | 1183,635 | 234638,141 | | 1736,32224 |
| 1974 | 59,7 | 1183,443 | 7250,531 | | 53,6539294 |
| 1975 | 31,801 | 1182,586 | 11399,009 | | 84,3526666 |
| 1976 | 31,299 | 1181,569 | 94998,031 | | 702,985429 |
| 1977 | 49,745 | 1181,254 | 3827,925 | | 28,326645 |
| 1978 | 56,778 | 1181,221 | 3641,417 | | 26,9464858 |
| 1979 | 60,62 | 1180,368 | 5690,042 | | 42,1063108 |
| 1980 | 50,733 | 1180,335 | 38225,785 | | 282,870809 |
| 1981 | 31,583 | 1179,597 | 236420,203 | | 1749,5095 |
| 1982 | 19,143 | 1178,579 | 11057,592 | | 81,8261808 |
| 1983 | 20,779 | 1178,495 | 131686,141 | | 974,477443 |
| 1984 | 51,247 | 1178,431 | 6476,849 | | 47,9286826 |
| 1985 | 41,654 | 1178,397 | 6623,212 | | 49,0117688 |
| 1986 | 56,782 | 1178,216 | 4255,032 | | 31,4872368 |
| 1987 | 25,333 | 1175,511 | 22551,197 | | 166,878858 |
| 1988 | 24,101 | 1172,463 | 51734,363 | | 382,834286 |
| 1989 | 53,214 | 1172,348 | 38737,391 | | 286,656693 |
| 1990 | 44,949 | 1170,829 | 10637,769 | | 78,7194906 |
| 1991 | 22,67 | 1169,26 | 18541,186 | | 137,204776 |
| 1992 | 54,977 | 1169,167 | 7155,165 | | 52,948221 |
| 1993 | 60,253 | 1166,411 | 2237,358 | | 16,5564492 |
| 1994 | 53,22 | 1166,339 | 140311,391 | | 1038,30429 |
| 1995 | 31,322 | 1165,301 | 58614,336 | | 433,746086 |
| 1996 | 31,542 | 1163,587 | 34373,684 | | 254,365262 |
| 1997 | 42,864 | 1163,561 | 317640,313 | | 2350,53832 |
| 1998 | 20,221 | 1162,602 | 72301,289 | | 535,029539 |
| 1999 | 30,294 | 1160,457 | 18931,172 | | 140,090673 |
| 2000 | 20,533 | 1159,644 | 11926,854 | | 88,2587196 |
| 2001 | 25,361 | 1158,633 | 29713,969 | | 219,883371 |
| 2002 | 31,529 | 1157,615 | 44626,973 | | 330,2396 |
| 2003 | 24,123 | 1156,492 | 33221,211 | | 245,836961 |
| 2004 | 19,431 | 1154,638 | 47105,656 | | 348,581854 |
| 2005 | 31,311 | 1154,523 | 72400,438 | | 535,763241 |
| 2006 | 23,924 | 1152,527 | 201380,953 | | 1490,21905 |
| 2007 | 30,384 | 1152,393 | 58578,801 | | 433,483127 |
| 2008 | 21,985 | 1150,625 | 37468,707 | | 277,268432 |
| 2009 | 53,194 | 1150,361 | 120706,516 | | 893,228218 |
| 2010 | 30,027 | 1149,312 | 119640,453 | | 885,339352 |
| 2011 | 42,541 | 1147,575 | 30441,695 | | 225,268543 |
| 2012 | 23,629 | 1144,649 | 29225,717 | | 216,270306 |
| 2013 | 31,311 | 1143,136 | 23237,896 | | 171,96043 |
| 2014 | 24,001 | 1142,545 | 23198,781 | | 171,670979 |
| 2015 | 50,597 | 1142,373 | 118697,242 | | 878,359591 |
| 2016 | 23,275 | 1141,59 | 47004,074 | | 347,830148 |
| 2017 | 21,014 | 1140,488 | 174479,281 | | 1291,14668 |
| 2018 | 41,649 | 1140,415 | 5001,429 | | 37,0105746 |
| 2019 | 19,442 | 1138,647 | 71437,797 | | 528,639698 |
| 2020 | 31,367 | 1138,553 | 111894,984 | | 828,022882 |
| 2021 | 25,359 | 1137,454 | 68654,234 | | 508,041332 |
| 2022 | 53,625 | 1137,364 | 4344,629 | | 32,1502546 |
| 2023 | 23,802 | 1136,534 | 21588,393 | | 159,754108 |
| 2024 | 25,107 | 1135,542 | 37640,664 | | 278,540914 |
| 2025 | 22,904 | 1134,648 | 71461,75 | | 528,81695 |
| 2026 | 24,113 | 1134,504 | 347155,5 | | 2568,9507 |
| 2027 | 60,091 | 1134,459 | 5499,415 | | 40,695671 |
| 2028 | 59,353 | 1134,437 | 3032,865 | | 22,443201 |
| 2029 | 53,188 | 1134,391 | 56996,746 | | 421,77592 |
| 2030 | 51,575 | 1134,21 | 9499,726 | | 70,2979724 |
| 2031 | 29,971 | 1133,34 | 41626,363 | | 308,035086 |
| 2032 | 20,936 | 1128,605 | 47002,93 | | 347,821682 |
| 2033 | 28,199 | 1128,485 | 105328,227 | | 779,42888 |
| 2034 | 53,209 | 1128,38 | 354067,813 | | 2620,10182 |
| 2035 | 50,589 | 1126,389 | 28851,957 | | 213,504482 |
| 2036 | 31,552 | 1125,231 | 11812,239 | | 87,4105686 |
| 2037 | 41,868 | 1125,221 | 15596,393 | | 115,413308 |
| 2038 | 32,903 | 1125,218 | 126022,156 | | 932,563954 |
| 2039 | 31,908 | 1125,217 | 25750,453 | | 190,553352 |
| 2040 | 39,864 | 1125,21 | 6728,264 | | 49,7891536 |
| 2041 | 36,571 | 1125,206 | 22293,379 | | 164,971005 |
| 2042 | 39,556 | 1125,202 | 16396,361 | | 121,333071 |
| 2043 | 26,016 | 1125,161 | 31188 | | 230,7912 |
| 2044 | 29,167 | 1125,148 | 41243,914 | | 305,204964 |
| 2045 | 38,059 | 1124,829 | 29475,412 | | 218,118049 |
| 2046 | 31,341 | 1122,552 | 34561,762 | | 255,757039 |
| 2047 | 23,72 | 1122,456 | 36783,59 | | 272,198566 |
| 2048 | 44,964 | 1121,808 | 44746,047 | | 331,120748 |
| 2049 | 18,812 | 1119,758 | 5198,635 | | 38,469899 |
| 2050 | 24,134 | 1118,525 | 119093,102 | | 881,288955 |
| 2051 | 25,151 | 1118,499 | 30842,926 | | 228,237652 |
| 2052 | 23,899 | 1114,554 | 208412,047 | | 1542,24915 |
| 2053 | 29,989 | 1111,346 | 183555,891 | | 1358,31359 |
| 2054 | 28,181 | 1110,479 | 70840,586 | | 524,220336 |
| 2055 | 51,147 | 1110,414 | 125425,094 | | 928,145696 |
| 2056 | 20,151 | 1108,578 | 25817,43 | | 191,048982 |
| 2057 | 23,801 | 1106,903 | 187048,984 | | 1384,16248 |
| 2058 | 50,557 | 1104,421 | 171816,922 | | 1271,44522 |
| 2059 | 58,615 | 1102,375 | 6383,561 | | 47,2383514 |
| 2060 | 51,593 | 1101,214 | 1985,06 | | 14,689444 |
| 2061 | 49,777 | 1100,828 | 2256,389 | | 16,6972786 |
| 2062 | 19,219 | 1100,543 | 6005,286 | | 44,4391164 |
| 2063 | 21,831 | 1100,522 | 38794,496 | | 287,07927 |
| 2064 | 25,329 | 1099,552 | 13957,11 | | 103,282614 |
| 2065 | 53,545 | 1099,417 | 30059,629 | | 222,441255 |
| 2066 | 21,401 | 1098,745 | 112194,32 | | 830,237968 |
| 2067 | 52,17 | 1098,418 | 3632,612 | | 26,8813288 |
| 2068 | 20,123 | 1097,551 | 4589,071 | | 33,9591254 |
| 2069 | 24,128 | 1096,552 | 522675,844 | | 3867,80125 |
| 2070 | 53,285 | 1096,434 | 26559,404 | | 196,53959 |
| 2071 | 51,387 | 1096,263 | 109630,906 | | 811,268704 |
| 2072 | 29,987 | 1095,392 | 58529 | | 433,1146 |
| 2073 | 19,018 | 1094,643 | 6701,293 | | 49,5895682 |
| 2074 | 20,501 | 1091,546 | 15707,116 | | 116,232658 |
| 2075 | 23,598 | 1090,488 | 10901,328 | | 80,6698272 |
| 2076 | 53,19 | 1090,433 | 338706,531 | | 2506,42833 |
| 2077 | 20,982 | 1082,62 | 65489,449 | | 484,621923 |
| 2078 | 21,397 | 1082,584 | 32035,098 | | 237,059725 |
| 2079 | 19,289 | 1080,554 | 12653,619 | | 93,6367806 |
| 2080 | 53,408 | 1076,445 | 4457,142 | | 32,9828508 |
| 2081 | 53,657 | 1076,436 | 3256,97 | | 24,101578 |
| 2082 | 52,905 | 1076,431 | 2378,771 | | 17,6029054 |
| 2083 | 20,569 | 1075,555 | 86250,758 | | 638,255609 |
| 2084 | 29,991 | 1073,413 | 40851,953 | | 302,304452 |
| 2085 | 23,494 | 1068,813 | 147886,688 | | 1094,36149 |
| 2086 | 21,443 | 1068,575 | 62297,086 | | 460,998436 |
| 2087 | 18,988 | 1068,472 | 18219,826 | | 134,826712 |
| 2088 | 23,8 | 1064,568 | 7053,475 | | 52,195715 |
| 2089 | 20,279 | 1063,503 | 21330,266 | | 157,843968 |
| 2090 | 19,116 | 1062,663 | 4397,519 | | 32,5416406 |
| 2091 | 32,024 | 1060,467 | 45340,27 | | 335,517998 |
| 2092 | 60,603 | 1060,416 | 6794,908 | | 50,2823192 |
| 2093 | 52,255 | 1058,393 | 12166,359 | | 90,0310566 |
| 2094 | 54,069 | 1058,366 | 9593,294 | | 70,9903756 |
| 2095 | 49,741 | 1057,321 | 5347,421 | | 39,5709154 |
| 2096 | 23,962 | 1054,469 | 82210,703 | | 608,359202 |
| 2097 | 23,588 | 1051,388 | 89102,742 | | 659,360291 |
| 2098 | 30,345 | 1044,353 | 4195,99 | | 31,050326 |
| 2099 | 23,401 | 1040,537 | 10620,867 | | 78,5944158 |
| 2100 | 20,577 | 1032,597 | 21339,391 | | 157,911493 |
| 2101 | 24,092 | 1025,286 | 62953,594 | | 465,856596 |
| 2102 | 19,87 | 1018,527 | 7472,115 | | 55,293651 |
| 2103 | 23,953 | 1016,515 | 121646,805 | | 900,186357 |
| 2104 | 30,418 | 1016,354 | 134689,156 | | 996,699754 |
| 2105 | 20,528 | 1015,594 | 21665,502 | | 160,324715 |
| 2106 | 23,573 | 1013,438 | 115542,805 | | 855,016757 |
| 2107 | 30,392 | 1011,276 | 33455,215 | | 247,568591 |
| 2108 | 18,922 | 1010,399 | 9148,562 | | 67,6993588 |
| 2109 | 20,423 | 1006,679 | 8503,45 | | 62,92553 |
| 2110 | 47,1 | 998,211 | 46778,898 | | 346,163845 |
| 2111 | 23,462 | 995,402 | 32905,055 | | 243,497407 |
| 2112 | 51,388 | 991,214 | 2437,004 | | 18,0338296 |
| 2113 | 21,942 | 988,585 | 32196,633 | | 238,255084 |
| 2114 | 20,878 | 988,565 | 12280,876 | | 90,8784824 |
| 2115 | 29,367 | 987,262 | 48744,594 | | 360,709996 |
| 2116 | 23,484 | 984,509 | 27134,879 | | 200,798105 |
| 2117 | 47,966 | 984,342 | 3780,828 | | 27,9781272 |
| 2118 | 23,398 | 981,665 | 20057,672 | | 148,426773 |
| 2119 | 30,895 | 981,502 | 25569,025 | | 189,210785 |
| 2120 | 19,773 | 978,532 | 4305,65 | | 31,86181 |
| 2121 | 30,386 | 973,323 | 32013,334 | | 236,898672 |
| 2122 | 29,291 | 971,253 | 5491,579 | | 40,6376846 |
| 2123 | 20,901 | 969,104 | 41305,473 | | 305,6605 |
| 2124 | 25,141 | 965,491 | 48757,68 | | 360,806832 |
| 2125 | 24,072 | 964,44 | 7556,274 | | 55,9164276 |
| 2126 | 20,829 | 963,206 | 16317,524 | | 120,749678 |
| 2127 | 28,81 | 962,49 | 19231,721 | | 142,314735 |
| 2128 | 20,219 | 960,462 | 9783,454 | | 72,3975596 |
| 2129 | 23,379 | 956,597 | 24888,623 | | 184,17581 |
| 2130 | 22,899 | 956,484 | 12570,926 | | 93,0248524 |
| 2131 | 59,245 | 955,564 | 1391,343 | | 10,2959382 |
| 2132 | 19,095 | 954,938 | 8325,797 | | 61,6108978 |
| 2133 | 20,123 | 952,494 | 5814,21 | | 43,025154 |
| 2134 | 60,591 | 950,571 | 4099,453 | | 30,3359522 |
| 2135 | 40,291 | 950,357 | 19953,055 | | 147,652607 |
| 2136 | 41,194 | 950,357 | 47130,363 | | 348,764686 |
| 2137 | 24,004 | 949,406 | 63025,402 | | 466,387975 |
| 2138 | 29,386 | 949,317 | 56691,895 | | 419,520023 |
| 2139 | 51,536 | 948,37 | 24475,225 | | 181,116665 |
| 2140 | 60,327 | 947,639 | 4107,147 | | 30,3928878 |
| 2141 | 23,951 | 945,481 | 43559,406 | | 322,339604 |
| 2142 | 20,894 | 944,588 | 148397,719 | | 1098,14312 |
| 2143 | 30,047 | 944,458 | 13261,684 | | 98,1364616 |
| 2144 | 23,976 | 933,488 | 10559,713 | | 78,1418762 |
| 2145 | 20,085 | 931,546 | 45600,914 | | 337,446764 |
| 2146 | 18,961 | 926,553 | 4596,708 | | 34,0156392 |
| 2147 | 20,898 | 925,264 | 49014,879 | | 362,710105 |
| 2148 | 20,978 | 920,557 | 5005,199 | | 37,0384726 |
| 2149 | 18,938 | 918,591 | 4865,919 | | 36,0078006 |
| 2150 | 56,883 | 918,079 | 1112,862 | | 8,2351788 |
| 2151 | 51,665 | 915,677 | 1583,223 | | 11,7158502 |
| 2152 | 52,923 | 915,395 | 2432,005 | | 17,996837 |
| 2153 | 29,904 | 914,917 | 25552,555 | | 189,088907 |
| 2154 | 40,82 | 912,413 | 454771,625 | | 3365,31003 |
| 2155 | 23,975 | 911,5 | 11979,838 | | 88,6508012 |
| 2156 | 18,986 | 911,287 | 4659,146 | | 34,4776804 |
| 2157 | 29,339 | 906,271 | 22190,25 | | 164,20785 |
| 2158 | 51,09 | 902,979 | 700,182 | | 5,1813468 |
| 2159 | 20,832 | 902,486 | 52433,75 | | 388,00975 |
| 2160 | 49,645 | 900,36 | 24044,1 | | 177,92634 |
| 2161 | 24,096 | 898,383 | 45429,156 | | 336,175754 |
| 2162 | 56,504 | 897,659 | 5498,265 | | 40,687161 |
| 2163 | 56,278 | 896,829 | 1467,63 | | 10,860462 |
| 2164 | 22,577 | 896,41 | 21323,018 | | 157,790333 |
| 2165 | 19,262 | 895,584 | 4205,946 | | 31,1240004 |
| 2166 | 23,992 | 882,405 | 17175,494 | | 127,098656 |
| 2167 | 55,786 | 880,774 | 2373,386 | | 17,5630564 |
| 2168 | 59,474 | 879,635 | 1745,675 | | 12,917995 |
| 2169 | 19,949 | 879,567 | 5377,363 | | 39,7924862 |
| 2170 | 29,754 | 876,473 | 23381,605 | | 173,023877 |
| 2171 | 42,753 | 875,379 | 114573,516 | | 847,844018 |
| 2172 | 21,028 | 872,459 | 5747,54 | | 42,531796 |
| 2173 | 19,956 | 868,588 | 5911,415 | | 43,744471 |
| 2174 | 51,593 | 867,774 | 1445,617 | | 10,6975658 |
| 2175 | 55,321 | 867,675 | 724,974 | | 5,3648076 |
| 2176 | 59,198 | 867,635 | 1190,664 | | 8,8109136 |
| 2177 | 58,799 | 867,622 | 2502,561 | | 18,5189514 |
| 2178 | 20,829 | 852,468 | 7367,667 | | 54,5207358 |
| 2179 | 49,047 | 846,946 | 1713,276 | | 12,6782424 |
| 2180 | 57,438 | 846,946 | 2112,215 | | 15,630391 |
| 2181 | 60,566 | 846,924 | 2585,42 | | 19,132108 |
| 2182 | 22,644 | 846,426 | 1215,356 | | 8,9936344 |
| 2183 | 29,086 | 841,303 | 8721,339 | | 64,5379086 |
| 2184 | 53,187 | 837,301 | 11143,638 | | 82,4629212 |
| 2185 | 55,22 | 835,737 | 611,264 | | 4,5233536 |
| 2186 | 54,381 | 832,253 | 1154,893 | | 8,5462082 |
| 2187 | 59,347 | 824,672 | 836,045 | | 6,186733 |
| 2188 | 20,776 | 824,421 | 2193,866 | | 16,2346084 |
| 2189 | 59,758 | 824,025 | 5333,992 | | 39,4715408 |
| 2190 | 58,792 | 823,646 | 7006,314 | | 51,8467236 |
| 2191 | 53,249 | 821,319 | 3106,751 | | 22,9899574 |
| 2192 | 60,055 | 815,685 | 1288,741 | | 9,5366834 |
| 2193 | 29,084 | 815,416 | 2310,018 | | 17,0941332 |
| 2194 | 50,654 | 813,317 | 13369,253 | | 98,9324722 |
| 2195 | 53,192 | 799,334 | 37386,438 | | 276,659641 |

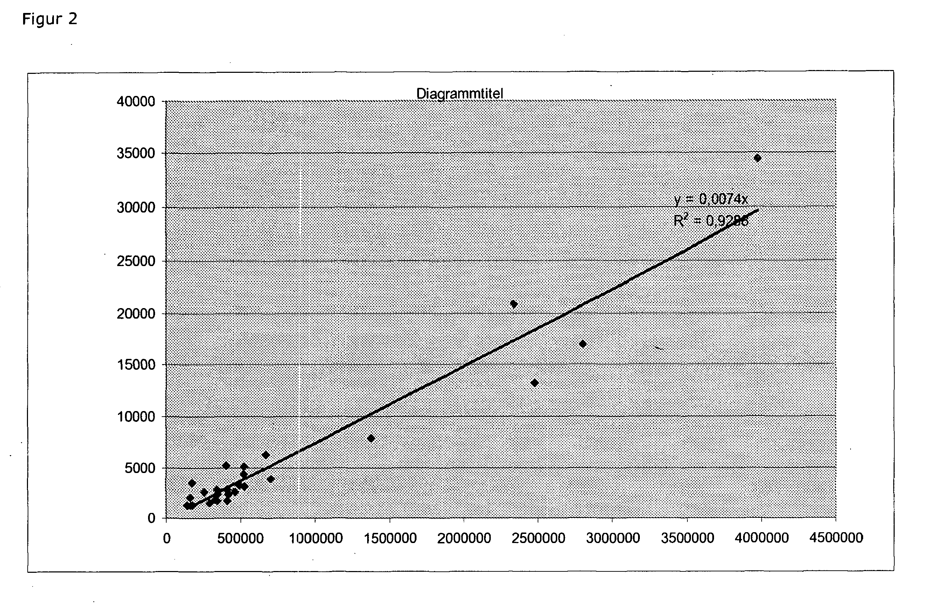

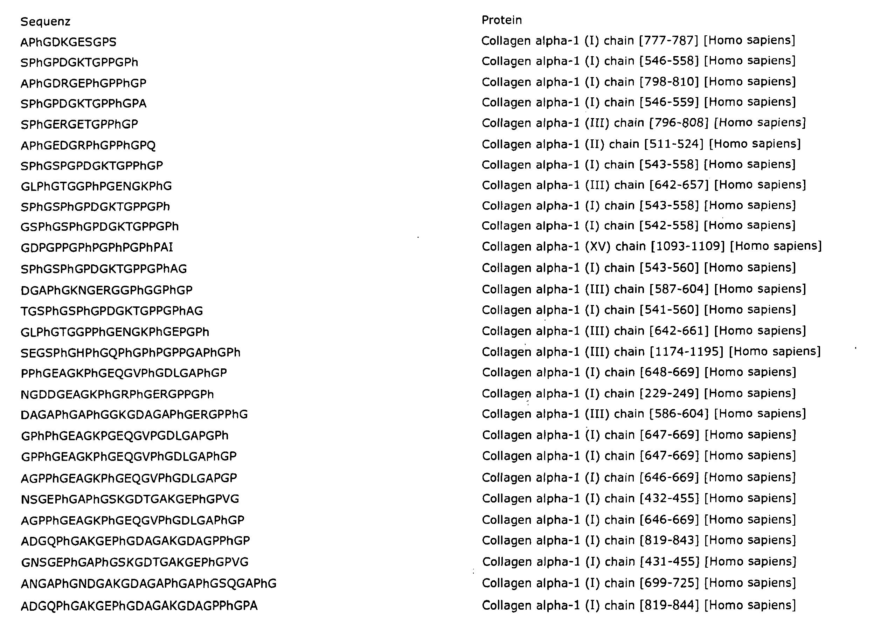

Die Qualität der Regression ist in Figur 2 dargestellt.

## Patentansprüche

1. Verfahren zur Normierung der Konzentration mindestens eines Analyten in einer Urinprobe umfassend folgende Schritte:
a) Bestimmung der Konzentration mindestens eines Analyten,
b) Bestimmung der Konzentration mindestens eines Kollagens oder Kollagenfragmentes aus der Urinprobe,
c) Normierung der Konzentration des mindestens einen Analyten relativ zur Konzentration des mindestens einen Kollagens oder Kollagenfragmentes.

2. Verfahren nach Anspruch 1, **dadurch gekennzeichnet, dass** die Urinprobe vor der Bestimmung der Konzentration des Analyten in mehrere Teilproben aufgetrennt wird.

3. Verfahren nach Anspruch 2, **dadurch gekennzeichnet, dass** die Auftrennung durch
- chromatographische Verfahren wie Affinitätschromatographie, Ionenaustauschchromatographie, reversed phase Chromatographie, Hydrophobe Interaktionschromatographie, Gelfiltration gegebenenfalls unter Verwendung von FPLC oder HPLC;
- Adsorption an bestimmte Materialien wie SELDI-Verfahren, CiinProt Verfahren,
- elektrophoretische Verfahren wie Gel-Elektrophorese oder Kapillarelektrophorese,
erfolgt.

4. Verfahren nach mindestens einem der Ansprüche 1 bis 3, **dadurch gekennzeichnet, dass** die Bestimmung des Gehaltes an Analyten und/oder Kollagen oder Kollagenfragmenten in den Teilproben über einen UV/VIS-Detektor, einen Fluoreszenz-Detektor, einen Brechungsindex-Detektor, ein Diodenarray-Detektor, ein Massenspektrometer, einen Gelscanner, Färbung oder Derivatisierung und nachfolgende Detektion mit geeigneten Mitteln erfolgt.

5. Verfahren nach mindestens einem der Ansprüche 1 bis 4, **dadurch gekennzeichnet, dass** das mindestens eine Kollagen oder Kollagenfragment über die Migrations- bzw. Retentionszeit, und eventuell weitere geeignete Parameter wie Massenspektren, UV/VIS Spektren, Fluoreszenzspektren, Affinität zu bestimmten Liganden beispielsweise Antikörpern, identifiziert wird.

6. Verfahren nach mindestens einem der Ansprüche 1 bis 5, **dadurch gekennzeichnet, dass** das mindestens eine Kollagenfragment ausgewählt wird aus den Peptiden mit der Seq. ID Nr. 1 bis 42.

7. Verfahren nach mindestens einem der Ansprüche 1 bis 6, **dadurch gekennzeichnet, dass** die Konzentration mindestens zwei oder mindestens drei, bevorzugt mindestens 5, 10, 20, 30, 40 oder 50 Kollagenen oder Kollagenfragmenten bestimmt und zur Normierung eingesetzt werden.

8. Verfahren nach mindestens einem der Ansprüche 1 bis 7, **dadurch gekennzeichnet, dass** die Normierung durch lineare Regression erfolgt.

9. Verwendung von Kollagenen oder Kollagenfragmenten zur Normierung von Analytkonzentrationen in Urinproben, wobei die Bestimmung der konzentration der Kollagene oder Kollagenfragmente aus der Urinprobe erfolgt.

10. Verwendung nach Anspruch 9, **dadurch gekennzeichnet, dass** die Kollagenfragmente ausgewählt werden aus den Peptiden mit der Seq. ID Nr. 1 bis 42.

11. Verwendung nach Anspruch 9 oder 10, **dadurch gekennzeichnet, dass** mindestens zwei oder mindestens drei, bevorzugt mindestens 5, 10, 20, 30, 40 oder 50 Kollagene oder Kollagenfragmente zur Normierung eingesetzt werden.

## Claims

1. A process for normalizing the concentration of at least one analyte in a urine sample, comprising the following steps:
a) determining the concentration of at least one analyte;
b) determining the concentration of at least one collagen or collagen fragment from said urine sample;
c) normalizing the concentration of said at least one analyte relative to the concentration of said at least one collagen or collagen fragment.

2. The process according to claim 1, **characterized in that** the urine sample is subdivided into several subsamples before the concentration of the analyte is determined.

3. The process according to claim 2, **characterized in that** said separation is effected by:
- chromatographic methods, such as affinity chromatography, ionexchange chromatography, reversed-phase chromatography, hydrophobic interaction chromatography, gel filtration, optionally using FPLC or HPLC;
- adsorption to specific materials, such as SELDI process, ClinProt process;
- electrophoretic methods, such as gel electrophoresis or capillary electrophoresis.

4. The process according to at least one of claims 1 to 3, **characterized in that** said determination of the contents of analytes and/or collagen or collagen fragments in said subsamples is effected by a UV/Vis detector, fluorescence detector, refractive index detector, diode array detector, mass spectrometer, gel scanner, staining or derivatization, followed by detection with suitable means.

5. The process according to at least one of claims 1 to 4, **characterized in that** said at least one collagen or collagen fragment is identified by its migration or retention time and optionally further suitable parameters, such as mass spectra, UV/Vis spectra, fluorescence spectra, affinity for particular ligands, for example, antibodies.

6. The process according to at least one of claims 1 to 5, **characterized in that** said at least one collagen fragment is selected from the peptides of SEQ ID Nos. 1 to 42.

7. The process according to at least one of claims 1 to 6, **characterized in that** the concentrations of at least two or at least three, preferably at least 5, 10, 20, 30, 40 or 50 collagens or collagen fragments are determined and employed for normalization.

8. The process according to at least one of claims 1 to 7, **characterized in that** said normalization is effected by linear regression.

9. Use of collagens or collagen fragments for the normalization of analyte concentrations in urine samples, wherein the determination of the concentration of said collagens or collagen fragments is effected from said urine sample.

10. The use according to claim 9, **characterized in that** said collagen fragments are selected from the peptides of SEQ ID Nos. 1 to 42.

11. The use according to claim 9 or 10, **characterized in that** at least two or at least three, preferably at least 5, 10, 20, 30, 40 or 50 collagens or collagen fragments are employed for normalization.

## Revendications

1. Procédé de normalisation de la concentration d'au moins un analyte dans un échantillon d'urine, ledit procédé comportant les étapes suivantes :
a) la détermination de la concentration d'au moins un analyte,
b) la détermination de la concentration d'au moins un collagène ou fragment de collagène dans l'échantillon d'urine,
c) la normalisation de la concentration de l'au moins un analyte par rapport à la concentration de l'au moins un collagène ou fragment de collagène.

2. Procédé selon la revendication 1, **caractérisé en ce que** l'échantillon d'urine est séparé en plusieurs parties d'échantillon d'urine avant de déterminer la concentration de l'analyte.

3. Procédé selon la revendication 2, **caractérisé en ce que** la séparation est effectuée par
- des procédés chromatographiques comme la chromatographie d'affinité, la chromatographie à échange d'ions, la chromatographie en phase reverse, la chromatographie par interaction hydrophobe, la filtration sur gel en utilisant éventuellement la FPLC ou HPLC ;
- adsorption sur certains matériaux comme le procédé SELDI, le procédé ClinProt,
- des procédés électrophorétiques comme l'électrophorèse sur gel ou l'électrophorèse capillaire.

4. Procédé selon au moins une des revendications 1 à 3, **caractérisé en ce que** la détermination de la teneur en analytes et/ou collagènes ou fragments de collagènes dans les parties d'échantillon est effectuée par un détecteur UV/VIS, un détecteur de fluorescence, un détecteur d'indice de réfraction, d'un détecteur à barrette de diodes, un spectromètre de masse, un scanner de gel, par coloration ou dérivation puis détection par des moyens appropriés.

5. Procédé selon au moins une des revendications 1 à 4, **caractérisé en ce que** l'au moins un collagène ou fragment de collagène est identifié par le temps de migration ou de rétention, et éventuellement par d'autres paramètres appropriés comme les spectres de masse, les spectres UV/VIS, les spectres de fluorescence, l'affinité pour certains ligands par exemples des anticorps.

6. Procédé selon au moins une des revendications 1 à 5, **caractérisé en ce que** l'au moins un fragment de collagène est choisi parmi les peptides ayant les séquences ID n°1 à n°42.

7. Procédé selon au moins une des revendications 1 à 6, **caractérisé en ce que** la concentration d'au moins deux ou d'au moins trois, de préférence d'au moins 5, 10, 20, 30, 40 ou 50 collagènes ou fragments de collagènes est déterminée et utilisée pour la normalisation.

8. Procédé selon au moins une des revendications 1 à 7, **caractérisé en ce que** la normalisation est effectuée par régression linaire.

9. Utilisation de collagènes ou fragments de collagènes pour normaliser des concentrations d'analytes dans des échantillons d'urine, la détermination de la concentration en collagènes ou fragments de collagènes étant effectuée à partir de l'échantillon d'urine.

10. Utilisation selon la revendication 9, **caractérisée en ce que** les fragments de collagènes sont choisis parmi les peptides ayant les séquences ID n°1 à 42.

11. Utilisation selon la revendication 9 ou 10, **caractérisée en ce que** au moins deux ou au moins trois, de préférence au moins 5, 10, 20, 30, 40 ou 50 collagènes ou fragments de collagènes sont utilisés pour la normalisation.
